(19) Europäisches Patentamt
European Patent Office
Office européen des brevets

(11) Veröffentlichungsnummer: **0 468 281 B1**

# (12) EUROPÄISCHE PATENTSCHRIFT

(45) Veröffentlichungstag der Patentschrift: **08.03.95**

(51) Int. Cl.$^{6}$: **C07C 259/06**, C07C 275/64, C07D 333/20, C07D 333/66, C07D 211/28

(21) Anmeldenummer: **91111487.4**

(22) Anmeldetag: **10.07.91**

## (54) Substituierte Phenylacetylene, diese enthaltende Arzneimittel und Verfahren zur Herstellung dieser Verbindungen und Arzneimittel.

(30) Priorität: **26.07.90 DE 4023742**

(43) Veröffentlichungstag der Anmeldung:
**29.01.92 Patentblatt 92/05**

(45) Bekanntmachung des Hinweises auf die Patenterteilung:
**08.03.95 Patentblatt 95/10**

(84) Benannte Vertragsstaaten:
**AT BE CH DE DK ES FR GB GR IT LI LU NL SE**

(56) Entgegenhaltungen:
**EP-A- 0 030 922**
**EP-A- 0 199 985**
**EP-A- 0 292 699**
**FR-A- 2 602 767**

(73) Patentinhaber: **Grünenthal GmbH**
**Zieglerstrasse 6**
**D-52078 Aachen (DE)**

(72) Erfinder: **Zimmer, Oswald, Dr. rer. nat.**
**Weckspfad 4**
**D-5160 Düren (DE)**
Erfinder: **Vollenberg, Werner, Dr. rer. nat.**
**Eupener Strasse 26A**
**D-5190 Stolberg (DE)**
Erfinder: **Seipp, Ulrich, Dr. rer. nat.**
**Pfeilstrasse 5**
**D-5100 Aachen (DE)**
Erfinder: **Englberger, Werner, Dr. rer. nat.**
**Rudolfstrasse 15**
**D-5190 Stolberg (DE)**
Erfinder: **Haurand, Michael, Dr. rer. nat.**
**Auf der Geiss 40**
**D-5190 Stolberg/Rhnld (DE)**
Erfinder: **Bosman, Brigitte J. Dr. med.**
**Waldsiedlung 5**
**D-5107 Simmerath-Lammersdorf (DE)**
Erfinder: **Schneider, Johannes, Dr. med. vet.**
**Rolandstrasse 40**
**D-5190 Stolberg (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Polyungesättigte höhere Fettsäuren, wie z. B. die Arachidonsäure dienen im Stoffwechsel des Menschen und von Säugetieren als Substrate für die enzymatisch katalysierte Bildung physiologisch bedeutsamer Eicosanoide wie z. B. Prostaglandine und Leukotriene, einer auch unter dem Namen "Slow reacting Substance of Anaphylaxis" (SRS-A) bekannten Substanzklasse. Dabei wird die Prostaglandinbildung durch die Cyclo-oxygenase (auch als "Prostaglandinsynthetase" bezeichnet), die Leukotrienbildung durch 5-Lipoxygenase katalysiert.

Während die Prostaglandine eine Anzahl erwünschter Wirkungen im Organismus entfalten, ist von den Leukotrienen bzw. der SRS-A, bekannt, daß sie für die Entstehung von allergischen Reaktionen bis zum anaphylaktischen Schock, Bronchokonstriktionen, Entzündungen, Asthma und eine Vielzahl weiterer unerwünschter Effekte verantwortlich sind. Es wäre daher wünschenswert, über chemisch und metabolisch stabile Verbindungen verfügen zu können, die im Organismus die Prostaglandinbildung unbeeinflußt lassen, gleichzeitig aber möglichst selektiv bzw. spezifisch die 5-Lipoxygenase hemmen und so die Bildung der unerwünschten Leukotriene verhindern.

Aus der europäischen Patentanmeldung EP 292 699 sind Harnstoffderivate bekannt, die eine Phenylethyl- oder eine Phenylethenyl-Gruppe enthalten können und sowohl 5-Lipoxygenase als auch 12-Lipoxygenase hemmen.

Die in der europäischen Patentanmeldung EP 199 985 offenbarten Benzoesäurederivate, beispielsweise 3-(11'-Cyclohexyl-11'-hydroxy-undeca-1',9'-diin-1'-yl)-N-methylbenzhydroxamsäure gemäß Beispiel 23b)III), sind spezifisch wirkende 5-Lipoxygenaseinhibitoren.

Es wurde nunmehr gefunden, daß bestimmte substituierte Phenylacetylene, bei denen es sich um chemisch und metabolisch (bei therapeutischer Anwendung) stabile Verbindungen handelt, eine spezifische Hemmwirkung gegenüber der 5-Lipoxygenase besitzen und sich insbesondere zum Einsatz als Antiasthmatika sowie beim anaphylaktischen Schock eignen.

Diese neuen substituierten Phenylacetylene entsprechen der allgemeinen Formel

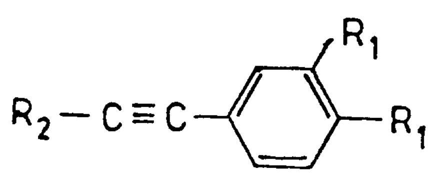

I

Darin bedeutet einer der Reste $R_1$ ein Wasserstoffatom, der andere die Gruppe der Formel

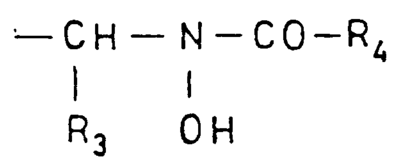

worin $R_3$ ein Wasserstoffatom, einen Methyl- oder einen Ethylrest und $R_4$ einen Methylrest oder eine Aminogruppe darstellt.

In der Formel I steht $R_2$

a) für einen ein- oder zweikernigen aromatischen Rest aus der Gruppe mit den Formeln

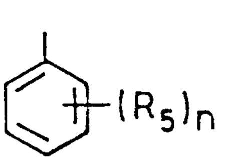 , 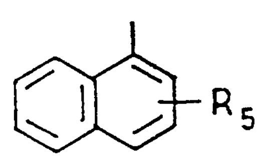 , 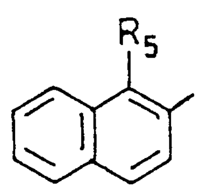 oder 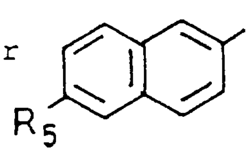

worin $R_5$ ein Wasserstoffatom, eine Benzyloxygruppe, eine geradkettige oder verzweigte Alkyl-, Alkoxy-

oder Alkylmercaptogruppe mit 1 bis 4 Kohlenstoffatomen, ein Fluor- oder Chloratom oder die Trifluormethylgruppe und n eine ganze Zahl von 1 bis 3 darstellt,

oder

b) für einen ein- oder zweikernigen, Schwefel, Stickstoff oder Sauerstoff enthaltenden heterocyclischen Rest aus der Gruppe mit den Formeln

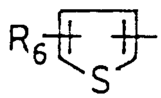 , 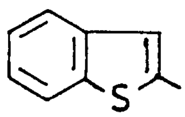 , 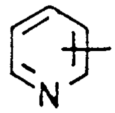 , 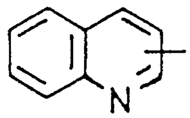 oder 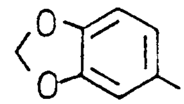

worin $R_6$ ein Wasserstoff- oder ein Chloratom oder eine Methylgruppe bedeutet.

Ist insbesondere der Rest $R_3$ von Wasserstoff verschieden, so tritt an dem diesen Rest tragenden Kohlenstoffatom ein Asymmetriezentrum auf. Ein solches Zentrum kann auch für den Fall (gegebenenfalls zusätzlich zu dem zuvor genannten) auftreten, daß $R_5$ einen 1-Methyl-propyl-rest (bzw. einen sek.-Butylrest) darstellt. Zum Gegenstand der Erfindung gehören in diesen Fällen sowohl die Enantiomerengemische als auch die Stereoisomeren, insbesondere bei Vorliegen nur eines Asymmetriezentrums die Razemate sowie die optisch aktiven Formen der Verbindungen der Formel I.

In bevorzugten Verbindungen der Formel I stellt $R_2$ eine der folgenden Gruppen dar:

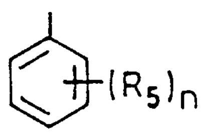 , 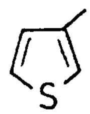 sowie 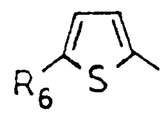

worin $R_5$, $R_6$ und n die gleiche Bedeutung wie oben haben und $R_5$ insbesondere für Methoxy steht.

Von dieser Gruppe von bevorzugten Verbindungen der Formel I werden wiederum diejenigen besonders bevorzugt, in denen $R_5$ eine Methoxygruppe in 3- und/oder 4-Stellung oder ein Fluor- oder Chloratom in 4-Stellung zur Acetylengruppe darstellt, n also die Zahl 1 oder 2 ist bzw. in denen $R_6$ für ein Wasserstoffatom steht.

Steht $R_2$ für einen Pyridin- oder Chinolinrest, so trägt dieser die Acetylengruppe vorzugsweise in der 3-Stellung.

Eine bevorzugte Bedeutung von $R_3$ ist die als Methylgruppe.

Bezüglich der bevorzugten Position des von Wasserstoff verschiedenen Restes $R_1$ am Phenylring gilt folgendes:

Stellt $R_2$ einen gegebenenfalls in der definierten Weise substituierten ein- oder zweikernigen aromatischen Rest, vorzugsweise einen durch $R_5$ ein- bis dreifach substituierten Phenylrest dar, so ist der Rest $R_1$ vorzugsweise in 3-Stellung zur Acetylengruppierung angeknüpft. Solche bevorzugten Verbindungen entsprechen dann den Formeln

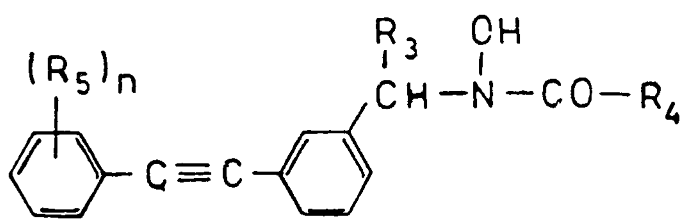

Ia

bzw.

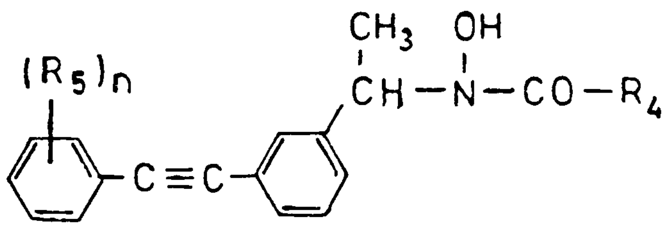

Ib

Insbesondere in der Formel Ib steht vorzugsweise $R_4$ für die Aminogruppe:

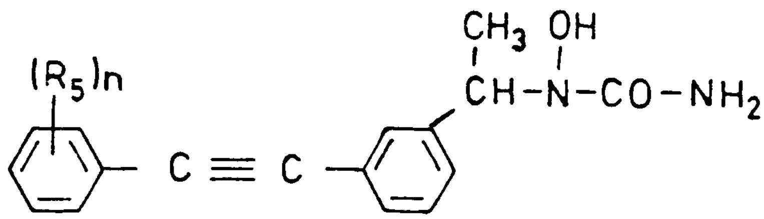

Ic

In diesen Formeln haben $R_3$, $R_5$ und n die gleiche Bedeutung wie oben.

Stellt dagegen $R_2$ einen gegebenenfalls durch $R_6$ substituierten Thienylrest dar, so ist der Rest $R_1$ vorzugsweise in 4-Stellung zur Acetylengruppierung angeknüpft.

Diese bevorzugten Verbindungen entsprechen dann der Formel

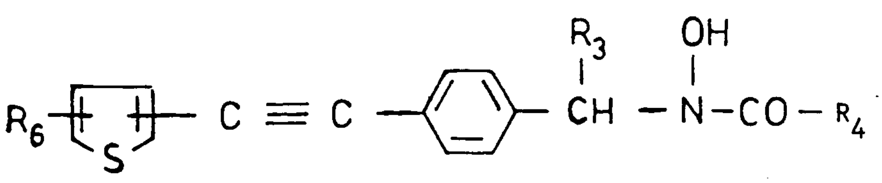

Id

worin $R_3$, $R_4$ und $R_6$ die gleiche Bedeutung wie oben haben.

Wie bereits einleitend ausgeführt, besitzen die Verbindungen der Formel I eine spezifische Hemmwirkung gegenüber der 5-Lipoxygenase, was u. a. durch in-vitro-Experimente ermittelt wurde.

Zur Ermittlung der 5-Lipoxygenasehemmung wurden basophile leukämische Leukozyten der Ratte (RBL-1-Zellen) in vitro gezüchtet, vom Nährmedium abzentrifugiert, mit Puffer (Kaliumphosphat 50 mM, pH 7,4) gewaschen und anschließend auf eine Zellzahl von 1 x $10^7$ Zellen pro ml eingestellt. Jeweils 1 ml dieser Zellsuspension wurde mit 10 $\mu$M Indomethacin, 2 mM $CaCl_2$ und mit Testsubstanz in Konzentrationsbereichen von 0,1 $\mu$M bis 100 $\mu$M oder entsprechenden Kontrollen mit Lösungsmittel bei Raumtemperatur für 3 Minuten vorinkubiert und anschließend mit 20 $\mu$M radioaktiv markierter Arachidonsäure und 20 $\mu$M des Calcium-Ionophors A 23187 für 10 Minuten inkubiert. Nach Abstoppen der Reaktion durch Zugabe von 20 $\mu$l Eisessig wurden die Reaktionsprodukte mit Ethylacetat extrahiert und mit einem für Lipoxygenaseprodukte geeigneten Laufmittelgemisch dünnschichtchromatographisch getrennt (Jakschik et al., Biochem. Biophys. Res. Commun. 102, 624 (1981)).

Die Radioakfivitätsverteilung der verschiedenen Arachidonsäuremetabolite wurde mit Hilfe eines TLC-Linear-Analyzers gemessen. Aus den prozentualen Bildungsanteilen der 5-Lipoxygenaseprodukte (5-HETE, $LTB_4$-Isomere) bei den Kontrollen (Abwesenheit von Testsubstanzen) und bei verschiedenen Konzentrationen der eingesetzten Verbindungen der Formel I wurden graphisch aus halblogarithmischen Diagrammen "$IC_{50}$-Werte" (d. h. die Konzentrationen, die eine 50 %ige Hemmung der 5-Lipoxygenase bewirken) bestimmt. Zur Standardisierung wurden diese Werte auf die im gleichen Test ermittelten $IC_{50}$-Werte für die Standardsubstanz Nordihydroguaiaretinsäure bezogen. Diese standardisierten "$IC_{50}$-Werte (A)" sind in Tabelle 1 angegeben.

Der Einfluß der Verbindungen der Formel I auf die Cyclooxygenase-Aktivität wurde mit Hilfe von Schafsamenblasenmikrosomen, suspendiert in Puffer (Kaliumphosphat 50 mM, pH 7,0), durch Inkubation mit Testsubstanz oder Lösungsmittel (als Kontrolle)und radioaktiv markierter Arachidonsäure untersucht. Die

"$IC_{50}$-Werte (B)" für die Hemmung der Cyclooxygenase wurden mittels Dünnschichtchromatographie und TLC-Linear-Analyzer bestimmt.

So wurden beispielsweise für die Produkte der in der folgenden Tabelle genannten Beispiele die jeweils angegebenen $IC_{50}$-Werte gefunden, aus denen der Quotient

$$\frac{IC_{50}\ (B)}{IC_{50}\ (A)}$$

errechnet wurde.

Tabelle 1

| Beispiel | $IC_{50}$ [$\mu$M] für die Hemmung der | | Quotient |
|---|---|---|---|
| | 5-Lipoxygenase (A) | Cyclooxygenase (B) | |
| 1 | 0,67 | >500 | >746 |
| 5a | 0,29 | 440 | 1517 |
| 5b | 0,28 | >500 | >1786 |
| 5c | 0,58 | >500 | >862 |
| 7 | 0,65 | >500 | >769 |
| 9 | 0,15 | 100 | 667 |
| 10b | 0,69 | >500 | >725 |
| 10c | 0,27 | >500 | >1852 |
| 10d | 0,55 | 500 | 909 |
| 10g | 0,32 | 270 | 844 |
| 10m | 0,26 | >500 | >1923 |
| 10n | 0,18 | >500 | >2778 |
| 11 | 0,67 | >500 | >746 |
| 15 | 0,41 | 500 | 1220 |
| 16b | 0,29 | 500 | 1724 |
| 16d | 0,37 | 500 | 1351 |
| 16f | 0,41 | 500 | 1220 |

Aus dieser Tabelle 1 ergibt sich, daß die aufgeführten $IC_{50}$-Werte für die Cyclooxygenasehemmung häufig um mehr als das 1000fache höher als die Werte für die 5-Lipoxygenasehemmung liegen, d. h. daß die Testsubstanzen sehr spezifisch nur die Hemmung der 5-Lipoxygenaseaktivität bewirken.

Zur Charakterisierung der In vivo-Verfügbarkeit der Verbindungen der Formel I (nach oraler Gabe) als 5-Lipoxygenasehemmer wurde ein von Tateson et al., Brit. J. Pharmacol., 94, 528 (1988) beschriebenes Testmodell eingesetzt. Hierzu wurden männlichen Ratten (Stamm Wistar) Verbindungen der Formel I peroral appliziert. Jeweils 1 Stunde nach Substanzapplikation wurde den Tieren nach letaler $CO_2$-Narkose unter Zusatz von Heparin als Antikoagulans Vollblut entnommen. Aliquote des Vollblutes wurden im Wasserbad bei 37° C für 30 Min. mit dem Calciumionophor A 23187 in einer Endkonzentration von 15 $\mu$g/ml inkubiert. Anschließend wurden die Inkubationsansätze zentrifugiert und in aliquoten Teilen des zellfreien Plasmas der Gehalt an immunreaktivem $LTB_4$ ($iLTB_4$) per Radio-Immun-Assay "RIA" ($^3H$-$LTB_4$-RIA, Fa. Amersham) bestimmt. Der $iLTB_4$-Gehalt jeder Probe wurde anhand einer mit verdünntem Rattenplasma erstellten Standardkurve von $LTB_4$-Standardkonzentrationen rechnerisch ermittelt und als ng $iLTB_4$/ml Rattenplasma berechnet. Parallel zu den mit den Prüfsubstanzen der Formel I behandelten Tieren wurden jeweils mit Vehikellösung peroral behandelte Tiere als Kontrollgruppe eingesetzt und ihr Blut untersucht. Der Mittelwert aus den $iLTB_4$-Gehalten der Rattenplasmen dieser Kontrollgruppen diente als 100 %-Wert einer normalen 5-Lipoxygenaseaktivität. Die jeweilige prozentuale Hemmung der Ex vivo $LTB_4$-Synthese nach oraler Applikation der Prüfsubstanzen wurde wie folgt berechnet: 100 minus dem 100-fachen des Quotienten aus dem Mittelwert der $iLTB_4$-Gehalte in ng $iLTB_4$/ml der Prüfsubstanzgruppe und dem Mittelwert der $iLTB_4$-Gehalte in ng $iLTB_4$/ml der jeweiligen Kontrollgruppe. Bei der Applikation einer Dosis von 21,5 mg/kg peroral ergeben sich so für die aufgeführten Verbindungen der Formel I folgende in Tabelle 2 zusammengestellte Hemmwerte:

Tabelle 2

| Beispiel | Hemmwert in % | Beispiel | Hemmwert in % |
|---|---|---|---|
| 2a | 76 % | 10d | 99 % |
| 5a | 75 % | 10e | 81 % |
| 5c | 88 % | 11 | 76 % |
| 5e | 82 % | 12 | 87 % |
| 7 | 90 % | 15 | 77 % |
| 8b | 99 % | 16b | 91 % |
| 10b | 86 % | 16f | 97 % |

In diesem Test bewirkte das Produkt des Beispiels 9 in einer Dosis von 10 mg/kg bereits eine Hemmung der Ex vivo $LTB_4$-Synthese um 86 %.

Als ein Testmodell für die Ermittlung der Wirkung von Prüfsubstanzen beim septischen Schock kann die an der Maus durch Endotoxin/Galaktosamin induzierte Hepatitis herangezogen werden. Dabei wird durch intravenöse Injektion von 300 µg/kg Endotoxin (LPS von S. abortus equi) zusammen mit 700 mg/kg Galaktosamin an wachen Mäusen eine Hepatitis induziert, die 8 Stunden nach der Endotoxinapplikation durch Anstieg von leberspezifischen Enzymen (Glutamatpyruvattransaminase, GPT; Sorbitoldehydrogenase, SDH) nachgewiesen wird. Die intraperitoneale Applikation von 5-Lipoxygenasehemmern 30 Min. vor, gleichzeitig mit sowie 2, 4 und 6 Stunden nach der Endotoxininjektion hemmt den hepatitisbedingten Anstieg dieser Serumenzymaktivitäten. Bei 5maliger Applikation von je 10 mg/kg von Verbindungen der Formel I wurden folgende Hemmwerte des Anstiegs der Enzyme GPT und SDH (bezogen auf den Wert 100 % des Enzymanstiegs in Kontrolltieren) gefunden:

Tabelle 3

| Beispiel | Hemmung des Anstiegs von | |
|---|---|---|
| | GPT | SDH |
| 1 | 47 | 58 |
| 4 | 87 | 81 |
| 9 | 94 | 90 |

Die antiasthmatische Wirkung der Verbindungen der Formel I wurde an narkotisierten beatmeten Meerschweinchen untersucht. Zur Auslösung einer asthmatischen Reaktion wurden die Tiere passiv sensibilisiert mit Antiovalbumin Serum (0,3 ml intraperitoneal), und 48 Stunden später wurde die asthmatische Reaktion durch intravenöse Gabe von 0,2 mg/kg Ovalbumin ausgelöst. Die sofort auftretende Bronchokonstriktion wurde nach der Konzett-Rössler-Methode anhand der Zunahme des "overflow" über einen Zeitraum von 10 Min. gemessen. Effekte, die durch Histamin, Serotonin und sympathische Gegensteuerung ausgelöst werden, wurden durch intravenöse Vorbehandlung der Tiere mit 2,15 mg/kg Mepyramin, 46,4 mg/kg Propranolol, 4,64 mg/kg Atropin und 1 mg/kg Methysergid 5 Min. vor Gabe des Ovalbumins ausgeschaltet.

Bei oraler Gabe von 100 mg/kg ergab sich so eine Hemmung der Bronchokonstriktion von 54 % für das Produkt des Beispiels 5a und 67 % für das Produkt des Beispiels 9.

Die erfindungsgemäßen Verbindungen der Formel I besitzen also aufgrund ihrer selektiven Hemmung der 5-Lipoxygenase und damit der Bildung von Arachidonsäuremetaboliten wie 5-Hydroxyperoxyeicosatetraensäure (5-HPETE), 5-Hydroxyeicosatetraensäure (5-HETE) bzw. SRS-A zahlreiche physiologisch wertvolle Eigenschaften wie z. B. antianaphylaktische, antiasthmatische, antiallergische sowie antiphlogistische, blutdrucksenkende und durchblutungsfördernde (coronarer und cerebraler Kreislauf) Wirkungen, Verminderung der Leukozytenaggregation bzw. der Bildung von Leukozytenthromben usw. Da die erfindungsgemäßen Verbindungen der Formel I chemisch und metabolisch für eine therapeutische Anwendung stabil und lagerfähig sind, eignen sie sich zum Einsatz als Arzneimittel z. B. als Antiallergica, Antianaphylaktica, Antiphlogistica, Antiasthmatica, Antihypertensiva, antithrombotische Mittel, Mittel zur Prophylaxe oder Therapie von ischämischem Herzinfarkt, Störungen der coronaren und/oder cerebralen Arterien usw.

Die erfindungsgemäßen Verbindungen der Formel I besitzen nur eine geringe Toxizität, die sich erst bei weit höheren als den therapeutisch oder prophylaktisch anzuwendenden Dosierungen bemerkbar macht.

Sie können daher als solche in geeigneten pharmazeutischen Zubereitungen an Mensch oder Tier verabreicht werden.

Die Erfindung betrifft dementsprechend auch Arzneimittel, die als Wirkstoff eine oder mehrere der erfindungsgemäßen Verbindungen der Formel I enthalten. Die an den Patienten zu verabreichende Wirkstoffmenge variiert in Abhängigkeit z. B. vom Gewicht des Patienten, vom Applikationsweg, der Indikation und dem Schweregrad der Erkrankung. Unter Berücksichtigung dieser Faktoren beträgt der Wirkstoffgehalt pro Einzeldosis im allgemeinen etwa 0,01 - 50 mg, und zwar bei Zubereitungsformen für die parenterale Applikation 0,01 bis 10 mg, bei Zubereitungen für orale oder rektale Applikationen etwa 0,1 bis 50 mg.

Arzneimittel zur parenteralen Applikation können sowohl Lösungen als auch Suspensionen darstellen, es kommen aber auch leicht rekonstituierbare Trockenzubereitungen in Betracht.

Gut geeignete Applikationsformen sind auch Sprays zur intranasalen oder oralen Applikation bzw. zur Verabreichung der Substanzen über die Bronchien.

Für viele prophylaktische oder therapeutische Anwendungen kommen zweckmäßig auch oral anwendbare Zubereitungsformen der Verbindungen der Formel I, wie Tabletten, Dragees, Kapseln, Granulate, Tropfen und Säfte bzw. Sirupe, aber auch Suppositorien sowie perkutane Applikationszubereitungen (wie z. B. die Wirkstoffe in einem Depot in gelöster Form, gegebenenfalls unter Zusatz von die Hautpenetration fördernden Mitteln enthaltende Pflaster oder dergleichen) in Betracht. Vorteilhaft werden diese oral, rektal oder perkutan anwendbaren Zubereitungsformen so hergestellt, daß daraus der Wirkstoff verzögert freigesetzt wird, um so über einen längeren Zeitraum (beispielsweise 24 Stunden) eine gleichförmige Versorgung des Patienten mit dem Wirkstoff zu gewährleisten.

Alle vorstehend erwähnten pharmazeutischen Zubereitungsformen sind an sich bekannt, und da die erfindungsgemäßen Verbindungen der Formel I chemisch stabil sind, wirft ihre Einarbeitung in diese Zubereitungsformen für den Fachmann keinerlei Probleme auf. Bei dieser erfindungsgemäßen Herstellung dieser Arzneimittel muß selbstverständlich mit der üblichen Sorgfalt bei Auswahl der Hilfsstoffe wie Trägermaterialien, Farbstoffe, Geschmackskorrigentien, Bindemitteln, Tablettensprengmitteln usw. vorgegangen werden, und insbesondere bei der Herstellung von parenteral anzuwendenden Zubereitungsformen ist auf Sterilität und - wenn sie in flüssiger Form vorliegen - Isotonie zu achten.

Die erfindungsgemäße Herstellung der Verbindungen der Formel I kann in der Weise erfolgen, daß man

i) eine Verbindung der Formel

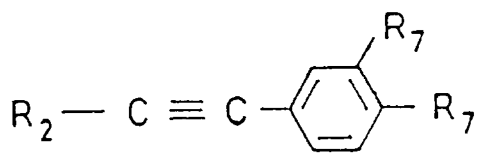

II

in der $R_2$ die gleiche Bedeutung wie oben hat und einer der Reste $R_7$ Wasserstoff, der andere die Gruppe der Formel $-CO-R_3$, in der $R_3$ wie oben definiert ist, bedeutet,
mit Hydroxylamin oder einen von dessen Salzen zum Oxim umsetzt und dieses zu einer Verbindung der Formel

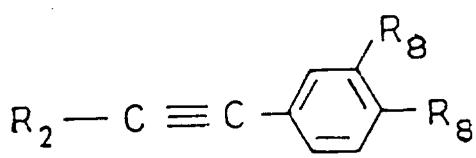

III

in der einer der Reste $R_8$ für Wasserstoff, der andere für die Gruppe der Formel $-CH(R_3)-NH-OH$ steht, reduziert.

Diese Umsetzung erfolgt in an sich bekannter Weise, z. B. in alkoholischer oder wässrig alkoholischer Lösung in Gegenwart von Basen, z. B. Pyridin, Kaliumcarbonat oder Natriumacetat bei Temperaturen von z.B. 20 - 60° C.

Die Reduktion wird mittels Borhydriden, vorzugsweise Natriumcyanoborhydrid, in Essigsäure oder ethanolischer Salzsäure bei Temperaturen zwischen z.B. 20° C und 60° C oder in alkoholischer Lösung mit Boran-Amin-Komplexen, z. B. dem Boran-Pyridin-Komplex, oder mit dem Boran-Tetrahydrofuran-Komplex, jeweils in Gegenwart von z. B. 6n-Salzsäure bei Temperaturen zwischen etwa 0° C und 20° C durchgeführt (vgl. u. a. J. B. Summers, et al., J. Med. Chem. 31, 1960 (1988)).

Durch Einführung des Acylrestes der Formel $-COR_4$ erhält man dann aus der Hydroxylaminverbindung der Formel III das gewünschte Produkt der Formel I.

Zur Herstellung von Verbindungen der Formel I mit $R_4 = NH_2$ (N-Hydroxyharnstoffe) wird die Verbindung der Formel III zunächst mit Trimethylsilyl-isocyanat in inerten Lösungsmitteln, vorzugsweise cyclischen Ethern wie Tetrahydrofuran oder 1,4-Dioxan, auf Temperaturen zwischen 20° C und der Siedetemperatur des Lösungsmittels erwärmt. Durch Hydrolyse, z. B. mit gesättigter Ammonium- oder Natriumchloridlösung, des intermediär gebildeten Additionsprodukts erhält man die gewünschte Verbindung der Formel I mit $R_4 = NH_2$.

Alternativ kann man diese in an sich bekannter Weise auch durch Umsetzung der Verbindung der Formel III entweder mit Kalium- oder Natriumcyanat in saurer Lösung oder mit Phosgen oder einem Chlorameisensäure-(niedrigalkyl)- oder -benzylester in Gegenwart eines säurebindenden Mittels, wie z. B. Natrium- oder Kaliumcarbonat, gefolgt von einer Behandlung mit Ammoniak oder z. B. Ammoniumcarbonat erhalten.

Zur Herstellung von Verbindungen der Formel I mit $R_4 = CH_3$ (Acetohydroxamsäuren) wird die Verbindung der Formel III, gegebenenfalls ohne vorherige Isolierung, zunächst mit einem Acetylierungsmittel, vorzugsweise Acetanhydrid oder Acetylchlorid in Gegenwart säurebindender Stoffe wie Pyridin oder Chinolin umgesetzt, wobei eine Verbindung der Formel

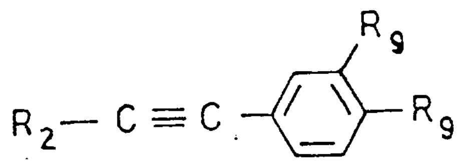

IV

worin einer der Reste $R_9$ ein Wasserstoffatom ist und der andere die Gruppe der Formel

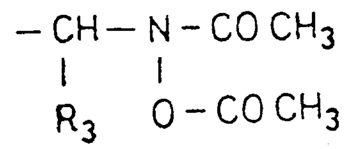

darstellt,
erhalten wird, aus der dann durch selektive basische Hydrolyse in Methanol oder Ethanol als Lösungsmittel bei Temperaturen zwischen etwa 20° C und 60° C die O-Acetylgruppe unter Bildung der gewünschten Verbindung der Formel I abgespalten wird. Als Basen dienen bei dieser Hydrolyse z. B. 0,1 n bis 1 n Kalium-, Natrium- oder Lithiumhydroxid oder auch Natrium- oder Kaliumcarbonat, die gegebenenfalls in Form wässriger Lösungen der alkoholischen Lösung der Verbindung der Formel IV zugesetzt werden.
ii) Weiterhin können die Verbindungen der Formel I dadurch erhalten werden, daß man eine Verbindung der Formel

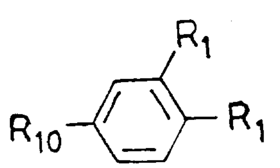

V

worin $R_1$ die gleiche Bedeutung wie oben hat und $R_{10}$ für ein Brom- oder Jodatom steht, mit einer Verbindung der Formel

$R_2$-C≡CH VI

worin $R_2$ die gleiche Bedeutung wie oben hat,
in Gegenwart eines bei etwa 0° - 80° C flüssigen sekundären oder tertiären Amins, insbesondere eines Dialkyl- oder Trialkylamins mit 2 oder 3 Kohlenstoffatomen in jedem der Alkylreste, Pyrrolidin oder Piperidin unter Zugabe katalytischer Mengen (d. h. etwa 0,01 bis 5 Prozent pro Mol eingesetzter

Verbindung der Formel VI eines komplexen Palladiumkatalysators, insbesondere Bis-(triphenylphosphin)-palladium(II)-chlorid oder -acetat oder Tetrakis-(triphenylphosphin)-palladium und gegebenenfalls unter Zusatz katalytischer Mengen Kupfer(I)-jodid bei etwa 20° - 80° C zur Reaktion bringt.

Die Herstellung der Ausgangsmaterialien der Formel II bzw. V erfolgt in an sich bekannter Weise.

So lassen sich die Verbindungen der Formel II in guter Ausbeute dadurch erhalten, daß man eine Verbindung der Formel VI mit einer Verbindung der Formel

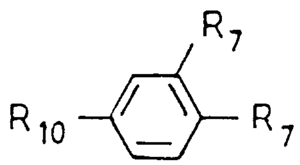

VII

in der $R_7$ und $R_{10}$ die gleiche Beutung wie oben haben, oder eine Verbindung der Formel

$R_2$-$R_{10}$ VIII

mit einer Verbindung der Formel

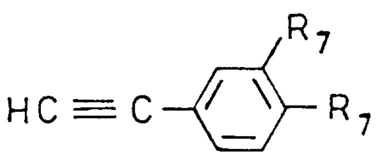

IX

worin $R_2$, $R_7$ und $R_{10}$ die gleiche Bedeutung wie oben haben, unter den für die Umsetzung der Verbindungen der Formeln V und VI genannten Bedingungen zur Reaktion bringt.

Die Herstellung der Ausgangsverbindung der Formel V kann z. B. durch Umsetzung einer Verbindung der Formel VII mit Hydroxylamin oder einem von dessen Salzen, anschließende Reduktion des gebildeten Oxims zu der entsprechenden Hydroxylaminverbindung und Einführung des Restes -$COR_4$ analog der oben geschilderten Überführung der Verbindung der Formel II in die Verbindung der Formel I erfolgen.

Die folgenden Beispiele dienen zur näheren Erläuterung der Erfindung. Bei ihrer Durchführung wurde auf die Erzielung maximaler Ausbeuten kein Wert gelegt.

Alle Temperaturangaben sind unkorrigiert. Die

$^1$H-NMR-Spektren wurden bei 300 MHz mit dem Gerät AC 300 der Firma Bruker gemessen.

Die chemische Verschiebung der spektroskopischen Resonanzdaten wurde in ppm gemessen.

Soweit nicht anders angegeben, handelt es sich bei dem in den Beispielen benutzten Petrolether um Petrolether mit dem Siedebereich 50° - 70° C, bei dem benutzten Ether um den Diethylether.

Als stationäre Phase für die Säulenchromatographie wurde - sofern nicht anders erwähnt - Kieselgel 60 (0,040 - 0,063 mm) der Firma E. Merck, Darmstadt, eingesetzt.

Die dünnschichtchromatographischen Untersuchungen wurden mit "HPTLC Fertigplatten, Kieselgel 60 F 254" der Firma E. Merck, Darmstadt, durchgeführt.

Die Mischungsverhältnisse der Laufmittel für alle chromatographischen Untersuchungen sind stets in Volumen/Volumen angegeben.

Beispiel 1

N-[3-(Naphth-2-yl-ethinyl)-benzyl]-acetohydroxamsäure

a) 3-(Naphth-2-yl-ethinyl)-benzaldehyd

Eine Lösung von 15,3 g Naphth-2-yl-acetylen und 16,65 g 3-Brombenzaldehyd in 120 ml absolutem Triethylamin wird bei 50°C Badtemperatur unter Rühren und Überleiten von trockenem Stickstoff mit 1,05 g Bis-(triphenylphosphin)-palladium(II)-chlorid und 0,07 g Kupfer(I)-jodid versetzt. Man rührt bei 70-80°C Badtemperatur und verfolgt den Reaktionsverlauf dünnschichtchromatographisch (Petrolether/Ether

- 5:1). Nach beendeter Umsetzung wird vom Ungelösten abfiltriert, mit Essigsäureethylester gewaschen und das Filtrat im Vakuum eingedampft. Der Rückstand wird durch Säulenchromatographie mit Petrolether/Ether (4:1) gereinigt. Man erhält so 15,1 g (65,5 % d. Th.) der Titelverbindung in Form von Kristallen, die bei 90-92 °C schmelzen.

$^1$H-NMR ($CDCl_3$) : 7,50 - 7,61 (m, 4H, aromat.); 7,81 - 7,88 (m, 5H, aromat.); 8,08 (s, 2H, aromat.); 10,02 (s, 1H, CHO)

b) 3-(Naphth-2-yl-ethinyl)-benzaldehydoxim

Eine Lösung von 15 g des in Beispiel 1a angefallenen Produkts in 80 ml Methanol wird mit einer Lösung von 6,08 g Hydroxylamin-hydrochlorid und 5,63 g Natrium acetat in 80 ml Wasser versetzt. Das Gemisch wird bei 60 °C Badtemperatur unter DC-Kontrolle (Petrolether/Ether - 4:1) gerührt. Nach vollständiger Umsetzung wird Methanol abgedampft und der wässrige Rückstand zweimal mit Essigsäureethylester extrahiert. Die Extrakte wäscht man je einmal mit Wasser und einer gesättigten Natriumchloridlösung. Nach Trocknen über Natriumsulfat und Eindampfen im Vakuum bleiben 15,4 g (97,2 % d. Th.) der Titelverbindung als sandfarbenes Pulver zurück (Isomerengemisch). E-Isomer:

$^1$H-NMR ($CDCl_3$): 7,34 - 7,43 (m, 1H, aromat.); 7,51 - 7,61 (m, 5H, aromat.); 7,78 - 7,86 (m, 4H, aromat.); 8,07 (s, 1H, aromat.); 8,12 (s, 1H, N=CH)

c) N-[3-(Naphth-2-yl-ethinyl)-benzyl]-acetohydroxamsäure

Eine Lösung von 15,3 g des in Beispiel 1b angefallenen Produkts in 170 ml Eisessig wird bei 50-55 °C Badtemperatur unter Rühren und Überleiten von trockenem Stickstoff portionsweise mit 4,45 g Natriumcyanoborhydrid versetzt. Man rührt noch eine Stunde nach, läßt auf Raumtemperatur abkühlen, versetzt mit 13,4 ml Essigsäureanhydrid und rührt zwölf Stunden. Dann wird im Vakuum eingedampft und der Rückstand mit Wasser verdünnt. Man extrahiert dreimal mit Essigsäureethylester, wäscht die Extrakte je zweimal mit gesättigten Lösungen von Natriumhydrogencarbonat bzw. Natriumchlorid und trocknet über Natriumsulfat. Nach Eindampfen wird aus dem Rückstand durch Chromatographie mit Petrolether/Ether (1:1) das zweifach acetylierte Zwischenprodukt isoliert. Dieses wird in 100 ml Methanol gelöst, die Lösung mit 100 ml einer 10%igen wässrigen Natriumcarbonatlösung versetzt und das Gemisch unter Rühren zwei Stunden auf 60 °C Badtemperatur erwärmt. Dann wird im Vakuum eingedampft und der Rückstand aus Essigsäureethylester/n-Hexan umkristallisiert. Man erhält so 11,14 g (62,6 % d. Th.) der Titelverbindung in Form farbloser Kristalle, die bei 144 - 146 °C schmelzen.

$^1$H-NMR (DMSO-$d_6$): 2,21 (s, 3H, $NCOCH_3$); 4,81 (s, 2H, $NCH_2$); 7,34 - 7,36 (m, 2H, aromat.); 7,50 - 7,58 (m, 5H, aromat.); 7,81 - 7,84 (m, 3H, aromat.); 8,05 (s, 1H, aromat.)

## Beispiel 2

Unter Anwendung der in Beispiel 1 beschriebenen Reaktionsfolge und -bedingungen erhält man analog:

a) N-{1-[4-(Phenylethinyl)-phenyl]-ethyl}-acetohydroxamsäure

Schmelzpunkt : 137 - 138 °C

$^1$H-NMR (DMSO-$d_6$): 1,45, 1,48 (d, 3H, $NCCH_3$); 2,03 (s, 3H, $NCOCH_3$); 5,58 - 5,72 (m, 1H, NCH); 7,32 - 7,58 (m, 9H, aromat.)

b) N-[3-(Naphth-1-yl-ethinyl)-benzyl]-acetohydroxamsäure

Schmelzpunkt : 98 - 100 °C

$^1$H-NMR (DMSO-$d_6$): 2,08 (s, 3H, $NCOCH_3$); 4,75 (s, 2H, $NCH_2$); 7,32 - 7,74 (m, 7H, aromat.); 7,83, 7,85 (d, 1H, aromat.); 7,95 - 8,05 (m, 2H, aromat.); 8,35, 8,38 (d, 1H, aromat.)

c) N-{1-[3-(4-Trifluormethyl-phenylethinyl)-phenyl]-ethyl}-acetohydroxamsäure

Schmelzpunkt : 97 - 98 °C

$^1$H-NMR (DMSO-$d_6$): 1,47, 1,49 (d, 3H, $NCCH_3$); 2,03 (s, 3H, $NCOCH_3$); 5,58 - 5,69 (m, 1H, NCH); 7,40 - 7,53 (m, 4H, aromat.); 7,78 (s, 4H, aromat.)

d) N-{1-[3-(2,4,6-Trimethyl-phenylethinyl)-phenyl]-ethyl}-acetohydroxamsäure

Schmelzpunkt : 45 - 47 °C

$^1$H-NMR (DMSO-$d_6$): 1,46, 1,49 (d, 3H, $NCCH_3$); 2,03 (s, 3H, $NCOCH_3$); 2,27 (s, 3H, $CH_3$); 2,42 (s, 6H, $CH_3$); 5,57 - 5,68 (m, 1H, NCH); 6,95 (s, 2H, aromat.); 7,32 - 7,45 (m, 4H, aromat.)

Beispiel 3

N-[3-(Phenylethinyl)-benzyl]-acetohydroxamsäure

a) 3-(Phenylethinyl)-benzaldehydoxim

Man verführt analog Beispiel 1b und erhält aus 16,5 g 3-(Phenylethinyl)-benzaldehyd (CA 109:P 23557v - 1988), 8,3 g Hydroxylaminhydrochlorid und 8,2 g Natriumacetat 16,8 g (94,8 % d. Th.) der Titelverbindung als gelbliches Pulver (Isomerengemisch).

b) N-[3-(Phenylethinyl)-benzyl]-acetohydroxamsäure

Analog Beispiel 1c werden 11,95 g des in Beispiel 3a erhaltenen Produkts zunächst mit 5,09 g Natriumcyanoborhydrid, dann mit 12,8 ml Essigsäureanhydrid zur Reaktion gebracht. Aus der so angefallenen Zwischenverbindung erhält man analog Beispiel 1c 9,48 g (66,2 % d. Th.) der Titelverbindung in Form farbloser Kristalle, die bei 91 - 93 °C schmelzen.

$^1$H-NMR (DMSO-$d_6$): 2,08 (s, 3H, $NCOCH_3$); 4,72 (s, 2H, $NCH_2$); 7,30 - 7,37 (m, 1H, aromat.); 7,40 - 7,48 (m, 6H, aromat.); 7,54 - 7,58 (m, 2H, aromat.)

Das Produkt aus Beispiel 3b kann man auch auf folgendem Weg erhalten:

i) 3-Brombenzyl-acetohydroxamsäure

4,0 g 3-Brombenzaldehydoxim werden analog Beispiel 1c mit 1,80 g Natriumcyanoborhydrid und 3,8 ml Essigsäureanhydrid umgesetzt. Nach Behandeln mit wässriger Natriumcarbonatlösung fallen 3,32 g (67,9 % d. Th.) der Titelverbindung als nahezu farbloses Pulver an (Schmelzpunkt: 63 - 64 °C).

$^1$H-NMR ($CDCl_3$): 2,18 (s, 3H, $NCOCH_3$); 4,78 (s, 2H, $NCH_2$); 7,20 - 7,50 (m, 4H, aromat.);

ii)N-[3-(Phenylethinyl)-benzyl]-acetohydroxamsäure

Durch Umsetzung analog Beispiel 1a von 2,20 g des in Beispiel 3i angefallenen Produkts und 1,65 ml Phenylacetylen in 30 ml absolutem Triethylamin unter der katalytischen Wirkung von 0,21g Bis-(triphenylphosphin)-palladium(II)-chloridund 0,02 g Kupfer(I)-jodid erhält man nach HPLC-Reinigung mit Methanol/Wasser (4:1) und Umkristallisation aus Essigsäureethylester/n-Hexan 0,98 g (41,2 % d. Th.) der Titelverbindung, deren physikalische und spektroskopische Daten mit denen der nach 3b hergestellten Verbindung übereinstimmen.

Beispiel 4

N-[3-(4-Methoxy-phenylethinyl)-benzyl]-acetohydroxamsäure

a) 3-(4-Methoxy-phenylethinyl)-benzaldehyd

4-Methoxy-phenylacetylen setzt man analog Beispiel 1a mit 3-Brom-benzaldehyd um und erhält so die Titelverbindung in Form weißer Kristalle, die bei 52-53 °C schmelzen.

$^1$H-NMR ($CDCl_3$): 3,84 (s, 3H, $OCH_3$); 6,88 - 6,91 (m, 2H, aromat.); 7,44 - 7,53 (m, 3H, aromat.); 7,73 - 7,76 (m, 1H, aromat.); 7,80 - 7,83 (m, 1H, aromat.); 8,00 - 8,02 (m, 1H, aromat.); 10,02 (s, 1H, CHO)

b) 3-(4-Methoxy-phenylethinyl)-benzaldehydoxim

Man verfährt analog Beispiel 1 b, verwendet aber eine Lösung des in Beispiel 4a angefallenen Produkts in Ethanol sowie Pyridin (statt Natriumacetat) als Base und fügt dann Hydroxylamin-hydrochlorid zu. Das nach Aufarbeitung erhaltene Rohprodukt wird durch Chromatographie mit Petrolether/Essigsäureethylester (3:1) gereinigt. Dabei fallen 10,67 g (93,8 % d. Th.) der Titelverbindung in Form hellgelb gefärbter Kristalle an, die bei 90-91 °C schmelzen.

$^1$H-NMR ($CDCl_3$): 3,83 (s, 3H, $OCH_3$); 6,87 - 6,90 (m, 2H, aromat.); 7,33 - 7,38 (m, 1H, aromat.); 7,45 - 7,55 (m, 4H, aromat.); 7,70, 7,71 (d, 1H, aromat.); 8,12 (s, 1H, N=CH)

c) N-Acetoxy-N-[3-(4-methoxy-phenylethinyl)-benzyl]-acetamid

Analog Beispiel 1 c setzt man 10,23 g des in Beispiel 4b erhaltenen Produkts in 120 ml Eisessig mit 3,84 g Natriumcyanoborhydrid um, fügt 8,8 ml Essigsäureanhydrid zu und arbeitet nach Vervollständigung der Reaktion auf. Das Rohprodukt wird durch Chromatographie mit Essigsäureethylester/Petrolether (1:1) gereinigt, wobei man 9,98 g (72,2 % d. Th.) der Titelverbindung in Form eines farblosen Öls erhält.

$^1$H-NMR ($CDCl_3$): 2,13 (s, 3H, $NCOCH_3$); 2,20 (s, 3H, $NOCOCH_3$); 3,82 (s, 3H, $OCH_3$); 4,86 (s, 2H, $NCH_2$); 6,85 - 6,90 (m, 2H, aromat.); 7,22 - 7,34 (m, 2H, aromat.); 7,42 - 7,49 (m, 4H, aromat.)

d) N-[3-(4-Methoxy-phenylethinyl)-benzyl]-acetohydroxamsäure

1,0 g des in Beispiel 4c erhaltenen Produkts wird in 10 ml Methanol gelöst und nach Zusatz von 0,205 g

wasserfreiem Kaliumcarbonat bei Raumtemperatur verrührt. Sobald die Reaktion beendet ist - DC-Kontrolle mit Essigsäureethylester/Petrolether (1:1) - wird mit 5%iger Salzsäure angesäuert und mit Essigsäureethylester verdünnt. Die organische Phase wird mit Wasser nachgewaschen, dann wird im Vakuum eingedampft. Das zurückbleibende Öl kristallisiert beim Trocknen über Phosphorpentoxid im Exsikkator. Man erhält so 0,853 g (97,6 % d. Th.) der Titelverbindung in Form weißer Kristalle, die bei 124-126 °C schmelzen.

$^1$H-NMR ($CDCl_3$): 2,17 (s, 3H, $NCOCH_3$); 3,82 (s, 3H, $OCH_3$); 4,79 (s, 2H, $NCH_2$); 6,86 - 6,89 (m, 2H, aromat.); 7,24 - 7,55 (m, 6H, aromat.)

Beispiel 5

Unter Anwendung der im Beispiel 4 beschriebenen Verfahrensweisen erhält man analog:

a) N-{1-[3-(4-Methoxy-phenylethinyl)-phenyl]-ethyl}-acetohydroxamsäure

Farbloses zähes Öl

$^1$H-NMR (DMSO-$d_6$): 1,46, 1,48 (d, 3H, $NCCH_3$); 2,03 (s, 3H, $NCOCH_3$); 3,79 (s, 3H, $OCH_3$); 5,61 - 5,63 (m, 1H, NCH); 6,95 - 6,99 (m, 2H, aromat.); 7,32 - 7,52 (m, 6H, aromat.)

b) N-{1-[3-(3,4-Dimethoxy-phenylethinyl)-phenyl]-ethyl}-acetohydroxamsäure

Schmelzpunkt : 107 - 108 °C

$^1$H-NMR (DMSO-$d_6$): 1,45, 1,48 (d, 3H, $NCCH_3$); 2,02 (s, 3H, $NCOCH_3$); 3,79 (s, 3H, $OCH_3$); 3,80 (s, 3H, $OCH_3$); 5,53 - 5,69 (m, 1H, NCH); 6,96 - 6,99 (m, 1H, aromat.); 7,10 - 7,14 (m, 2H, aromat.); 7,31 - 7,45 (m, 4H, aromat.)

c) N-{1-[3-(3-Methoxy-phenylethinyl)-phenyl]-ethyl}-acetohydroxamsäure

Farbloses zähes Öl

$^1$H-NMR ($CDCl_3$): 1,53 - 1,56 (d, 3H, $NCCH_3$); 2,15 (s, 3H, $NCOCH_3$); 3,82 (s, 3H, $OCH_3$); 4,99 - 5,18 und 5,78 - 5,89 (m, 1H, NCH); 6,88 - 6,92 (m, 1H, aromat.); 7,05 - 7,14 (m, 2H, aromat.); 7,23 - 7,54 (m, 5H, aromat.)

d) N-{1-[4-(3,4-Dimethoxy-phenylethinyl)-phenyl]-ethyl}-acetohydroxamsäure

Schmelzpunkt : 123 - 124 °C

$^1$H-NMR (DMSO-$d_6$): 1,45, 1,47 (d, 3H, $NCCH_3$); 2,03 (s, 3H, $NCOCH_3$); 3,79 (s, 3H, $OCH_3$); 3,80 (s, 3H, $OCH_3$); 5,62 - 5,64 (m, 1H, NCH); 6,95 - 6,98 (m, 1H, aromat.); 7,07 - 7,12 (m, 2H, aromat.); 7,32 - 7,35 (m, 2H, aromat.); 7,45 - 7,48 (m, 2H, aromat.)

e) N-[3-(4-Isopropyl-phenylethinyl)-benzyl]-acetohydroxamsäure

Schmelzpunkt : 120 - 122 °C

$^1$H-NMR (DMSO-$d_6$): 1,20 - 1,23 (d, 6H, $C(CH_3)_2$); 2,07 (s, 3H, $NCOCH_3$); 2,87 - 2,96 (m, 1H, $CH(C)_2$); 4,70 (s, 2H, $NCH_2$); 7,26 - 7,48 (m, 8H, aromat.)

f) N-{1-[3-(4-Isopropyl-phenylethinyl)-phenyl]-ethyl}-acetohydroxamsäure

Farbloses zähes Öl

$^1$H-NMR (DMSO-$d_6$): 1,20, 1,22 (d, 6H, $C(CH_3)_2$); 1,46, 1,48 (d, 3H, $NCCH_3$); 2,02 (s, 3H, $NCOCH_3$); 2,87 - 2,97 (m, 1H, $CH(C)_2$); 5,55 - 5,69 (m, 1H, NCH); 7,27 - 7,49 (m, 8H, aromat.)

g) N-{1-[3-(4-Methoxy-naphth-1-yl-ethinyl)-phenyl]-ethyl}-acetohydroxamsäure

Schmelzbereich : 70 - 75 °C

$^1$H-NMR (DMSO-$d_6$): 1,51, 1,53 (d, 3H, $NCCH_3$); 2,07 (s, 3H, $NCOCH_3$); 4,03 (s, 3H, $OCH_3$); 5,63 - 5,76 (m, 1H, NCH); 7,01, 7,04 (d, 1H, aromat.); 7,38 - 7,45 (m, 2H, aromat.); 7,55 - 7,80 (m, 5H, aromat.); 8,23 - 8,35 (m, 2H, aromat.)

h) N-{1-[4-(4-Methoxy-naphth-1-yl-ethinyl)-phenyl]-ethyl}-aceto-hydroxamsäure

Schmelzpunkt: 139-142 °C

$^1$H-NMR (DMSO-$d_6$): 1,47, 1,49 (d, 3H, $NCCH_3$); 2,03 (s, 3H, $NCOCH_3$); 4,03 (s, 3H, $OCH_3$); 5,59 - 5,72 (m, 1H, NCH); 7,01, 7,04 (d, 1H, aromat.); 7,37 - 7,40 (m, 2H, aromat.); 7,59 - 7,77 (m, 5H, aromat.); 8,21, 8,24 (d, 1H, aromat.); 8,29, 8,30 (d, 1H, aromat.)

i) N-{1-[3-(2-Methoxy-phenylethinyl)-phenyl]-ethyl}-acetohydroxamsäure

viskoses Material

$^1$H-NMR (DMSO-$d_6$): 1,46, 1,48 (d, 3H, $NCCH_3$); 2,03 (s, 3H, $NCOCH_3$); 3,86 (s, 3H, $OCH_3$); 5,57 - 5,70 (m, 1H, NCH); 6,94 - 6,99 (m, 1H, aromat.); 7,06 - 7,08 (d, 1H, aromat.); 7,35 - 7,49 (m, 6H, aromat.)

k) N-{1-[3-(2-Methoxy-napth-1-yl-ethinyl)-phenyl]-ethyl}-acetohydroxamsäure

Schmelzpunkt: 138 - 140 °C

$^1$H-NMR (DMSO-$d_6$): 1,49, 1,51 (d, 3H, $NCCH_3$); 2,04 (s, 3H, $NCOCH_3$); 4,02 (s, 3H, $OCH_3$); 5,65 - 5,67 (m , 1H, NCH); 7,37 - 7,64 (m, 7H, aromat.); 7,91, 7,94 (d, 1H, aromat.); 8,00, 8,03 (d, 1H, aromat.); 8,21, 8,24 (d, 1H, aromat.)

1) N-{1-[3-(3,4,5-Trimethoxy-phenylethinyl)-phenyl]-ethyl}-acetohydroxamsäure

Schmelzpunkt: 145 °C

1H-NMR (DMSO-$d_6$): 1,46, 1,48 (d, 3H, $NCCH_3$); 2,03 (s, 3H, $NCOCH_3$); 3,70 (s, 3H, $OCH_3$); 3,82 (s, 6H, $OCH_3$); 5,58 - 5,63 (m, 1H, NCH); 6,85 (s, 2H, aromat.); 7,35 - 7,47 (m, 4H, aromat.)

m) N-{1-[3-(4-Methylphenylethinyl)-phenyl]-ethyl}-acetohydroxamsäure

viskoses Material

$^1$H-NMR (DMSO-$d_6$): 1,45, 1,48 (d, 3H, $NCCH_3$); 2,02 (s, 3H, $NCOCH_3$); 2,34 (s, 3H, $CH_3$); 5,61 - 5,63 (m, 1H, NCH); 7,22 - 7,25 (m, 2H, aromat.); 7,33 - 7,46 (m, 6H, aromat.)

n) N-[3-(2-Methoxy-phenylethinyl)-benzyl]-acetohydroxamsäure

zähes Öl

$^1$H-NMR (DMSO-$d_6$): 2,19 (s, 3H, $NCOCH_3$); 3,98 (s, 3H, $OCH_3$); 4,83 (s, 2H, $NCH_2$); 7,08 - 7,13, (t, 1H, aromat.); 7,20, 7,23 (d, 1H, aromat.); 7,41 - 7,63 (m, 6H, aromat.)

o) N-[3-(4-Chlorphenylethinyl)-benzyl]-acetohydroxamsäure

Schmelzpunkt: 149 °C

$^1$H-NMR (DMSO-$d_6$): 2,06 (s, 3H, $NCOCH_3$); 4,70 (s, 2H, $NCH_2$); 7,31 - 7,60 (m, 8H, aromat.)

Beispiel 6

N-Hydroxy-N-[3-(naphth-2-yl-ethinyl)-benzyl]-harnstoff

2,07 g des Produkts aus Beispiel 1b in 20 ml Eisessig werden analog Beispiel 1c mit 0,60 g Natriumcyanoborhydrid zu dem entsprechenden Hydroxylamin reduziert. Dieses wird in 25 ml absolutem 1,4-Dioxan gelöst, die Lösung mit 2,1 ml Trimethylsilyl-isocyanat versetzt und drei Stunden zum Rückfluß erhitzt. Nach dem Abkühlen verdünnt man mit 50 ml Essigsäureethylester, fügt gesättigte Ammoniumchloridlösung zu und trennt die organische Schicht ab. Diese wird mit gesättigter Ammoniumchloridlösung und zweimal mit gesättigter Natriumchloridlösung gewaschen, über Natriumsulfat getrocknet und dann im Vakuum eingedampft. Der ölige Rückstand wird durch Chromatographie mit Essigsäureethylester/Methanol (25:1) gereinigt und das Produkt aus Essigsäureethylester/ n-Hexan umkristallisiert. Man erhält so 1,45 g (61,0 % d. Th.) der Titelverbindung in Form farbloser Kristalle, die bei 184-186 °C schmelzen.

$^1$H-NMR (DMSO-$d_6$): 4,57 (s, 2H, $NCH_2$); 6,44 (s, 2H, $NH_2$); 7,32 - 7,44 (m, 2H, aromat.); 7,48 - 7,65 (m, 5H, aromat.); 7,95 - 7,99 (m, 3H, aromat.); 8,21 (s, 1H, aromat.)

Beispiel 7

N-Hydroxy-N-[3-(napth-1-yl-ethinyl)-benzyl]-harnstoff

5,43 g 3-(Naphth-1-yl-ethinyl)-benzaldehydoxim werden wie in Beispiel 1c beschrieben mit 1,96 g Natriumcyanoborhydrid umgesetzt. Das so erhaltene Hydroxylamin wird in 60 ml absolutem Tetrahydrofuran gelöst, die Lösung mit 4 ml Trimethylsilyl-isocyanat versetzt und vier Stunden bei 50 °C Badtemperatur gerührt. Nach Aufarbeitung und Reinigung analog Beispiel 6 fallen 3,78 g (59,7 % d. Th.) der Titelverbindung in Form farbloser Kristalle an, die bei 170-172 °C schmelzen.

$^1$H-NMR (DMSO-$d_6$): 4,59 (s, 2H, $NCH_2$); 6,44 (s, 2H, $NH_2$); 7,35 - 7,46 (m, 2H, aromat.); 7,55 - 7,73 (m, 5H, aromat.); 7,80 - 7,84 (m, 1H, aromat.); 7,98 - 8,02 (m, 2H, aromat.); 8,35 - 8,39 (m, 1H, aromat.)

Beispiel 8

Unter Anwendung der in den Beispielen 1 und 7 beschriebenen Verfahrensweisen bzw. Reaktionsbedingungen erhält man analog:

a) N-Hydroxy-N-{1-[3-(4-methoxy-naphth-1-yl-ethinyl)-phenyl]-ethyl}-harnstoff

Schmelzpunkt : 162 - 164 °C

$^1$H-NMR (DMSO-$d_6$): 1,45, 1,47 (d, 3H, $NCCH_3$); 4,03 (s, 3H, $OCH_3$); 5,29 - 5,40 ( m, 1H, NCH);

6,37 (s, 2H, $NH_2$); 7,02, 7,05 (d, 1H, aromat.): 7,38 - 7,40 (m, 2H, aromat.); 7,50 - 7,80 (m, 5H, aromat.); 8,22 - 8,33 (m, 2H, aromat.)

b) N-Hydroxy-N-{1-[3-(4-fluor-phenylethinyl)-phenyl]-ethyl}-harnstoff

Schmelzpunkt: 143 - 144 °C

$^1$H-NMR (DMSO-$d_6$): 1,42, 1,44 (d, 3H, $NCCH_3$); 5,26 - 5,36 (m, 1H, NCH); 7,22 - 7,28 (m, 2H, aromat.); 7,32 - 7,43 (m, 3H, aromat.); 7,51 (s, 1H, aromat.); 7,59 - 7,64 (m, 2H, aromat.);

c) N-Hydroxy-N-{1-[3-(3,4-methylendioxy-phenylethinyl)-phenyl]-ethyl}-harnstoff

Schmelzpunkt: 153 - 155 °C

$^1$H-NMR (DMSO-$d_6$): 1,42, 1,44 (d, 3H, $NCCH_3$); 5,26 - 5,35 (m, 1H, NCH); 6,08 (s, 2H, $OCH_2O$); 6,34 (s, 2H, $NH_2$); 6,93 - 6,96 (m, 1H, aromat.); 7,06 - 7,10 (m, 2H, aromat.); 7,30 - 7,39 (m, 3H, aromat.); 7,48 (s, 1H, aromat.)

d) N-Hydroxy-N-{1-[3-(4-tert-butyl-phenylethinyl)-phenyl]-ethyl}-harnstoff

Schmelzpunkt: 74 - 76 °C

$^1$H-NMR (DMSO-$d_6$): 1,30 (s, 9H, t.-$C_4H_9$); 1,43, 1,45 (d, 3H, $NCCH_3$); 5,28 - 5,36 (m, 1H, NCH); 6,35 (s, 2H, $NH_2$); 7,31 - 7,55 (m, 8H, aromat.)

e) N-Hydroxy-N-{1-[3-(2,4,6-trimethyl-phenylethinyl)-phenyl]-ethyl}-harnstoff

Schmelzpunkt: 147 - 149 °C

$^1$H-NMR (DMSO-$d_6$): 1,43, 1,45 (d, 3H, $NCCH_3$); 2,26 (s, 3H, $CH_3$); 2,42 (s, 6H, $CH_3$); 5,28 - 5,36 (m, 1H, NCH); 6,35 (s, 2H, $NH_2$); 6,94 (s, 2H, aromat.); 7,31 - 7,42 (m, 3H, aromat.); 7,49 (s, 1H, aromat.)

f) N-Hydroxy-N-[3-(4-fluorphenylethinyl)-benzyl]-harnstoff

Schmelzpunkt: 166 - 168 °C

$^1$H-NMR (DMSO-$d_6$): 4,55 (s, 2H, $NCH_2$); 6,42 (s, 2H, $NH_2$); 7,22 - 7,47 (m, 6H, aromat.); 7,59 - 7,64 (m, 2H, aromat.)

g) N-Hydroxy-N-[3-(4-fluor-naphth-1-yl-ethinyl)-benzyl]-harnstoff

Schmelzpunkt: 168 - 170 °C

$^1$H-NMR (DMSO-$d_6$); 4,61 (s, 2H, $NCH_2$); 6,47 (s, 2H, $NH_2$); 7,37 - 7,47 (m, 3H, aromat.); 7,58 - 7,63 (m, 2H, aromat.); 7,72 - 7,86 (m, 3H, aromat.); 8,13, 8,16 (d, 1H, aromat.); 8,40, 8,43 (d, 1H, aromat.)

h) N-Hydroxy-N-{1-[3-(3,4-dichlorphenylethinyl)-phenyl]-ethyl}-harnstoff

Schmelzpunkt: 151 - 152 °C

$^1$H-NMR (DMSO-$d_6$): 1,43, 1,45 (d, 3H, $NCCH_3$), 5,29 - 5,36 (m, 1H, NCH); 6,35 (s, 2H, $NH_2$); 7,34 - 7,45 (m, 3H, aromat.); 7,52 - 7,56 (m, 2H, aromat.); 7,65, 7,68 (d, 1H, aromat.); 7,83, 7,84 (d, 1H, aromat.)

Beispiel 9

N-Hydroxy-N-{1-[3-(4-methoxy-phenylethinyl)-phenyl]-ethyl}-harnstoff

Eine Lösung von 1,0 g 3-(4-Methoxy-phenylethinyl)-acetophenonoxim (hergestellt analog Beispiel 4a/b) in 10 ml Eisessig wird analog Beispiel 1 c mit 0,355 g Natriumcyanoborhydrid reduziert. Das nach Aufarbeitung gewonnene Hydroxylamin wird in 10 ml trockenem Tetrahydrofuran aufgenommen und tropfenweise mit 0,82 ml Trimethylsilyl-isocyanat versetzt. Nach Vervollständigung der Reaktion wird mit Essigsäureethylester verdünnt, gesättigte Ammoniumchloridlösung zugefügt, die organische Schicht abgetrennt und mit Wasser gewaschen. Das nach Eindampfen angefallene Rohprodukt wird mit Essigsäureethylester verrührt. Beim Absaugen erhält man so 0,851 g (72,8 % d. Th.) der Titelverbindung in Form weißer Kristalle, die bei 141-142 °C schmelzen.

$^1$H-NMR (DMSO-$d_6$): 1,41, 1,43 (d, 3H, $NCCH_3$); 3,79 (s, 3H, $OCH_3$); 5,27 - 5,34 (m, 1H, NCH); 6,34 (s, 2H, $NH_2$); 6,95 - 6,98 (m, 2H, aromat.); 7,29 - 7,37 (m, 2H, aromat.); 7,38 - 7,39 (m, 2H, aromat.); 7,47 - 7,50 (m, 2H, aromat.)

Beispiel 10

Man verfährt analog Beispiel 9 und erhält aus den entsprechenden Ausgangsverbindungen:

a) N-Hydroxy-N-{1-[4-(3,4-dimethoxy-phenylethinyl)-phenyl]-ethyl}-harnstoff

Schmelzpunkt : 167 °C

$^1$H-NMR (DMSO-$d_6$): 1,40, 1,42 (d, 3H, $NCCH_3$); 3,79 (s, 3H, $OCH_3$); 3,80 (s, 3H, $OCH_3$); 5,28 -

5,32 (m, 1H, NCH); 6,33 (s, 2H, $NH_2$); 6,96 - 6,99 (m, 1H, aromat.); 7,07 - 7,12 (m, 2H, aromat.); 7,34 - 7,37 (m, 2H, aromat.); 7,37 - 7,46 (m, 2H, aromat.)

b) N-Hydroxy-N-{1-[3-(3,4-dimethoxy-phenylethinyl)-phenyl]-ethyl}-harnstoff

Schmelzpunkt : 144 - 146 °C
$^1$H-NMR (DMSO-$d_6$): 1,41, 1,44 (d, 3H, $NCCH_3$); 3,79 (s, 3H, $OCH_3$); 3,80 (s, 3H, $OCH_3$); 5,27 - 5,32 (m, 1H, NCH); 6,33 (s, 2H, $NH_2$); 6,96 - 6,98 (m, 1H, aromat.); 7,10 - 7,14 (m, 2H, aromat.); 7,32 - 7,36 (m, 3H, aromat.); 7,48 (s, 1H, aromat.)

c) N-Hydroxy-N-{1-[3-(3-methoxy-phenylethinyl)-phenyl]-ethyl}-harnstoff

Schmelzpunkt : 128 - 130 °C
$^1$H-NMR (DMSO-$d_6$): 1,42, 1,44 (d, 3H, $NCCH_3$); 3,79 (s, 3H, $OCH_3$); 5,30 - 5,34 (m, 1H, NCH); 6,34 (s, 2H, $NH_2$); 6,96 - 7,10 (m, 1H, aromat.); 7,11 - 7,13 (m, 2H, aromat.); 7,30 - 7,42 (m, 4H, aromat.); 7,51 (s, 1H, aromat.)

d) N-Hydroxy-N-{1-[3-(4-chlor-phenylethinyl)-phenyl]-ethyl}-harnstoff

Schmelzpunkt : 148 - 149 °C
$^1$H-NMR ($CDCl_3$): 1,42, 1,43 (d, 3H, $NCCH_3$); 5,28 - 5,34 (m, 1H, NCH); 6,34 (s, 2H, $NH_2$); 7,32 - 7,59 (m, 8H, aromat.)

e) N-Hydroxy-N-[3-(4-isopropyl-phenylethinyl)-benzyl]-harnstoff

Schmelzpunkt : 148 - 149 °C.
$^1$H-NMR (DMSO-$d_6$): 1,20, 1,24 (d, 6H, $C(CH_3)_2$); 2,87 - 2,96 (m, 1H, $CH(C)_2$); 4,55 (s, 2H, $NCH_2$); 6,39 (s, 2H, $NH_2$); 7,27 - 7,48 (m, 8H, aromat.)

f) N-Hydroxy-N-{1-[3-(6-methoxy-naphth-2-yl-ethinyl)-phenyl]-ethyl}-harnstoff

Schmelzpunkt : 150 - 152 °C
$^1$H-NMR (DMSO-$d_6$): 1,43, 1,45 (d, 3H, $NCCH_3$); 3,89 (s, 3H, $OCH_3$); 5,29 - 5,36 (m, 1H, NCH); 6,35 (s, 2H, $NH_2$); 7,19 - 7,23 (m, 1H, aromat.); 7,35 - 7,57 (m, 6H, aromat.); 7,82 - 7,87 (m, 2H, aromat.); 8,10 - 8,17 (m, 1H, aromat.)

g) N-Hydroxy-N-{1-[3-(2-methoxy-phenylethinyl)-phenyl]-ethyl}-harnstoff

viskoses Material
$^1$H-NMR (DMSO-$d_6$): 1,42, 1,44 (d, 3H, $NCCH_3$); 3,86 (s, 3H, $OCH_3$); 5,27 - 5,34 (m, 1H, NCH); 6,34 (s, 2H, $NH_2$); 6,94 - 6,99 (m, 1H, aromat.); 7,06 - 7,09 (m, 1H, aromat.); 7,30 - 7,41 (m, 4H, aromat.); 7,46 - 7,49 (m, 2H, aromat.)

h) N-Hydroxy-N-{1-[4-(4-methoxy-naphth-1-yl-ethinyl)-phenyl]-ethyl}-harnstoff

Schmelzpunkt: 153 - 154 °C
$^1$H-NMR (DMSO-$d_6$): 1,43, 1,45 (d, 3H, $NCCH_3$); 4,02 (s, 3H, $OCH_3$); 5,31 - 5,38 (m, 1H, NCH); 6,35 (s, 2H, $NH_2$); 7,01, 7,04 (d, 1H, aromat.); 7,40 - 7,43 (m, 2H, aromat.); 7,57 - 7,77 (m, 5H, aromat.); 8,21, 8,24 (d, 1H, aromat.); 8,29, 8,32 (d, 1H, aromat.)

i) N-Hydroxy-N-{1-[3-(4-methyl-phenylethinyl)-phenyl]-ethyl}-harnstoff

Schmelzpunkt : 142 - 143 °C
$^1$H-NMR (DMSO-$d_6$): 1,42, 1,44 (d, 3H, $NCCH_3$); 2,35 (s, 3H, $CH_3$); 5,26 - 5,35 (m, 1H, NCH); 6,35 (s, 2H, $NH_2$); 7,22, 7,25 (d, 2H, aromat.); 7,31 - 7,50 (m, 6H, aromat.)

k) N-Hydroxy-N-{1-[3-(2-methoxy-naphth-1-yl-ethinyl)-phenyl]-ethyl}-harnstoff

viskoses Material
$^1$H-NMR (DMSO-$d_6$): 1,44, 1,46 (d, 3H, $NCCH_3$); 4,02 (s, 3H, $OCH_3$); 5,30 - 5,37 (m, 1H, NCH); 6,38 (s, 2H, $NH_2$); 7,38 - 7,65 (m, 7H, aromat.); 7,92, 7,95 (d, 1H, aromat.); 8,00, 8,03 (d, 1H, aromat.); 8,21, 8,23 (d, 1H, aromat.)

l) N-Hydroxy-N-[3-(2-methoxyphenylethinyl)-benzyl]-harnstoff

Schmelzpunkt: 122 - 123,5 °C
$^1$H-NMR (DMSO-$d_6$): 3,86 (s, 3H, $OCH_3$); 4,54 (s, 2H, $CH_2$); 6,40 (s, 2H, $NH_2$); 6,94 - 6,99 (t, 1H, aromat.); 7,06, 7.09 (d, 1H, aromat.); 7,29 - 7,49 (m, 6H, aromat.)

m) N-Hydroxy-N-[3-(4-chlorphenylethinyl)-benzyl]-harnstoff

Schmelzpunkt: 165 - 166 °C
$^1$H-NMR (DMSO-$d_6$): 4,54 (s, 2H, $NCH_2$); 6,41 (s, 2H, $NH_2$); 7,35 - 7,59 (m, 8H aromat.)

n) N-Hydroxy-N-[3-(4-methoxyphenylethinyl)-benzyl]-harnstoff

Schmelzpunkt: 170 °C
$^1$H-NMR (DMSO-$d_6$): 3,79 (s, 3H, $OCH_3$); 4,54 (s, 2H, $NCH_2$); 6,40 (s, 2H, $NH_2$); 6,95 - 6,99 (m, 2H, aromat.); 7,28 - 7,50 (m, 6H, aromat.)

o) N-Hydroxy-N-{1-[3-(3,4,5-trimethoxyphenylethinyl)-phenyl]-ethyl}-harnstoff

Schmelzpunkt: 166,5 °C

$^1$H-NMR (DMSO-$d_6$): 1,42, 1,44 (d, 3H, $NCCH_3$); 3,70 (s, 3H, $OCH_3$); 3,82 (s, 6H, $OCH_3$); 5,28 - 5,30 (m, 1H, NCH); 6,33 (s, 2H, $NH_2$); 6,86 (s, 2H, aromat.); 7,31 - 7,41 (m, 3H, aromat.); 7,51 (s, 1H, aromat.)

p) N-Hydroxy-N-{1-[3-(phenylethinyl)-phenyl]-ethyl}-harnstoff

Schmelzpunkt: 136 - 137 °C

$^1$H-NMR (DMSO-$d_6$): 1,42, 1,44 (d, 3H, $NCCH_3$); 5,28 - 5,35 (m, 1H, NCH); 6,34 (s, 2H, $NH_2$); 7,32 - 7,57 (m, 9H, aromat.)

q) N-Hydroxy-N-[4-(2,6-dichlorphenylethinyl)-benzyl]-harnstoff

Schmelzpunkt: 157 °C

$^1$H-NMR (DMSO-$d_6$): 4,57 (s, 2H, $NCH_2$); 6,41 (s, 2H, $NH_2$); 7,36 - 7,45 (m, 3H, aromat.); 7,53 - 7,60 (m, 4H, aromat.)

Beispiel 11

N-Hydroxy-N-{1-[3-(4-trifluormethyl-phenylethinyl)-phenyl]-ethyl}-harnstoff

a) 3-Ethinyl-acetophenon

12,53 g 3-Brom-acetophenon und 11 ml Trimethylsilylacetylen werden analog Beispiel 1a in Gegenwart von 0,62 g Bis-(triphenyl-phosphin)-palladium (II)-chlorid und 0,05 g Kupfer(I)-jodid bei einer Reaktionstemperatur von 40-50 °C miteinander umgesetzt. Das durch Säulenchromatographie mit Petrolether/Ether (3:1) isolierte 3-(Trimethylsilyl-ethinyl)-acetophenon wird in 100 ml Methanol gelöst, die Lösung unter Eiswasserkühlung mit 65 ml 1N Natriumhydroxidlösung versetzt und eine Stunde gerührt. Dann versetzt man mit 250 ml gesättigter Ammoniumchloridlösung und extrahiert dreimal mit je 150 ml Ether. Die Extrakte werden zweimal mit einer gesättigten Natriumchloridlösung gewaschen und dann über Natriumsulfat getrocknet. Nach Eindampfen bleiben 8,60 g (96,2 % d. Th.) der Titelverbindung in Form eines schwach gelb gefärbten Feststoffs zurück, der bei 53 - 54 °C schmilzt.

$^1$H-NMR ($CDCl_3$): 2,61 (s, 3H, $COCH_3$); 3,14 (s, 1H, ≡ CH); 7,42 - 7,47 (m, 1H, aromat.); 7,66 - 7,70 (m, 1H, aromat.); 7,92 - 7,96 (m, 1H, aromat.); 8,07 - 8,08 (m, 1H, aromat.)

b) 3-(4-Trifluormethyl-phenylethinyl)-acetophenon

8,60 g des in Beispiel 11a erhaltenen Produkts werden mit 9,8 ml 4-Brombenzotrifluorid in Gegenwart von 0,63 g Bis-(triphenylphosphin)-palladium(II)-chlorid und 0,03 g Kupfer(I)-jodid analog Beispiel 1a umgesetzt. Nach Chromatographie mit Petrolether/Ether (2:1) erhält man 14,81 g (86,2 % d. Th.) der Titelverbindung in Form farbloser Kristalle mit einem Schmelzpunkt von 72 - 73 °C.

$^1$H-NMR ($CDCl_3$): 2,64 (s, 3H, $COCH_3$); 7,46 - 7,52 (m, 1H, aromat.); 7,61 - 7,67 (m, 4H, aromat.); 7,72 - 7,75 (m, 1H, aromat.); 7,94 - 7,98 (m, 1H, aromat.); 8,13 - 8,14 (m, 1H, aromat.)

c) 3-(4-Trifluormethyl-phenylethinyl)-acetophenonoxim

Man erhält 12,32 g (96,5 % d. Th.) der Titelverbindung mit Schmelzpunkt 117 - 119 °C durch Reaktion von 12,1 g des Produkts aus Beispiel 11b mit 4,90 g Hydroxylaminhydrochlorid und 4,55 g Natriumacetat analog Beispiel 1b.

$^1$H-NMR (DMSO-$d_6$): 2,19 (s, 3H, $N=CCH_3$); 7,45 - 7,50 (m, 1H, aromat.); 7,58 - 7,62 (m, 1H, aromat.); 7,73 - 7,85 (m, 6H, aromat.)

d) N-Hydroxy-N-{1-[3-(4-trifluormethyl-phenylethinyl)-phenyl]-ethyl}-harnstoff

Analog Beispiel 7 werden 3,52 g des Produkts aus Beispiel 11c mit 1,20 g Natriumcyanoborhydrid reduziert und dann das so erhaltene Hydroxylamin mit 3,2 ml Trimethylsilyl-isocyanat umgesetzt. Man erhält dabei 2,74 g (67,8 % d. Th.) der Titelverbindung in Form weißer Kristalle, die bei 151-152 °C schmelzen.

$^1$H-NMR (DMSO-$d_6$): 1,42, 1,44 (d, 3H, $NCCH_3$); 5,27 - 5,37 (m, 1H, NCH); 7,38 - 7,56 (m, 4H, aromat.) 7,78 (s, 4H, aromat.)

Beispiel 12

N-{1-[3-(4-Chlor-phenylethinyl)-phenyl]-ethyl}-acetohydroxamsäure

a) N-Acetoxy-N-{1-[3-(4-chlor-phenylethinyl)-phenyl]-ethyl}-acetamid

1,0 g 3-(4-Chlor-phenylethinyl)-acetophenonoxim wird in 20 ml Ethanol heiß gelöst, anschließend wird auf

0°C abgekühlt, wobei ein Teil der Substanz wieder ausfällt. Nun tropft man 0,82 ml Boran-Pyridin-Komplex zu. Nach 10 Minuten werden langsam 1,24 ml Salzsäure (6N) zugefügt. Man rührt 2 Stunden bei 0°C und gibt weitere 3 ml Salzsäure langsam zum Reaktionsgemisch. Die Temperatur wird innerhalb von 2 Stunden auf 15°C erhöht, wobei Lösung erfolgt und dann wird noch 2 Stunden bei 3°C gerührt. Man versetzt portionsweise mit Natriumcarbonat, bis das Gemisch etwa pH 8 erreicht hat, verdünnt mit Ether und wäscht dreimal mit gesättigter Natriumchloridlösung. Nach Trocknen über Natriumsulfat und Eindampfen im Vakuum erhält man als Rückstand ein leicht bräunlich gefärbtes Öl. Dieses wird in 10 ml wasserfreiem Tetrahydrofuran gelöst und unter Kühlen im Eiswasserbad nacheinander mit 1,51 ml Pyridin und 1,32 ml Essigäurechlorid versetzt. Der Reaktionsverlauf wird dünnschichtchromatographisch verfolgt (Petrolether/Essigsäureethylester- 1:1). Sobald die Reaktion beendet ist, wird mit Essigsäureethylester und mit gesättigter Natriumhydrogencarbonatlösung versetzt und dann die organische Schicht mit gesättigter Natriumchloridlösung gewaschen. Nach dem Trocknen über Natriumsulfat und Eindampfen im Vakuum hinterbleibt ein viskoses Öl, das durch Chromatographie mit Petrolether/Essigsäureethylester (1:1) gereinigt wird. Man erhält 1,039 g (83,5 % d. Th.) der Titelverbindung in Form eines farblosen Öls.

$^1$H-NMR ($CDCl_3$): 1,53, 1,56 (d, 3H, $NCCH_3$); 2,13 (s, 3H, $NOCOCH_3$); 2,16 (s, 3H, $NCOCH_3$); 5,70 - 5,85 (m, 1H, NCH); 7,15 - 7,54 (m, 8H, aromat.)

b) N-{1-[3-(4-Chlor-phenylethinyl)-phenyl]-ethyl}-acetohydroxamsäure

Man verfährt analog Beispiel 4d und erhält aus 1,031 g des in Beispiel 12a gewonnenen Produkts, 0,20 g wasserfreiem Kaliumcarbonat und 11 ml Methanol nach Chromatographie mit Petrolether/Essigsäureethylester (1:1) 0,786 g (89,2 % d. Th.) der Titelverbindung in Form weißer Kristalle, die bei 129°C schmelzen.

$^1$H-NMR (DMSO-$d_6$): 1,46, 1,48 (d, 3H, $NCCH_3$); 2,02 (s, 3H, $NCOCH_3$); 5,53 - 5,68 (m, 1H, NCH); 7,27 - 7,56 (m, 6H, aromat.); 7,57 - 7,59 (m, 2H, aromat.)

## Beispiel 13

Analog Beispiel 12 werden aus den entsprechenden Ausgangsmaterialien erhalten:

a) N-{1-[4-(4-Isopropyl-phenylethinyl)-phenyl]-ethyl}-acetohydroxamsäure

Farblose Kristalle, die bei 142-143°C schmelzen.

$^1$H-NMR (DMSO-$d_6$): 1,20, 1,22 (d, 6H, $C(CH_3)_2$); 1,45, 1,47 (d, 3H, $NCCH_3$); 2,02 (s, 3H, $NCOCH_3$); 2,87 - 2,96 (m, 1H, $CH(C)_2$); 5,57 - 5,71 (m, 1H, NCH); 7,20 - 7,36 (m, 4H, aromat.); 7,44 - 7,50 (m, 4H, aromat.)

b) N-[4-(2,6-Dichlorphenylethinyl)-benzyl]-acetohydroxamsäure

Schmelzpunkt: 139 - 139,5°C

$^1$H-NMR (DMSO-$d_6$): 2,06 (s, 3H, $NCOCH_3$); 4,73 (s, 2H, $NCH_2$); 7,34 - 7,45 (m, 3H, aromat.); 7,55 - 7,60 (m, 4H, aromat.)

## Beispiel 14

N-{1-[4-(Thien-2-yl-ethinyl)-phenyl]-ethyl}-acetohydroxamsäure

a) 4-(Thien-2-yl-ethinyl)-acetophenon

Analog Beispiel 1 a wird 4-Bromacetophenon mit 2-Thienylacetylen in Triethylamin in Gegenwart von Bis-(triphenylphosphin)-palladium-(II)-chlorid und Kupfer-(I)-jodid umgesetzt. Das Rohprodukt wird mit n-Hexan/Essigsäureethylester 9:1 chromatographiert. Man erhält so die Titelverbindung als gelbliches Öl.

$^1$H-NMR ($CDCl_3$): 2,61 (s, 3H, $COCH_3$); 7,05 (dd, 1H, aromat.); 7,34 (m, 2H, aromat.); 7,59 (m, 2H, aromat.); 7,93 (m, 2H, aromat.)

b) 4-(Thien-2-yl-ethinyl)-acetophenonoxim

Man setzt 4,03 g Hydroxylamin-hydrochlorid mit 6,57 g 4-(Thien-2-yl-ethinyl)-acetophenon und 3,07 g Natriumcarbonat in 150 ml Ethanol/Wasser 2:1 bei Raumtemperatur um. Nach üblicher Aufarbeitung wird das Rohprodukt aus Essigsäureethylester umkristallisiert. Man erhält so 4,62 g (66,0% d. Th.) eines farblosen Feststoffs.

Schmelzpunkt: 187 - 188°C.

$^1$H-NMR ($CDCl_3$): 2,25 (s, 3H, $N=CCH_3$); 7,03 (dd, 1H, aromat.); 7,31 (m, 2H, aromat.); 7,48 (m, 2H, aromat.); 7,66 (m, 2H, aromat.)

c) N-{1-[4-(Thien-2-yl-ethinyl)-phenyl]-ethyl}-hydroxylamin

i) Zu 0,24 g 4-(Thien-2-yl-ethinyl)-acetophenonoxim in 5 ml Eisessig werden bei Raumtemperatur 0,252 g Natriumcyanoborhydrid gegeben. Das Reaktionsgemisch wird 30 Minuten gerührt, mit weiteren 0,126 g Natriumcyanoborhydrid versetzt und nochmals 30 Minuten gerührt. Die Weiterverarbeitung des Reaktionsgemisches wird in Beispiel 14d beschrieben.

ii) Zu 0,24 g 4-(Thien-2-yl-ethinyl)-acetophenonoxim und 0,125 g Natriumcyanoborhydrid in 5 ml Ethanol werden drei Tropfen gesättigte, ethanolische Methylorangelösung gegeben. Diese Mischung wird bei Raumtemperatur unter Rühren langsam mit 1,05 ml einer 10 %igen, ethanolischen Salzsäure versetzt, bis die Rosafärbung 30 Sekunden bestehen bleibt. Das Reaktionsgemisch wird zu 30 ml gesättigter Kochsalzlösung gegeben und dreimal mit je 15 ml Essigsäureethylester ausgeschüttelt. Die organischen Auszüge werden mit 10 ml gesättigter Natriumhydrogencarbonatlösung gewaschen, über Magnesiumsulfat getrocknet und im Vakuum eingeengt. Das Rohprodukt wird mit n-Hexan/Isopropanol 9:1 chromatographiert. Man erhält 0,088 g (36 % d. Th.) eines gelblichen Öls.

iii) 0,24 g 4-(Thien-2-yl-ethinyl)-acetophenonoxim in 5 ml Ethanol werden bei 0 °C unter Überleiten von Stickstoff mit 0,2 ml Boran-Pyridin-Komplex versetzt und 15 Minuten gerührt. Das Reaktionsgemisch wird mit 1,6 ml 20 %iger ethanolischer Salzsäure versetzt, 1 Stunde bei Raumtemperatur gerührt, in 30 ml gesättigte Kochsalzlösung gegeben und mit Kaliumcarbonat auf pH 9 gebracht. Anschließend wird dreimal mit je 10 ml Essigsäureethylester ausgeschüttelt. Die organischen Auszüge werden über Magnesiumsulfat getrocknet und im Vakuum eingeengt. Man erhält 0,25 g (praktisch quantitative Ausbeute) eines gelblichen Öls.

d) N-Acetoxy-N-{1-[4-(thien-2-yl-ethinyl)-phenyl]-ethyl}-acetamid

i) Das Reaktionsgemisch aus Beispiel 14 c)i) wird bei Raumtemperatur mit 0,5 ml Essigsäureanhydrid versetzt, 16 Stunden gerührt und vorsichtig zu 7,0 g Natriumhydrogencarbonat in 100 ml Wasser gegeben. Sobald die Kohlendioxidentwicklung abgeklungen ist, wird dreimal mit je 30 ml Essigsäureethylester ausgeschüttelt. Die organischen Auszüge werden über Magnesiumsulfat getrocknet und im Vakuum eingeengt. Das Rohprodukt wird mit n-Hexan/Essig-säureethylester 4:1 chromatographiert. Man erhält so 0,16 g (49 % d. Th. bezogen auf Beispiel 14c)i) eines gelblichen Öls.

$^1$H-NMR ($CDCl_3$): 1,46 (d, 3H, $NCCH_3$); 1,98 (s, 3H, $NCOCH_3$); 2,18 (s, 3H, $OCOCH_3$); 5,67 (m, 1H, NCH); 7,13 (dd, 1H, aromat.); 7,41 (m, 3H, aromat.); 7,52 (d, 2H, aromat.); 7,65 (m, 1H, aromat.)

ii) 0,25 g des Produkts aus Beispiel 14 c)iii) in 5 ml Toluol werden mit 1 ml Essigsäureanhydrid und 0,2 ml Pyridin versetzt und zwei Stunden zum Rückfluß erhitzt. Das abgekühlte Reaktionsgemisch wird zu 25 ml gesättigter Kochsalzlösung und 2 ml 1N Salzsäure gegeben. Die organische Phase wird abgetrennt, die wässrige Phase noch zweimal mit je 15 ml Essigsäureethylester ausgeschüttelt. Die organischen Auszüge werden mit 5 ml gesättigter Kochsalzlösung gewaschen, über Magnesiumsulfat getrocknet und im Vakuum eingeengt. Das zurückbleibende Öl wird mit n-Hexan/Isopropanol 9:1 chromatographiert. Man erhält so 0,20 g (61 % d. Th.) eines gelblichen Öls.

e) N-{1-[4-(Thien-2-yl-ethinyl)-phenyl]-ethyl}-acetohydroxamsäure

Zu 0,145 g des in Beispiel 14 d) erhaltenen Produkts in 2 ml Methanol werden bei Raumtemperatur 0,57 ml einer 1-molaren Lösung von Lithiumhydroxid in Methanol gegeben. Das Reaktionsgemisch wird 30 Minuten bei Raumtemperatur gerührt, zu 4 ml gesättigter Kochsalzlösung und 1 ml gesättigter Natriumhydrogencarbonatlösung gegeben und dreimal mit je 2 ml Essigsäureethylester ausgeschüttelt. Die organischen Auszüge werden mit 1 ml gesättigter Kochsalzlösung gewaschen, über Magnesiumsulfat getrocknet, filtriert und im Vakuum eingeengt. Das Rohprodukt wird aus n-Hexan/Essigsäureethylester umkristallisiert. Man erhält so 0,065 g (52 % d. Th.) eines farblosen Feststoffs.

Schmelzpunkt: 128-129 °C

$^1$H-NMR (DMSO-$d_6$): 1,46 (d, 3H, $NCCH_3$); 2,03 (s, 3H, $NCOCH_3$); 5,63 (m, 1H, NCH); 7,11 (dd, 1H, aromat.); 7,37 (m, 3H, aromat.); 7,49 (d, 2H, aromat.); 7,64 (m 1H, aromat.)

Beispiel 15

N-Hydroxy-N-{1-[4-(thien-2-yl-ethinyl)-phenyl]-ethyl}-harnstoff

1,37 g des in Beispiel 14 c) dargestellten Hydroxylamins in 25 ml Tetrahydrofuran werden mit 1 ml Trimethylsilylisocyanat analog Beispiel 7 umgesetzt und dann wird in üblicher Weise aufgearbeitet. Das Rohprodukt wird aus n-Hexan/Essigsäureethylester umkristallisiert. Man erhält so 0,662 g (46,0 % d. Th.) eines farblosen Feststoffs.

Schmelzpunkt: 154 - 155 °C
$^1$H-NMR (DMSO-$d_6$): 1,53 (d, 3H, $NCCH_3$); 5,48 (q, 1H, NCH); 5,59 (s, 2H, $NH_2$); 7,02 (dd, 1H, aromat.); 7,29 (m, 2H, aromat.); 7,44 (m, 4H, aromat.)

Beispiel 16

Unter Anwendung von in den Beispielen 14 und 15 beschriebenen Verfahrensweisen erhält man aus den entsprechenden Ausgangsmaterialien

a) N-{1-[4-(Thien-3-yl-ethinyl)-phenyl]-ethyl}-acetohydroxamsäure
Schmelzpunkt: 130 - 131 °C
$^1$H-NMR (DMSO-$d_6$): 1,51 (d, 3H, $NCCH_3$); 2,09 (s, 3H, $NCOCH_3$); 5,69 (m, 1H, NCH); 7,33 (dd, 1H, aromat.) 7,41 (m, 2H, aromat.); 7,56 (m, 2H, aromat.); 7,70 (dd, 1H, aromat.); 7,95 (dd, 1H, aromat.)

b) N-Hydroxy-N-{1-[4-(thien-3-yl-ethinyl)-phenyl]-ethyl}-harnstoff
Schmelzpunkt: 156 - 157 °C
$^1$H-NMR (DMSO-$d_6$): 1,42 (d, 3H, $NCCH_3$); 5,32 (q, 1H, NCH); 6,33 (s, 2H, $NH_2$); 7,24 (dd, 1H, aromat.); 7,37 (d, 2H, aromat.); 7,45 (d, 2H, aromat.); 7,62 (dd, 1H, aromat.); 7,84 (dd, 1H, aromat.)

c) N-[4-(Thien-2-yl-ethinyl)-benzyl]-acetohydroxamsäure
Schmelzpunkt: 113 - 114 °C
$^1$H-NMR (DMSO-$d_6$): 2,06 (s, 3H, $NCOCH_3$); 4,71 (s, 2H, $NCH_2$); 7,12 (dd, 1H, aromat.); 7,31 (d, 2H, aromat.); 7,40 (dd, 1H, aromat.); 7,51 (d, 2H, aromat.); 7,64 (dd, 1H, aromat.)

d) N-Hydroxy-N-[4-(thien-2-yl-ethinyl)-benzyl]-harnstoff
Schmelzpunkt: 177 °C
$^1$H-NMR (DMSO-$d_6$): 4,57 (s, 2H, $NCH_2$); 6,44 (s, 2H, $NH_2$); 7,11 (m, 1H, aromat.); 7,34 (d, 2H, aromat.); 7,40 (d, 1H, aromat.); 7,49 (d, 2H, aromat.); 7,63 (d, 1H, aromat.)

e) N-[4-(Thien-3-yl-ethinyl)-benzyl]-acetohydroxamsäure
Schmelzpunkt : 147 - 152 °C
$^1$H-NMR (DMSO-$d_6$): 2,06 (s, 3H, $NCOCH_3$); 4,71 (s, 2H, $NCH_2$); 7,25 (d, 1H, aromat.); 7,30 (d, 2H, aromat.); 7,49 (d, 2H, aromat.); 7,63 (m, 1H, aromat.); 7,86 (d, 1H, aromat.)

f) N-Hydroxy-N-[4-(thien-3-yl-ethinyl)-benzyl]-harnstoff
Schmelzpunkt: 187 - 188 °C (Zers.)
$^1$H-NMR (DMSO-$d_6$): 4,56 (s, 2H, $NCH_2$); 6,41 (s, 2H, $NH_2$); 7,25 (d, 1H, aromat.); 7,33 (d, 2H, aromat.); 7,47 (d, 2H, aromat.); 7,62 (m, 1H, aromat.); 7,85 (m, 1H, aromat.)

g) N-Hydroxy-N-{1-[4-(5-chlor-thien-2-yl-ethinyl)-phenyl]-ethyl}-harnstoff
Schmelzpunkt: 151 - 152 °C
$^1$H-NMR (DMSO-$d_6$): 1,42 (d, 3H, $NCCH_3$); 5,34 (q, 1H, NCH); 6,36 (s, 2H, $NH_2$); 7,13 (d, 1H, aromat.); 7,28 (d, 1H, aromat.); 7,39 (d, 2H, aromat.); 7,47 (d, 2H, aromat.)

h) N-{1-[4-(5-Chlor-thien-2-yl-ethinyl)-phenyl]-ethyl}-acetohydroxamsäure
Schmelzpunkt: 130 - 131 °C
$^1$H-NMR (DMSO-$D_6$): 1,47 (d, 3H, $NCCH_3$); 2,04 (s, 3H, $NCOCH_3$); 5,64 (m, 1H, NCH); 7,13 (d, 1H, aromat.); 7,28 (d, 1H, aromat); 7,37 (d, 2H, aromat.); 7,50 (d, 2H, aromat.)

i) N-Hydroxy-N-{1-[4-(benzothien-2-yl-ethinyl)-phenyl]-ethyl}-harnstoff
Schmelzpunkt: 164 - 165 °C
$^1$H-NMR (DMSO-$d_6$): 1,44 (d, 3H, $NCCH_3$); 5,35 (m, 1H, NCH); 6,37 (s, 2H, $NH_2$); 7,35 - 7,96 (m, 9H, aromat.)

k) N-{1-[4-(Benzothien-2-yl-ethinyl)-phenyl]-ethyl}-acetohydroxamsäure
Schmelzpunkt: 172 - 173 °C
$^1$H-NMR (DMSO-$d_6$): 1,49, (d, 3H, $NCCH_3$); 2,06 (s, 3H, $NCOCH_3$); 5,67 (m, 1H, NCH); 7,39 - 7,45 (m, 4H, aromat.); 7,56 (d, 2H, aromat.); 7,74 (s, 1H, aromat.); 7,86 (m, 1H, aromat.); 7,95 (m, 1H, aromat.)

Beispiel 17

Man verfährt wie in Beispiel 12, geht aber von den entsprechenden (Pyridin-3-yl-ethinyl)-acetophenon- oder -benzaldehydoximen aus und erhält so:

a) N-{1-[4-(Pyridin-3-yl-ethinyl)-phenyl]-ethyl}-acetohydroxamsäure

Schmelzpunkt: 121 °C

$^1$H-NMR (DMSO-$d_6$): 1,45, 1,48 (d, 3H, $NCCH_3$); 2,03 (s, 3H, $NCOCH_3$); 5,63 - 5,65 (m, 1H, NCH); 7,37 - 7,56 (m, 5H, aromat.); 7,94 - 7,99 (m, 1H, aromat.); 8,57 - 8,59 (m, 1H, aromat.); 8,73, 8,74 (d, 1H, aromat.)

b) N-[4-(Pyridin-3-yl-ethinyl)-benzyl]-acetohydroxamsäure

Schmelzpunkt: 125,5 °C

$^1$H-NMR (DMSO-$d_6$): 2,06 (s, 3H, $NCOCH_3$); 4,72 (s, 2H, $NCH_2$); 7,31 - 7,34 (m, 2H, aromat.); 7,45 - 7,47 (m, 1H , aromat.); 7,54 - 7,57 (m, 2H, aromat.); 7,95 - 7,97 (m, 1H, aromat.); 8,57 - 8,59 (m, 1H, aromat.); 8,74, 8,75 (d, 1H, aromat.)

c) N-[3-(Pyridin-3-yl-ethinyl)-benzyl]-acetohydroxamsäure

Schmelzpunkt: 114,5-115,5 °C

$^1$H-NMR (DMSO-$d_6$): 2,07 (s, 3H, $NCOCH_3$); 4,71 (s, 2H, $NCH_2$); 7,33 - 7,51 (m, 5H, aromat.); 7,97 - 7,99 (m, 1H, aromat.); 8,58 - 8,60 (m, 1H, aromat.); 8,76, 8,77 (d, 1H, aromat.)

## Patentansprüche

## Patentansprüche für folgende Vertragsstaaten : AT, BE, CH, DE, DK, FR, GB, IT, LI, LU, NL, SE

**1.** Substituierte Phenylacetylene der allgemeinen Formel

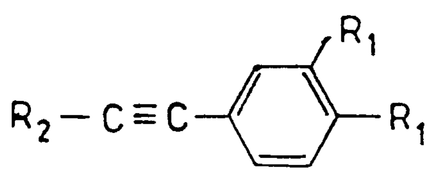

I

worin einer der Reste $R_1$ ein Wasserstoffatom, der andere die Gruppe der Formel

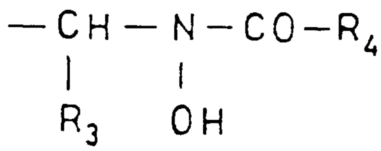

in der $R_3$ ein Wasserstoffatom, einen Methyl- oder einen Ethylrest und $R_4$ einen Methylrest oder eine Aminogruppe darstellt, bedeutet und

in der $R_2$

a) für einen ein- oder zweikernigen aromatischen Rest aus der Gruppe mit den Formeln

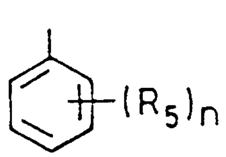 , 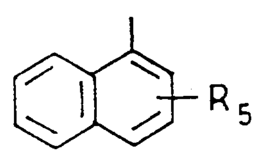 , 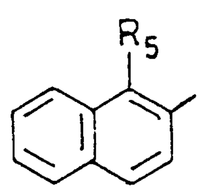 oder 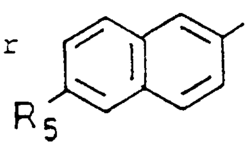

worin $R_5$ ein Wasserstoffatom, eine Benzyloxygruppe, eine geradkettige oder verzweigte Alkyl-, Alkoxy- oder Alkylmercaptogruppe mit 1 bis 4 Kohlenstoffatomen, ein Fluor- oder Chloratom oder die Trifluormethylgruppe und n eine ganze Zahl von 1 bis 3 darstellt,

oder

b) für einen ein- oder zweikernigen, Schwefel, Stickstoff oder Sauerstoff enthaltenden heterocyclischen Rest aus der Gruppe mit den Formeln

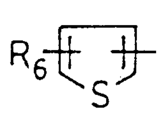 , 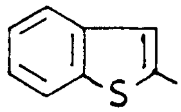 , 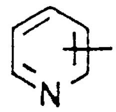 , 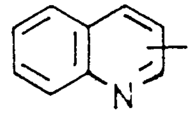 oder 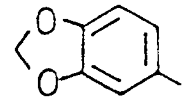

worin $R_6$ ein Wasserstoff- oder ein Chloratom oder eine Methylgruppe bedeutet,
steht und die Stereoisomeren und optisch aktiven Formen der substituierten Phenylacetylene der Formel I.

**2.** Substituierte Phenylacetylene der allgemeinen Formel I aus Anspruch 1, in der $R_1$ und $R_4$ die gleiche Bedeutung wie oben haben, $R_3$ für ein Wasserstoffatom oder für einen Methylrest steht und in der $R_2$
a) für einen ein- oder zweikernigen aromatischen Rest aus der Gruppe mit den Formeln

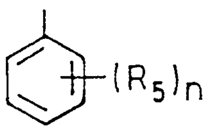 , 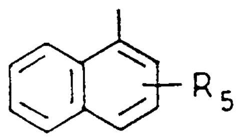 , 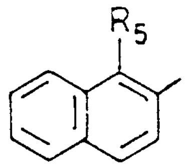 oder 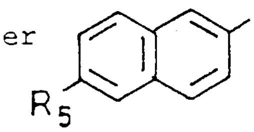

worin $R_5$ ein Wasserstoffatom, eine geradkettige oder verzweigte Alkyl-, Alkoxy- oder Alkylmercaptogruppe mit 1 bis 4 Kohlenstoffatomen, ein Fluor- oder Chloratom oder die Trifluormethylgruppe und n eine ganze Zahl von 1 bis 3 darstellt,
oder
b) für einen ein- oder zweikernigen, Schwefel oder Sauerstoff enthaltenden heterocyclischen Rest aus der Gruppe mit den Formeln

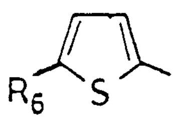 , 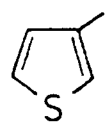 , 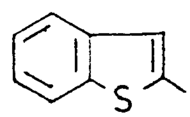 oder 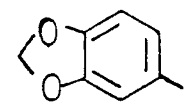

worin $R_6$ ein Wasserstoff- oder ein Chloratom oder eine Methylgruppe bedeutet,
steht und die Stereoisomeren und optisch aktiven Formen der substituierten Phenylacetylene der Formel I.

**3.** Substituierte Phenylacetylene der allgemeinen Formel I aus Anspruch 1, in der $R_1$ und $R_4$ die gleiche Bedeutung wie oben haben, $R_3$ für ein Wasserstoffatom oder für einen Methylrest steht und in der $R_2$
a) für einen ein- oder zweikernigen aromatischen Rest aus der Gruppe mit den Formeln

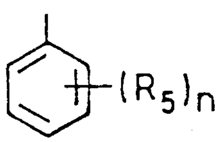 , 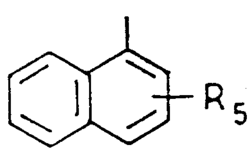 , 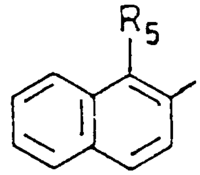 oder 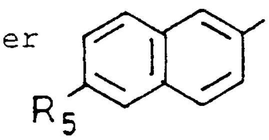

worin $R_5$ ein Wasserstoffatom, eine Methoxygruppe, einen geradkettigen oder verzweigten Alkylrest mit 1 bis 4 Kohlenstoffatomen, ein Fluor- oder Chloratom oder die Trifluormethylgruppe und n eine ganze Zahl von 1 bis 3 darstellt,
oder
b) für einen ein- oder zweikernigen, Schwefel oder Sauerstoff enthaltenden heterocyclischen Rest aus der Gruppe mit den Formeln

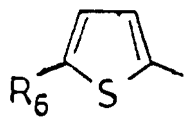 , 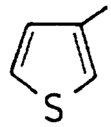 , 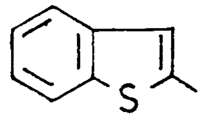 oder 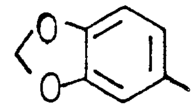

worin $R_6$ ein Wasserstoff- oder ein Chloratom oder eine Methylgruppe bedeutet,
steht und die Stereoisomeren und optisch aktiven Formen dieser substituierten Phenylacetylene der Formel I.

**4.** Substituierte Phenylacetylene gemäß Anspruch 3 der Formel I aus Anspruch 1, in denen $R_2$ eine der Gruppen

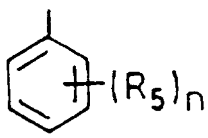 , 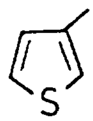 sowie 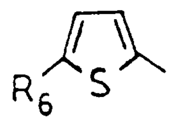

worin $R_5$, $R_6$ und n die in Anspruch 2 angegebene Bedeutung haben,
darstellt.

**5.** Substituierte Phenylacetylene der Formel I aus Anspruch 1, in denen $R_2$ der 3-Chinolylrest oder vorzugsweise der 3-Pyridylrest ist.

**6.** Substituierte Phenylacetylene gemäß Anspruch 4, in denen $R_5$ eine Methoxygruppe in 3- und/oder 4-Stellung zur Acetylengruppe darstellt und n eine der Zahlen 1 oder 2 bedeutet.

**7.** Substituierte Phenylacetylene gemäß Anspruch 4, in denen $R_5$ ein Fluor- oder Chloratom in 4-Stellung zur Acetylengruppe darstellt.

**8.** Substituierte Phenylacetylene gemäß Ansprüchen 2 bis 4, in denen $R_6$ ein Wasserstoffatom darstellt.

**9.** Substituierte Phenylacetylene gemäß Ansprüchen 2 bis 4, 6 und 7 der Formel

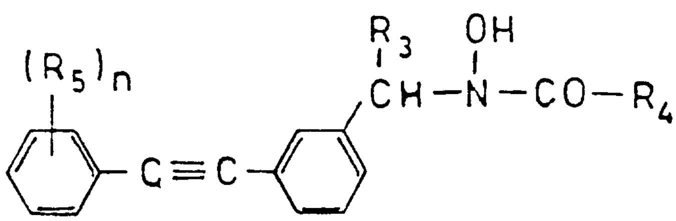

Ia

worin $R_3$ bis $R_5$ und n die gleiche Bedeutung wie oben haben.

**10.** Substituierte Phenylacetylene gemäß Ansprüchen 2 bis 4, 6 und 7 der Formel

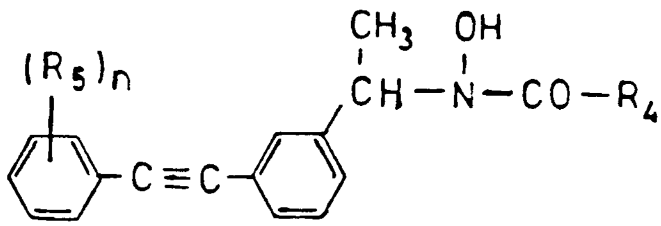

Ib

worin $R_4$ eine Methyl- oder eine Aminogruppe darstellt und $R_5$ für eine Methoxygruppe in 3- und/oder

4-Stellung zur Acetylengruppe (bei n = 1 oder 2) oder für ein Fluor- oder Chloratom in 4-Stellung zur Acetylengruppe steht.

**11.** Substituierte Phenylacetylene gemäß Anspruch 10 der Formel

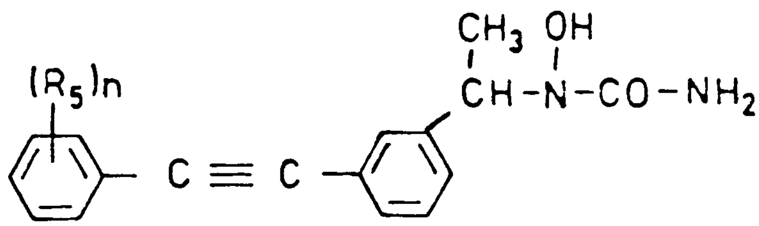

Ic

worin $R_5$ und n die gleiche Bedeutung wie in Anspruch 10 haben.

**12.** Substituierte Phenylacetylene gemäß Ansprüchen 1 bis 4 der Formel

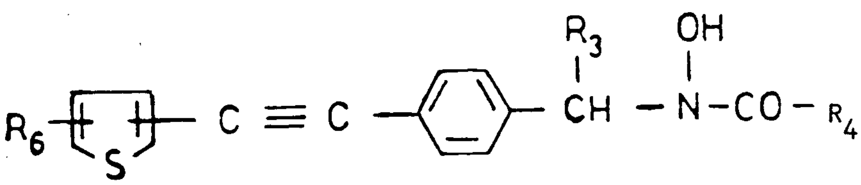

Id

worin $R_3$, $R_4$ und $R_6$ die gleiche Bedeutung wie oben haben.

**13.** Substituierte Phenylacetylene gemäß Ansprüchen 2 bis 4 und 12 der Formel

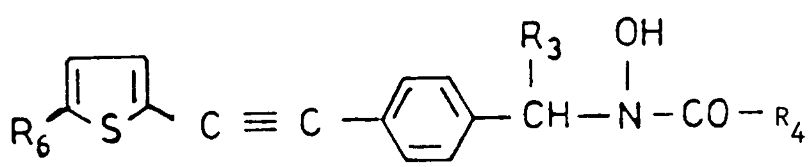

Ie

worin $R_3$ ein Wasserstoffatom oder eine Methylgruppe darstellt und $R_4$ und $R_6$ die gleiche Bedeutung wie oben haben.

**14.** Substituierte Phenylacetylene gemäß Ansprüchen 2 bis 4 der Formel

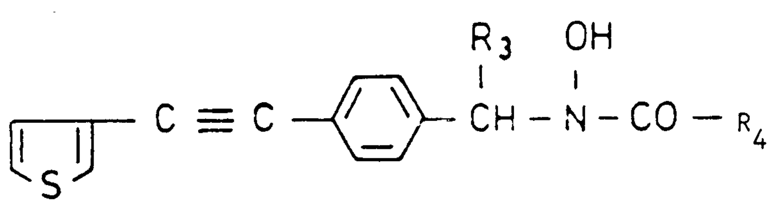

If

worin $R_3$ ein Wasserstoffatom oder die Methylgruppe und $R_4$ eine Methyl- oder eine Aminogruppe darstellt.

**15.** Arzneimittel, dadurch gekennzeichnet, daß es als Wirkstoff wenigstens eines der substituierten Phenylacetylene entsprechend Ansprüchen 1 bis 14 enthält und zur parenteralen, oralen, intranasalen, perkutanen oder rektalen Applikation geeignet ist.

**16.** Arzneimittel gemäß Anspruch 15, dadurch gekennzeichnet, daß es als Wirkstoff pro Einzeldosis 0,01 bis 50 mg eines substituierten Phenylacetylens entsprechend Ansprüchen 1 bis 14 enthält.

**17.** Arzneimittel gemäß Ansprüchen 15 und 16, dadurch gekennzeichnet, daß es zur parenteralen Applikation geeignet ist und 0,01 bis 10 mg eine substituierten Phenylacetylens entsprechend Ansprüchen 1 bis 14 enthält.

**18.** Arzneimittel gemäß Ansprüchen 15 und 16, dadurch gekennzeichnet, daß es in Form von Tabletten, Dragees oder Kapseln, gegebenenfalls mit verzögerter Wirkstofffreigabe, vorliegt und 0,1 bis 50 mg eines substituierten Phenylacetylens entsprechend Ansprüchen 1 bis 14 enthält.

**19.** Arzneimittel gemäß Anspruch 15 zur intranasalen, oralen oder peroralen Verabreichung eines substituierten Phenylacetylens entsprechend Ansprüchen 1 bis 14 in Spray-Form.

**20.** Arzneimittel gemäß Anspruch 15 zur perkutanen Applikation, bestehend aus einem auf die Haut aufzubringenden, ein substituiertes Phenylacetylen entsprechend Ansprüchen 1 bis 14 in gelöster Form, vorzugsweise unter Zusatz von die Hautpenetration fördernden Mitteln, enthaltendem Reservoir.

**21.** Verfahren zur Herstellung der Arzneimittel gemäß Ansprüchen 15 bis 20, dadurch gekennzeichnet, daß man die substituierten Phenylacetylene entsprechend Ansprüchen 1 bis 14 in üblicher Weise mit Trägermaterialien, Lösungsmitteln bzw. Verdünnungsstoffen, sowie gegebenenfalls Bindemitteln, Tablettensprengstoffen und anderen Hilfsstoffen verarbeitet und aus dem erhaltenen Gemisch die Einzeldosierungsformen fertigt.

**22.** Verfahren zur Herstellung der substituierten Phenylacetylene der Formel I aus Anspruch 1, dadurch gekennzeichnet, daß man

(i) eine Verbindung der Formel

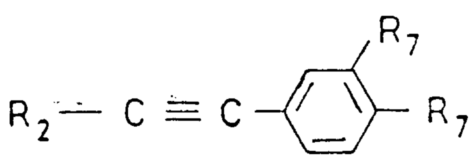

II

in der $R_2$ die gleiche Bedeutung wie oben hat und einer der Reste $R_7$ Wasserstoff, der andere die Gruppe der Formel -CO-$R_3$ bedeutet,
mit Hydroxylamin oder einen von dessen Salzen in Gegenwart von Basen zum Oxim umsetzt, dieses mittels Borhydriden oder Boran-Amin-Komplexen in Gegenwart von Säuren zu einer Verbindung der Formel

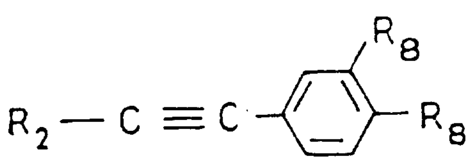

III

in der einer der Reste $R_8$ für Wasserstoff, der andere für die Gruppe der Formel

-CH($R_3$)-NH-OH

steht, reduziert und in diese den Acylrest der Formel -COR$_4$ einführt oder

(ii) eine Verbindung der Formel

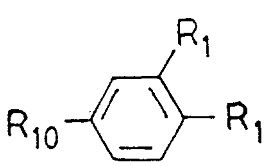

V

worin $R_1$ die gleiche Bedeutung wie oben hat und $R_{10}$ für ein Brom- oder Jodatom steht, mit einer Verbindung der Formel

$R_2$-C≡CH VI

worin $R_2$ die gleiche Bedeutung wie oben hat,
in Gegenwart eines bei etwa 0° - 80° C flüssigen sekundären oder tertiären Amins, insbesondere eines Dialkyl- oder Trialkylamins mit 2 oder 3 Kohlenstoffatomen in jedem der Alkylreste, Pyrrolidin oder Piperidin unter Zugabe von etwa 0,01 bis 5 Prozent pro Mol eingesetzter Verbindung der Formel VI eines komplexen Palladiumkatalysators, insbesondere Bis-(triphenylphosphin)-palladium-(II)-chlorid oder -acetat oder Tetrakis-(triphenylphosphin)-palladium und gegebenenfalls unter Zusatz katalytischer Mengen Kupfer(I)-jodid bei etwa 20° - 80° C zur Reaktion bringt.

**23.** Verfahren zur Herstellung der substituierten Phenylacetylene der Formel I aus Anspruch 1, in denen $R_4$ eine Aminogruppe bedeutet gemäß Anspruch 22, Verfahrensweise (i), dadurch gekennzeichnet, daß man die Verbindung der Formel III
a) mit Trimethylsilyl-isocyanat bei Temperaturen zwischen 20° C und 100° C umsetzt und anschließend die Trimethylsilylgruppe abspaltet, oder
b) mit Kalium- oder Natriumcyanat in saurer Lösung oder mit Phosgen oder einem Chlorameisensäure(niedrigalkyl)- oder -benzylester in Gegenwart eines säurebindenden Mittels, und anschließend mit Ammoniak oder Ammoniumcarbonat umsetzt.

**24.** Verfahren zur Herstellung der substituierten Phenylacetylene der Formel I aus Anspruch 1, in denen $R_4$ eine Methylgruppe bedeutet, gemäß Anspruch 22, Verfahrensweise (i), dadurch gekennzeichnet, daß man die Verbindung der Formel III mit einem Acetylierungsmittel umsetzt und aus der so erhaltenen Verbindung der Formel

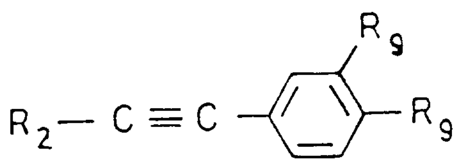

IV

worin einer der Reste $R_9$ ein Wasserstoffatom ist und der andere die Gruppe der Formel

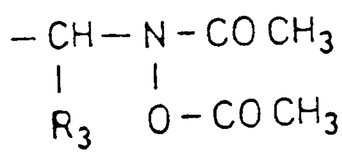

darstellt,
durch selektive basische Hydrolyse die O-Acetylgruppe abspaltet.

**25.** Verfahren gemäß Anspruch 22, Verfahrensweise (i), dadurch gekennzeichnet, daß die Bildung des Oxims der Verbindung der Formel II bei Temperaturen von etwa 20 ° bis 60 °C durchgeführt wird.

**26.** Verfahren gemäß Anspruch 22, Verfahrensweise (i), dadurch gekennzeichnet, daß das Oxim der Verbindung der Formel II mit Natriumcyanoborhydrid in Gegenwart von Säuren bei Temperaturen von etwa 20° C bis 60° C zum Hydroxylaminderivat der Formel III reduziert wird.

**27.** Verfahren gemäß Anspruch 22, Verfahrensweise (i), dadurch gekennzeichnet, daß das Oxim der Verbindung der Formel II mit Boran-Amin-Komplexen in Gegenwart von Säuren bei Temperaturen von etwa 0° bis 20°C zum Hydroxylaminderivat der Formel III reduziert wird.

**Patentansprüche für folgenden Vertragsstaat : ES**

**1.** Verfahren zur Herstellung von substituierten Phenylacetylenen der allgemeinen Formel

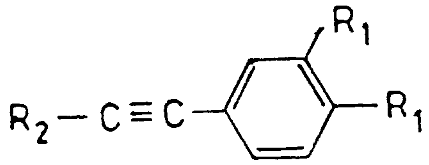

I

worin einer der Reste $R_1$ ein Wasserstoffatom, der andere die Gruppe der Formel

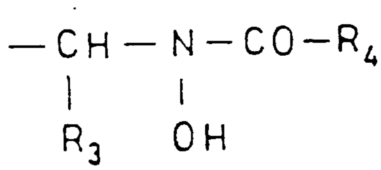

in der $R_3$ ein Wasserstoffatom, einen Methyl- oder einen Ethylrest und $R_4$ einen Methylrest oder eine Aminogruppe darstellt, bedeutet und
in der $R_2$
a) für einen ein- oder zweikernigen aromatischen Rest aus der Gruppe mit den Formeln

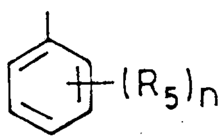 , 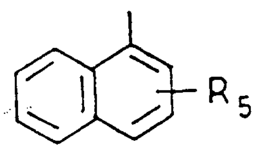 , 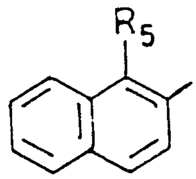 oder 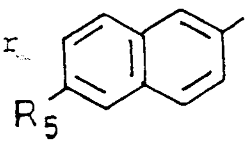

worin $R_5$ ein Wasserstoffatom, eine Benzyloxygruppe, einen geradkettigen oder verzweigten Alkyl-, Alkoxy- oder Alkylmercaptorest mit 1 bis 4 Kohlenstoffatomen, ein Fluor- oder Chloratom oder die Trifluormethylgruppe und n eine ganze Zahl von 1 bis 3 darstellt,
oder
b) für einen ein- oder zweikernigen, Schwefel, Stickstoff oder Sauerstoff enthaltenden heterocyclischen Rest aus der Gruppe mit den Formeln

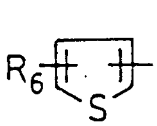 , 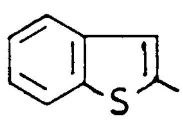 , 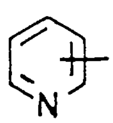 , 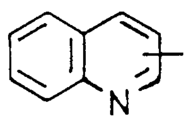 oder 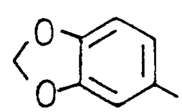

worin $R_6$ ein Wasserstoff- oder ein Chloratom oder eine Methylgruppe bedeutet,
steht und Stereoisomeren und optisch aktiven Formen dieser substituierten Phenylacetylene der Formel I, dadurch gekennzeichnet, daß man
(i) eine Verbindung der Formel

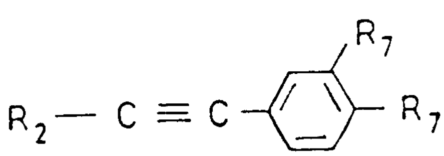

II

in der $R_2$ die gleiche Bedeutung wie oben hat und einer der Reste $R_7$ Wasserstoff, der andere

die Gruppe der Formel $-CO-R_3$ bedeutet,
mit Hydroxylamin oder einen von dessen Salzen in Gegenwart von Basen zum Oxim umsetzt, dieses mittels Borhydriden oder Boran-Amin-Komplexen in Gegenwart von Säuren zu einer Verbindung der Formel

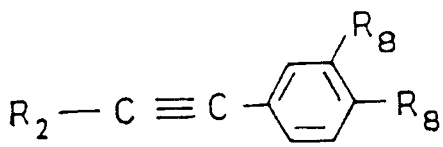

III

in der einer der Reste $R_8$ für Wasserstoff, der andere für die Gruppe der Formel $-CH(R_3)-NH-OH$ steht,
reduziert und in diese den Acylrest der Formel $-COR_4$ einführt oder
(ii) eine Verbindung der Formel

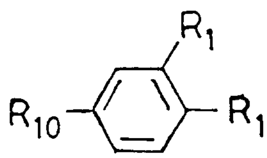

V

worin $R_1$ die gleiche Bedeutung wie oben hat und $R_{10}$ für ein Brom- oder Jodatom steht, mit einer Verbindung der Formel

$R_2-C{\equiv}CH$ VI

worin $R_2$ die gleiche Bedeutung wie oben hat,
in Gegenwart eines bei etwa 0° - 80° C flüssigen sekundären oder tertiären Amins, insbesondere eines Dialkyl- oder Trialkylamins mit 2 oder 3 Kohlenstoffatomen in jedem der Alkylreste, Pyrrolidin oder Piperidin unter Zugabe von etwa 0,01 bis 5 Prozent pro Mol eingesetzter Verbindung der Formel VI eines komplexen Palladiumkatalysators, insbesondere Bis-(triphenylphosphin)-palladium(II)-chlorid oder -acetat oder Tetrakis-(triphenylphosphin)-palladium und gegebenenfalls unter Zusatz katalytischer Mengen Kupfer(I)-jodid bei etwa 20° - 80° C zur Reaktion bringt.

**2.** Verfahren gemäß Anspruch 1 zur Herstellung von substituierten Phenylacetylenen der allgemeinen Formel I aus Anspruch 1, dadurch gekennzeichnet, daß in diesen Verbindungen
$R_1$ und $R_4$ die gleiche Bedeutung wie oben haben
$R_3$ für ein Wasserstoffatom oder für einen Methylrest steht und
$R_2$ für einen ein- oder zweikernigen aromatischen Rest aus der Gruppe mit den Formeln

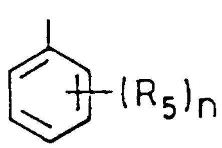 , 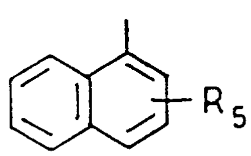 , 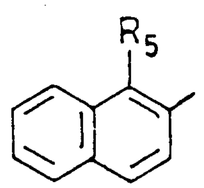 oder 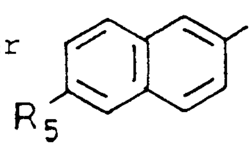

worin $R_5$ ein Wasserstoffatom, eine Methoxygruppe, einen geradkettigen oder verzweigten Alkylrest mit 1 bis 4 Kohlenstoffatomen, ein Fluor- oder Chloratom oder die Trifluormethylgruppe und n eine ganze Zahl von 1 bis 3 darstellt,
oder
für einen ein- oder zweikernigen, Schwefel oder Sauerstoff enthaltenden heterocyclischen Rest aus der Gruppe mit den Formeln

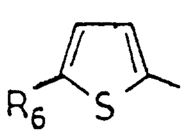 , 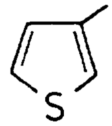 , 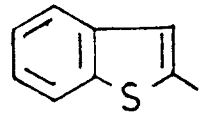 oder 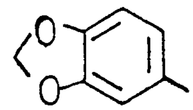

worin $R_6$ ein Wasserstoff- oder ein Chloratom oder eine Methylgruppe bedeutet, steht.

**3.** Verfahren zur Herstellung der substituierten Phenylacetylene der Formel I aus Ansprüchen 1 und 2, in denen $R_4$ eine Aminogruppe bedeutet, gemäß Anspruch 1, Verfahrensweise (i), dadurch gekennzeichnet, daß man die Verbindung der Formel III

a) mit Trimethylsilyl-isocyanat bei Temperaturen zwischen 20° C und 100° C umsetzt und anschließend die Trimethylsilylgruppe abspaltet, oder

b) mit Kalium- oder Natriumcyanat in saurer Lösung oder mit Phosgen oder einem Chlorameisensäure-(niedrigalkyl)- oder -benzylester in Gegenwart eines säurebindenden Mittels, und anschließend mit Ammoniak oder Ammoniumcarbonat umsetzt.

**4.** Verfahren zur Herstellung der substituierten Phenylacetylene der Formel I aus Ansprüchen 1 und 2, in denen $R_4$ eine Methylgruppe bedeutet, gemäß Anspruch 1, Verfahrensweise (i), dadurch gekennzeichnet, daß man die Verbindung der Formel III mit einem Acetylierungsmittel umsetzt und aus der so erhaltenen Verbindung der Formel

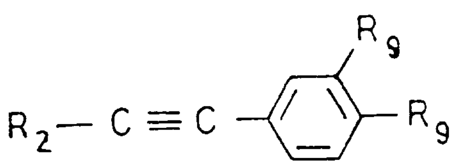

IV

worin einer der Reste $R_9$ ein Wasserstoffatom ist und der andere die Gruppe der Formel

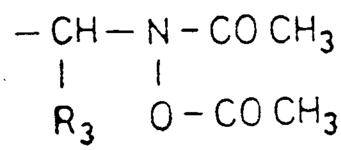

darstellt,
durch selektive basische Hydrolyse die O-Acetylgruppe abspaltet.

**5.** Verfahren gemäß Anspruch 1, Verfahrensweise (i), dadurch gekennzeichnet, daß die Bildung des Oxims der Verbindung der Formel II bei Temperaturen von etwa 20° bis 60° C durchgeführt wird.

**6.** Verfahren gemäß Anspruch 1, Verfahrensweise (i), dadurch gekennzeichnet, daß das Oxim der Verbindung der Formel II mit Natriumcyanoborhydrid in Gegenwart von Säuren bei Temperaturen von etwa 20° C bis 60° C zum Hydroxylaminderivat der Formel III reduziert wird.

**7.** Verfahren gemäß Anspruch 1, Verfahrensweise (i), dadurch gekennzeichnet, daß das Oxim der Verbindung der Formel II mit Boran-Amin-Komplexen in Gegenwart von Säuren bei Temperaturen von etwa 0° bis 20°C zum Hydroxylaminderivat der Formel III reduziert wird.

**Patentansprüche für folgenden Vertragsstaat : GR**

**1.** Substituierte Phenylacetylene der allgemeinen Formel

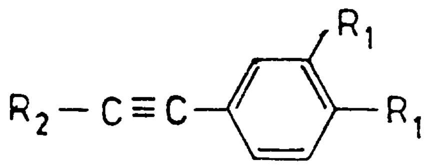

I

worin einer der Reste $R_1$ ein Wasserstoffatom, der andere die Gruppe der Formel

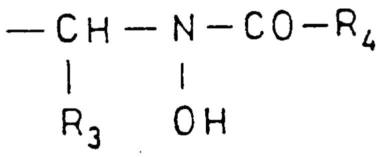

in der $R_3$ ein Wasserstoffatom, einen Methyl- oder einen Ethylrest und $R_4$ einen Methylrest oder eine Aminogruppe darstellt, bedeutet und
in der $R_2$

a) für einen ein- oder zweikernigen aromatischen Rest aus der Gruppe mit den Formeln

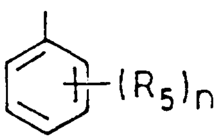 , 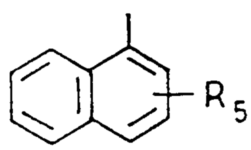 , 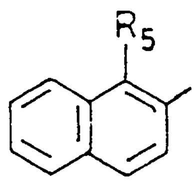 oder 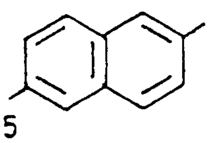

worin $R_5$ ein Wasserstoffatom, eine Benzyloxygruppe, eine geradkettige oder verzweigte Alkyl-, Alkoxy- oder Alkylmercaptogruppe mit 1 bis 4 Kohlenstoffatomen, ein Fluor- oder Chloratom oder die Trifluormethylgruppe und n eine ganze Zahl von 1 bis 3 darstellt,
oder

b) für einen ein- oder zweikernigen, Schwefel, Stickstoff oder Sauerstoff enthaltenden heterocyclischen Rest aus der Gruppe mit den Formeln

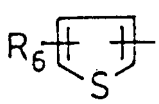 , 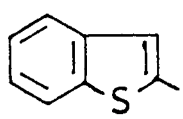 , 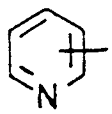 , 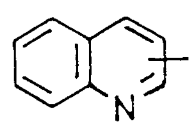 oder 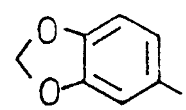

worin $R_6$ ein Wasserstoff- oder ein Chloratom oder eine Methylgruppe bedeutet,

steht und die Stereoisomeren und optisch aktiven Formen der substituierten Phenylacetylene der Formel I.

**2.** Substituierte Phenylacetylene der allgemeinen Formel I aus Anspruch 1, in der $R_1$ und $R_4$ die gleiche Bedeutung wie oben haben, $R_3$ für ein Wasserstoffatom oder für einen Methylrest steht und in der $R_2$

a) für einen ein- oder zweikernigen aromatischen Rest aus der Gruppe mit den Formeln

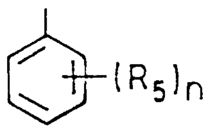 , 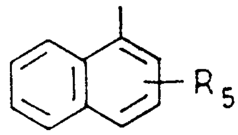 , 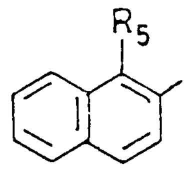 oder 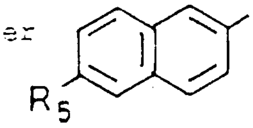

worin $R_5$ ein Wasserstoffatom, eine geradkettige oder verzweigte Alkyl-, Alkoxy- oder Alkylmercaptogruppe mit 1 bis 4 Kohlenstoffatomen, ein Fluor- oder Chloratom oder die Trifluormethylgruppe und n eine ganze Zahl von 1 bis 3 darstellt,

oder

b) für einen ein- oder zweikernigen, Schwefel oder Sauerstoff enthaltenden heterocyclischen Rest aus der Gruppe mit den Formeln

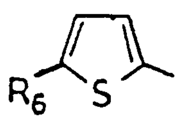 , 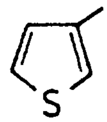 , 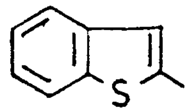 oder 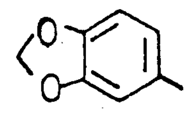

worin $R_6$ ein Wasserstoff- oder ein Chloratom oder eine Methylgruppe bedeutet,

steht und die Stereoisomeren und optisch aktiven Formen der substituierten Phenylacetylene der Formel I.

**3.** Substituierte Phenylacetylene der allgemeinen Formel I aus Anspruch 1, in der $R_1$ und $R_4$ die gleiche Bedeutung wie oben haben, $R_3$ für ein Wasserstoffatom oder für einen Methylrest steht und in der $R_2$

a) für einen ein- oder zweikernigen aromatischen Rest aus der Gruppe mit den Formeln

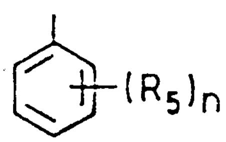 , 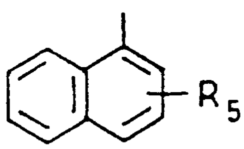 , 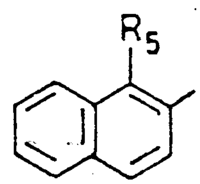 oder 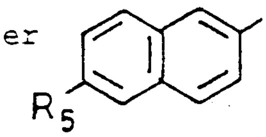

worin $R_5$ ein Wasserstoffatom, eine Methoxygruppe, einen geradkettigen oder verzweigten Alkylrest mit 1 bis 4 Kohlenstoffatomen, ein Fluor- oder Chloratom oder die Trifluormethylgruppe und n eine ganze Zahl von 1 bis 3 darstellt,

oder

b) für einen ein- oder zweikernigen, Schwefel oder Sauerstoff enthaltenden heterocyclischen Rest aus der Gruppe mit den Formeln

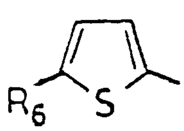 , 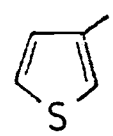 , 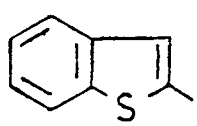 oder 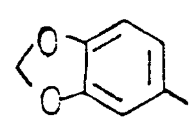

worin $R_6$ ein Wasserstoff- oder ein Chloratom oder eine Methylgruppe bedeutet,

steht und die Stereoisomeren und optisch aktiven Formen dieser substituierten Phenylacetylene der Formel I.

**4.** Substituierte Phenylacetylene gemäß Anspruch 3 der Formel I aus Anspruch 1, in denen $R_2$ eine der Gruppen

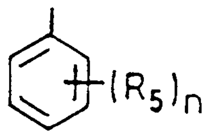 , 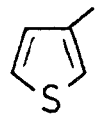 sowie 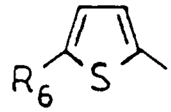

worin $R_5$, $R_6$ und n die in Anspruch 3 angegebene Bedeutung haben, darstellt.

**5.** Substituierte Phenylacetylene der Formel I aus Anspruch 1, in denen $R_2$ der 3-Chinolylrest oder vorzugsweise der 3-Pyridylrest ist.

**6.** Substituierte Phenylacetylene gemäß Anspruch 4, in denen $R_5$ eine Methoxygruppe in 3- und/oder 4-Stellung zur Acetylengruppe darstellt und n eine der Zahlen 1 oder 2 bedeutet.

**7.** Substituierte Phenylacetylene gemäß Anspruch 4, in denen $R_5$ ein Fluor- oder Chloratom in 4-Stellung zur Acetylengruppe darstellt.

**8.** Substituierte Phenylacetylene gemäß Ansprüchen 2 bis 4, in denen $R_6$ ein Wasserstoffatom darstellt.

**9.** Substituierte Phenylacetylene gemäß Ansprüchen 2 bis 4, 6 und 7 der Formel

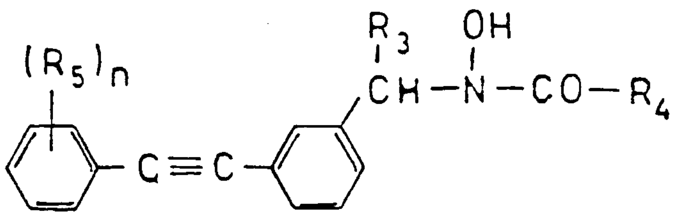

Ia

worin $R_3$ bis $R_5$ und n die gleiche Bedeutung wie oben haben.

**10.** Substituierte Phenylacetylene gemäß Ansprüchen 2 bis 4, 6 und 7 der Formel

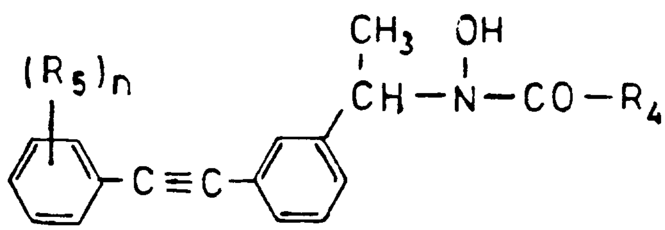

Ib

worin $R_4$ eine Methyl- oder eine Aminogruppe darstellt und $R_5$ für eine Methoxygruppe in 3- und/oder 4-Stellung zur Acetylengruppe (bei n = 1 oder 2) oder für ein Fluor- oder Chloratom in 4-Stellung zur Acetylengruppe steht.

**11.** Substituierte Phenylacetylene gemäß Anspruch 10 der Formel

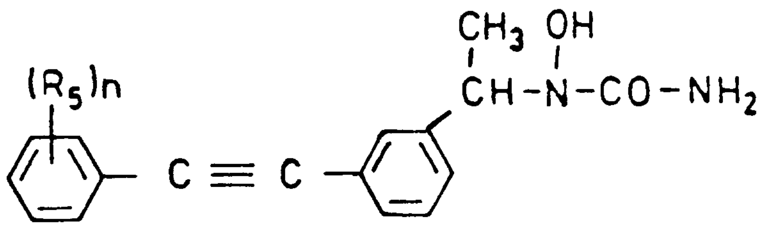

Ic

worin $R_5$ und n die gleiche Bedeutung wie in Anspruch 10 haben.

**12.** Substituierte Phenylacetylene gemäß Ansprüchen 1 bis 4 der Formel

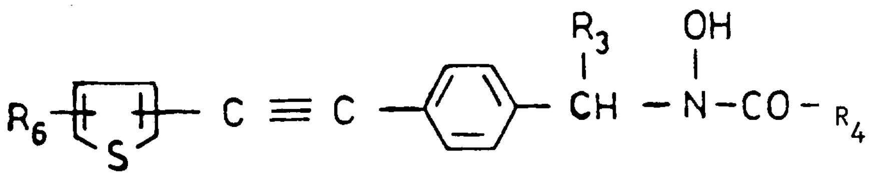

Id

worin $R_3$, $R_4$ und $R_6$ die gleiche Bedeutung wie oben haben.

**13.** Substituierte Phenylacetylene gemäß Ansprüchen 2 bis 4 und 12 der Formel

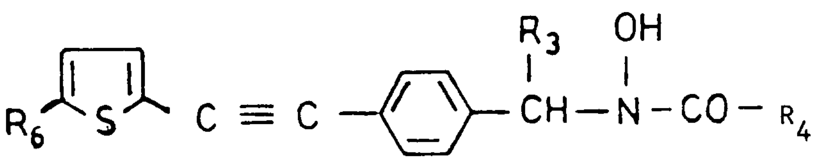

Ie

worin $R_3$ ein Wasserstoffatom oder eine Methylgruppe darstellt und $R_4$ und $R_6$ die gleiche Bedeutung wie oben haben.

**14.** Substituierte Phenylacetylene gemäß Ansprüchen 2 bis 4 der Formel

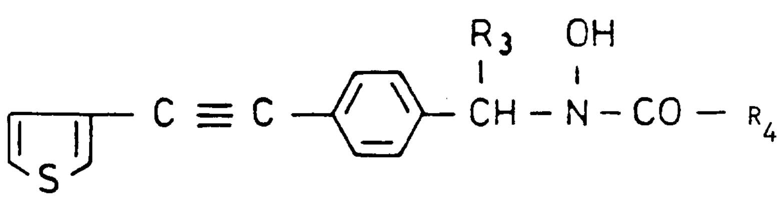

If

worin $R_3$ ein Wasserstoffatom oder die Methylgruppe und $R_4$ eine Methyl- oder eine Aminogruppe darstellt.

**15.** Verfahren zur Herstellung der substituierten Phenylacetylene der Formel I aus Anspruch 1, dadurch gekennzeichnet, daß man

(i) eine Verbindung der Formel

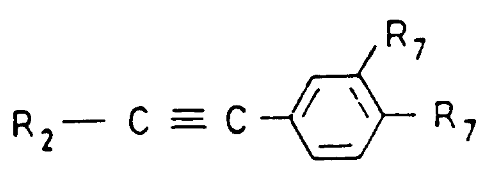

II

in der $R_2$ die gleiche Bedeutung wie oben hat und einer der Reste $R_7$ Wasserstoff, der andere die Gruppe der Formel -CO-$R_3$ bedeutet,
mit Hydroxylamin oder einen von dessen Salzen in Gegenwart von Basen zum Oxim umsetzt, dieses mittels Borhydriden oder Boran-Amin-Komplexen in Gegenwart von Säuren zu einer Verbindung der Formel

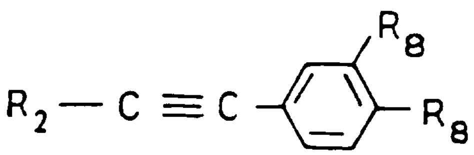

III

in der einer der Reste $R_8$ für Wasserstoff, der andere für die Gruppe der Formel

-CH($R_3$)-NH-OH

steht, reduziert und in diese den Acylrest der Formel -COR$_4$ einführt oder
(ii) eine Verbindung der Formel

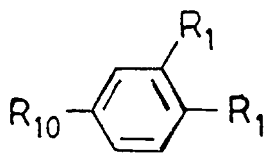

V

worin $R_1$ die gleiche Bedeutung wie oben hat und $R_{10}$ für ein Brom- oder Jodatom steht, mit einer Verbindung der Formel

$R_2$-C≡CH VI

worin $R_2$ die gleiche Bedeutung wie oben hat,
in Gegenwart eines bei etwa 0° - 80° C flüssigen sekundären oder tertiären Amins, insbesondere eines Dialkyl- oder Trialkylamins mit 2 oder 3 Kohlenstoffatomen in jedem der Alkylreste, Pyrrolidin oder Piperidin unter Zugabe von etwa 0,01 bis 5 Prozent pro Mol eingesetzter Verbindung der Formel VI eines komplexen Palladiumkatalysators, insbesondere Bis-(triphenylphosphin)-palladium-(II)-chlorid oder -acetat oder Tetrakis-(triphenylphosphin)-palladium und gegebenenfalls unter Zusatz katalytischer Mengen Kupfer(I)-jodid bei etwa 20° - 80° C zur Reaktion bringt.

**16.** Verfahren zur Herstellung der substituierten Phenylacetylene der Formel I aus Anspruch 1, in denen $R_4$ eine Aminogruppe bedeutet gemäß Anspruch 15, Verfahrensweise (i), dadurch gekennzeichnet, daß man die Verbindung der Formel III
a) mit Trimethylsilyl-isocyanat bei Temperaturen zwischen 20° C und 100° C umsetzt und anschliessend die Trimethylsilylgruppe abspaltet, oder
b) mit Kalium- oder Natriumcyanat in saurer Lösung oder mit Phosgen oder einem Chlorameisensäure(niedrigalkyl)- oder -benzylester in Gegenwart eines säurebindenden Mittels, und anschließend mit Ammoniak oder Ammoniumcarbonat umsetzt.

**17.** Verfahren zur Herstellung der substituierten Phenylacetylene der Formel I aus Anspruch 1, in denen $R_4$ eine Methylgruppe bedeutet, gemäß Anspruch 15, Verfahrensweise (i), dadurch gekennzeichnet, daß man die Verbindung der Formel III mit einem Acetylierungsmittel umsetzt und aus der so erhaltenen Verbindung der Formel

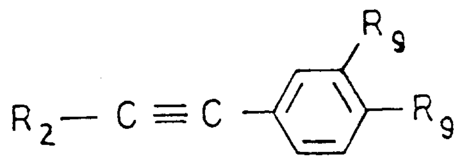

IV

worin einer der Reste $R_9$ ein Wasserstoffatom ist und der andere die Gruppe der Formel

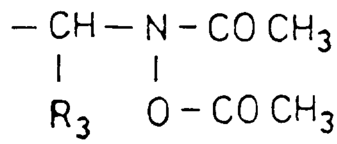

darstellt,
durch selektive basische Hydrolyse die O-Acetylgruppe abspaltet.

**18.** Verfahren gemäß Anspruch 15, Verfahrensweise (i), dadurch gekennzeichnet, daß die Bildung des Oxims der Verbindung der Formel II bei Temperaturen von etwa 20 ° bis 60 °C durchgeführt wird.

**19.** Verfahren gemäß Anspruch 15, Verfahrensweise (i), dadurch gekennzeichnet, daß das Oxim der Verbindung der Formel II mit Natriumcyanoborhydrid in Gegenwart von Säuren bei Temperaturen von etwa 20° C bis 60° C zum Hydroxylaminderivat der Formel III reduziert wird.

**20.** Verfahren gemäß Anspruch 15, Verfahrensweise (i), dadurch gekennzeichnet, daß das Oxim der Verbindung der Formel II mit Boran-Amin-Komplexen in Gegenwart von Säuren bei Temperaturen von etwa 0° bis 20°C zum Hydroxylaminderivat der Formel III reduziert wird.

**Claims**
**Claims for the following Contracting States : AT, BE, CH, DE DK, FR, GB, IT, LI, LU, NL, SE**

**1.** Substituted phenylacetylenes of the general formula

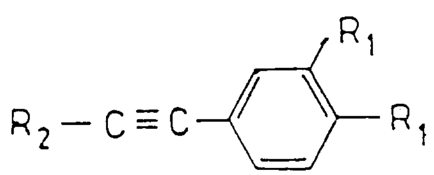

(I)

in which one of the radicals $R_1$ denotes a hydrogen atom, and the other the group of the formula

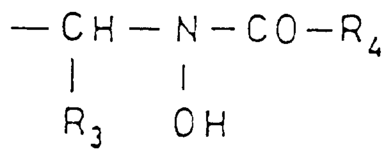

in which $R_3$ represents a hydrogen atom, a methyl radical or an ethyl radical and $R_4$ represents a methyl radical or an amino group and
in which $R_2$ stands

a) for a mononuclear or binuclear aromatic radical taken from the group having the formulae

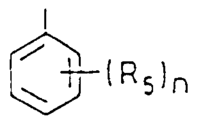, 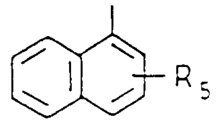, 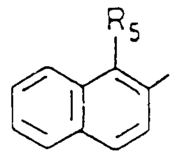 or 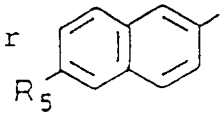

in which $R_5$ represents a hydrogen atom, a benzyloxy group, a straight- or branched-chain alkyl, alkoxy or alkylmercapto group having 1 to 4 carbon atoms, a fluorine or chlorine atom or the trifluoromethyl group, and n is an integer from 1 to 3,

or

b) for a mononuclear or binuclear heterocyclic radical containing sulphur, nitrogen or oxygen, taken from the group having the formulae

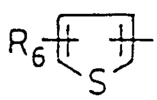, 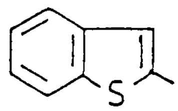, 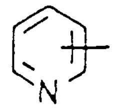, 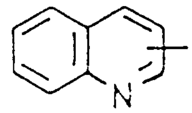 or 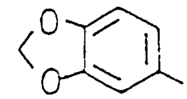

in which $R_6$ denotes a hydrogen or chlorine atom or a methyl group,

and the stereoisomers and optically active forms of the substituted phenylacetylenes of the formula I.

**2.** Substituted phenylacetylenes of the general formula I from Claim 1, in which $R_1$ and $R_4$ denote the same as hereinbefore, $R_3$ stands for a hydrogen atom or a methyl radical and in which $R_2$ stands

a) for a mononuclear or binuclear aromatic radical taken from the group having the formulae

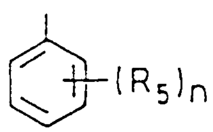, 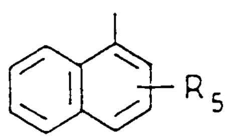, 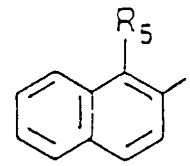 or 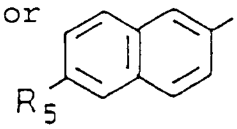

in which $R_5$ represents a hydrogen atom, a straight- or branched-chain alkyl, alkoxy or alkylmercapto group having 1 to 4 carbon atoms, a fluorine or chlorine atom or the trifluoromethyl group, and n represents an integer from 1 to 3,

or

b) for a mononuclear or binuclear heterocyclic radical containing sulphur or oxygen, taken from the group having the formulae

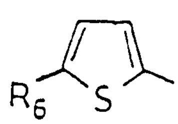, 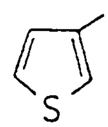, 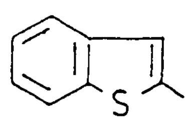 or 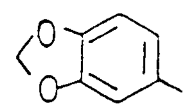

in which $R_6$ denotes a hydrogen or chlorine atom or a methyl group,

and the stereoisomers and optically active forms of the substituted phenylacetylenes of the formula I.

**3.** Substituted phenylacetylenes of the general formula I from Claim 1, in which $R_1$ and $R_4$ denote the same as hereinbefore, $R_3$ stands for a hydrogen atom or a methyl radical and in which $R_2$ stands

a) for a mononuclear or binuclear aromatic radical taken from the group having the formulae

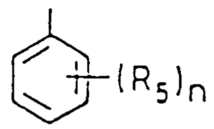 , 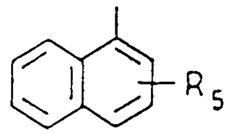 , 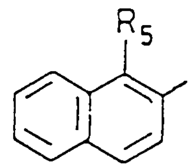 or 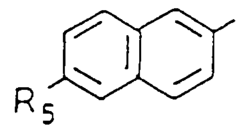

in which $R_5$ represents a hydrogen atom, a methoxy group, a straight- or branched-chain alkyl radical having 1 to 4 carbon atoms, a fluorine or chlorine atom or the trifluoromethyl group, and n represents an integer from 1 to 3,

or

b) for a mononuclear or binuclear heterocyclic radical containing sulphur or oxygen, taken from the group having the formulae

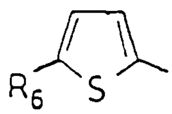 , 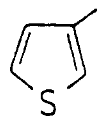 , 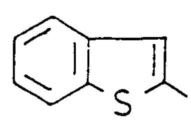 or 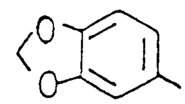

in which $R_6$ denotes a hydrogen or chlorine atom or a methyl group,

and the stereoisomers and optically active forms of the latter substituted phenylacetylenes of the formula I.

**4.** Substituted phenylacetylenes according to Claim 3 of the formula I from Claim 1, in which $R_2$ represents one of the groups

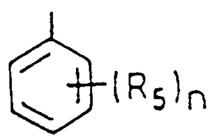 , 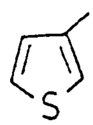 and 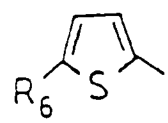

in which $R_5$, $R_6$ and n denote the same as in Claim 2.

**5.** Substituted phenylacetylenes of the formula I from Claim 1, in which $R_2$ is the 3-quinolyl radical or preferably the 3-pyridyl radical.

**6.** Substituted phenylacetylenes according to Claim 4, in which $R_5$ represents a methoxy group in 3rd and/or 4th position relative to the acetylene group, and n denotes one of the numbers 1 or 2.

**7.** Substituted phenylacetylenes according to Claim 4, in which $R_5$ represents a fluorine or chlorine atom in 4th position relative to the acetylene group.

**8.** Substituted phenylacetylenes according to Claims 2 to 4, in which $R_6$ represents a hydrogen atom.

**9.** Substituted phenylacetylenes according to Claims 2 to 4, 6 and 7 of the formula

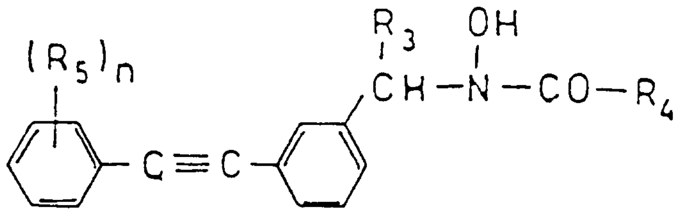

Ia)

in which $R_3$ to $R_5$ and n denote the same as hereinbefore.

**10.** Substituted phenylacetylenes according to Claims 2 to 4, 6 and 7 of the formula

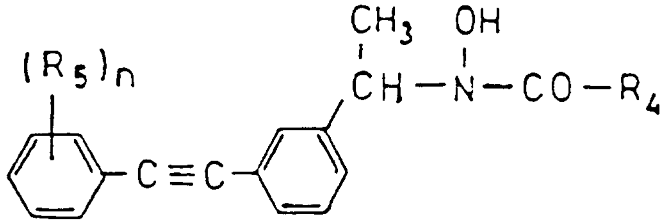

Ib)

in which $R_4$ represents a methyl or amino group and $R_5$ stands for a methoxy group in 3rd and/or 4th position relative to the acetylene group (when n = 1 or 2) or for a fluorine or chlorine atom in 4th position relative to the acetylene group.

**11.** Substituted phenylacetylenes according to Claim 10 of the formula

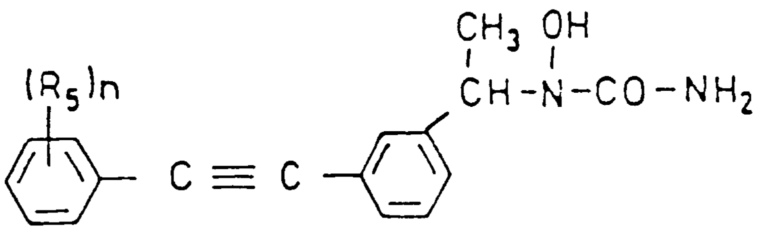

(Ic)

in which $R_5$ and n denote the same as in Claim 10.

**12.** Substituted phenylacetylenes according to Claims 1 to 4 of the formula

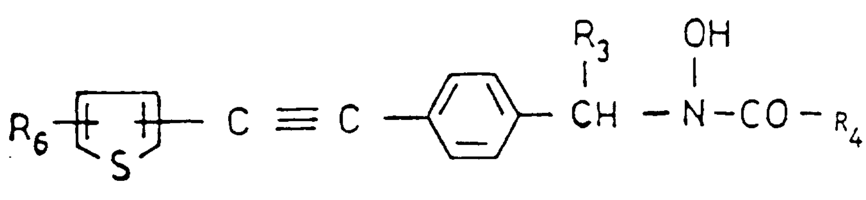

(Id)

in which $R_3$, $R_4$ and $R_6$ denote the same as hereinbefore.

**13.** Substituted phenylacetylenes according to Claims 2 to 4 and 12 of the formula

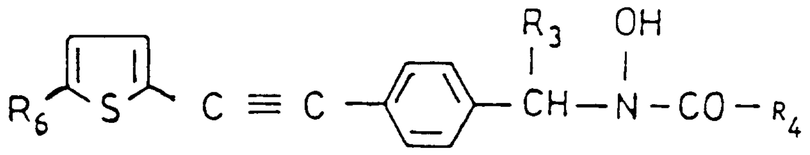

(Ie)

in which $R_3$ represents a hydrogen atom or a methyl group and $R_4$ and $R_6$ denote the same as hereinbefore.

**14.** Substituted phenylacetylenes according to Claims 2 to 4 of the formula

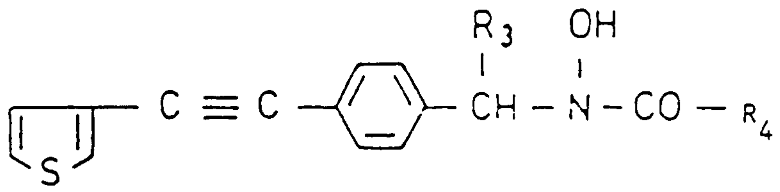

(If)

in which $R_3$ represents a hydrogen atom or the methyl group and $R_4$ represents a methyl or amino group.

**15.** Pharmaceutical preparation, characterised in that it contains as an active ingredient at least one of the substituted phenylacetylenes conforming to Claims 1 to 14 and is suitable for parenteral, oral, intranasal, percutaneous or rectal administration.

**16.** Pharmaceutical preparation according to Claim 15, characterised in that it contains as an active ingredient from 0.01 to 50 mg, per single dose, of a substituted phenylacetylene conforming to Claims 1 to 14.

**17.** Pharmaceutical preparation according to Claims 15 and 16, characterised in that it is suitable for parenteral administration and contains from 0.01 to 10 mg of a substituted phenylacetylene conforming to Claims 1 to 14.

**18.** Pharmaceutical preparation according to Claims 15 and 16, characterised in that it is present in the form of tablets, dragees or capsules, optionally having delayed active ingredient release, and contains from 0.1 to 50 mg of a substituted phenylacetylene conforming to Claims 1 to 14.

**19.** Pharmaceutical preparation according to Claim 15 for intranasal, oral or peroral administration of a substituted phenylacetylene conforming to Claims 1 to 14 in spray form.

**20.** Pharmaceutical preparation according to Claim 15 for percutaneous administration, comprising a reservoir to be applied to the skin containing a substituted phenylacetylene conforming to Claims 1 to 14 in dissolved form, preferably with addition of means which promote skin penetration.

**21.** Process for the preparation of the pharmaceutical preparations according to Claims 15 to 20, characterised in that the substituted phenylacetylenes conforming to Claims 1 to 14 are processed in conventional manner with excipients, solvents and/or diluents, and optionally binders, disintegrants and other auxiliary substances and the single-dose forms are produced from the mixture obtained.

**22.** Process for the preparation of the substituted phenylacetylenes of the formula I from Claim 1, characterised in that

(i) a compound of the formula

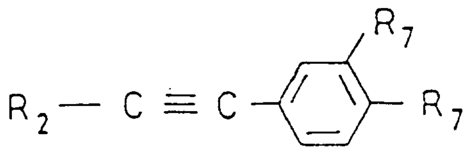

(II)

in which $R_2$ denotes the same as hereinbefore and one of the radicals $R_7$ denotes hydrogen and the other the group of the formula $-CO-R_3$,
is reacted with hydroxylamine or one of the salts thereof in the presence of bases to form the oxime, the latter is reduced by means of borohydrides or borane-amine complexes in the presence of acids to form a compound of the formula

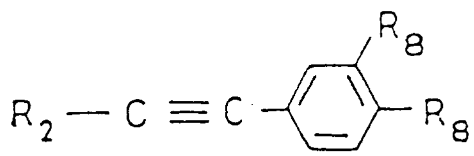

III)

in which one of the radicals $R_8$ stands for hydrogen, and the other for the group of the formula

$-CH(R_3)-NH-OH$

and the acyl radical of the formula $-COR_4$ is introduced into the said compound or
(ii) a compound of the formula

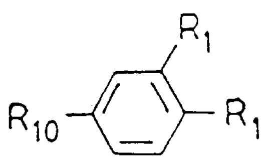

(V)

in which $R_1$ denotes the same as hereinbefore and $R_{10}$ stands for a bromine or iodine atom, is reacted at approximately 20 to 80°C with a compound of the formula

$R_2-C \equiv CH$ (VI)

in which $R_2$ denotes the same as hereinbefore,
in the presence of a secondary or tertiary amine which is liquid at approximately 0 to 80°C, in particular a dialkylamine or trialkylamine having 2 or 3 carbon atoms in each alkyl radical, pyrrolidine or piperidine, with addition of from approximately 0.01 to 5 per cent of a complex palladium catalyst, in particular bis(triphenylphosphine)-palladium(II) chloride or bis(triphenylphosphine)-palladium(II) acetate or tetrakis(triphenylphosphine)-palladium, per mole of compound of the formula VI utilised, and optionally with addition of catalytic quantities of copper (I) iodide.

**23.** Process for the preparation of the substituted phenylacetylenes of the formula I from Claim 1, in which $R_4$ denotes an amino group according to Claim 22, method (i), characterised in that the compound of the formula III

a) is reacted with trimethylsilyl isocyanate at temperatures between 20 and 100°C, followed by dissociation of the trimethylsilyl group, or

b) is reacted with potassium cyanate or sodium cyanate in acidic solution or with phosgene or a chloroformic acid-(low alkyl) ester or -benzyl ester in the presence of an acid-binding agent, and then with ammonia or ammonium carbonate.

**24.** Process for the preparation of the substituted phenylacetylenes of the formula I from Claim 1, in which $R_4$ denotes a methyl group, according to Claim 22, method (i), characterised in that the compound of the formula III is reacted with an acetylating agent and the O-acetyl group is dissociated by selective basic hydrolysis from the compound of the formula

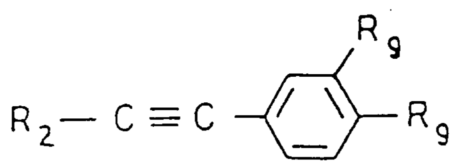

(IV)

thus obtained, in which one of the radicals $R_9$ is a hydrogen atom and the other represents the group of the formula

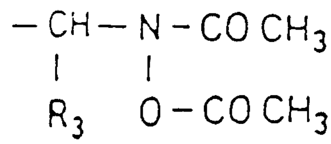

**25.** Process according to Claim 22, method (i), characterised in that the oxime of the compound of the formula II is formed at temperatures of from approximately 20 to 60 °C.

**26.** Process according to Claim 22, method (i), characterised in that the oxime of the compound of the formula II is reduced with sodium cyanoborohydride in the presence of acids at temperatures of from approximately 20 to 60 °C to form the hydroxylamine derivative of the formula III.

**27.** Process according to Claim 22, method (i), characterised in that the oxime of the compound of the formula II is reduced with borane-amine complexes in the presence of acids at temperatures of from approximately 0 to 20 °C to form the hydroxylamine derivative of the formula III.

**Claims for the following Contracting State : ES**

**1.** Process for the preparation of substituted phenylacetylenes of the general formula

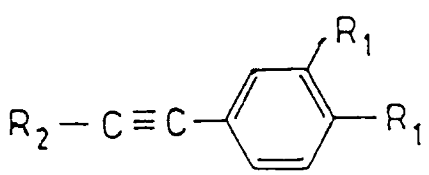

(I)

in which one of the radicals $R_1$ denotes a hydrogen atom, and the other the group of the formula

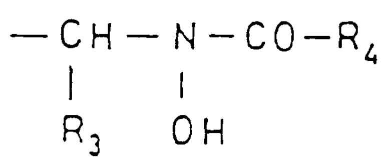

in which $R_3$ represents a hydrogen atom, a methyl radical or an ethyl radical and $R_4$ represents a methyl radical or an amino group and
in which $R_2$ stands

a) for a mononuclear or binuclear aromatic radical taken from the group having the formulae

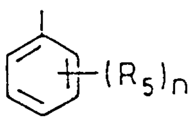 , 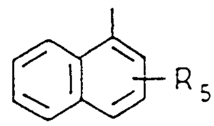 , 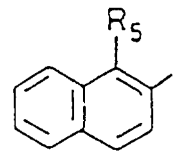 or 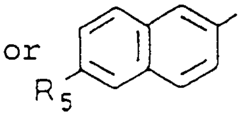

in which $R_5$ represents a hydrogen atom, a benzyloxy group, a straight- or branched-chain alkyl, alkoxy or alkylmercapto radical having 1 to 4 carbon atoms, a fluorine or chlorine atom or the trifluoromethyl group, and n is an integer from 1 to 3,
or
b) for a mononuclear or binuclear heterocyclic radical containing sulphur, nitrogen or oxygen, taken from the group having the formulae

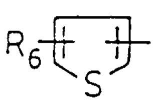 , 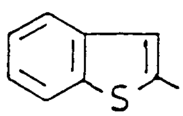 , 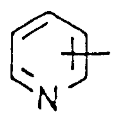 , 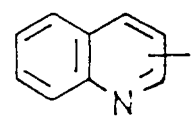 or 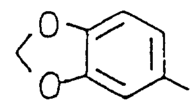

in which $R_6$ denotes a hydrogen or chlorine atom or a methyl group,
and the stereoisomers and optically active forms of the latter substituted phenylacetylenes of the formula I, characterised in that
(i) a compound of the formula

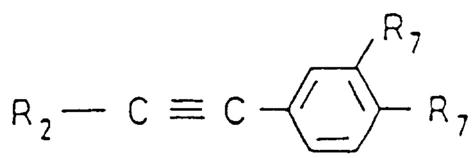

(II)

in which $R_2$ denotes the same as hereinbefore and one of the radicals $R_7$ denotes hydrogen and the other the group of the formula $-CO-R_3$,
is reacted with hydroxylamine or one of the salts thereof in the presence of bases to form the oxime, the latter is reduced by means of borohydrides or borane-amine complexes in the presence of acids to form a compound of the formula

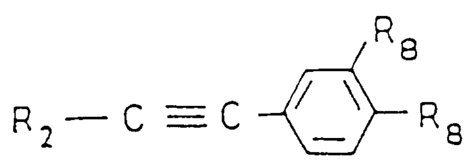

(III)

in which one of the radicals $R_8$ stands for hydrogen, and the other for the group of the formula $-CH(R_3)-NH-OH$
and the acyl radical of the formula $-COR_4$ is introduced into the said compound or
(ii) a compound of the formula

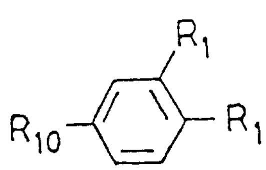

(V)

in which $R_1$ denotes the same as hereinbefore and $R_{10}$ stands for a bromine or iodine atom, is reacted at approximately 20 to 80 °C with a compound of the formula

$R_2$-C≡CH (VI)

in which $R_2$ denotes the same as hereinbefore,

in the presence of a secondary or tertiary amine which is liquid at approximately 0 to 80 °C, in particular a dialkylamine or trialkylamine having 2 or 3 carbon atoms in each alkyl radical, pyrrolidine or piperidine, with addition of from approximately 0.01 to 5 per cent of a complex palladium catalyst, in particular bis(triphenylphosphine)-palladium(II) chloride or bis(triphenylphosphine)-palladium(II) acetate or tetrakis(triphenylphosphine)-palladium, per mole of compound of the formula VI utilised, and optionally with addition of catalytic quantities of copper (I) iodide.

**2.** Process according to Claim 1 for the preparation of substituted phenylacetylenes of the general formula I from Claim 1, characterised in that in the latter compounds
$R_1$ and $R_4$ denote the same as hereinbefore,
$R_3$ stands for a hydrogen atom or a methyl radical and
$R_2$ stands for a mononuclear or binuclear aromatic radical taken from the group having the formulae

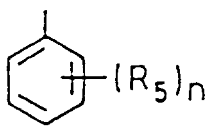 , 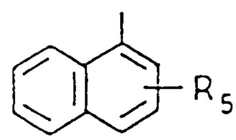 , 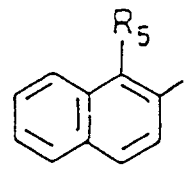 or 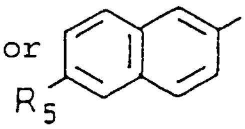

in which $R_5$ represents a hydrogen atom, a methoxy group, a straight- or branched-chain alkyl radical having 1 to 4 carbon atoms, a fluorine or chlorine atom or the trifluoromethyl group, and n represents an integer from 1 to 3,
or
for a mononuclear or binuclear heterocyclic radical containing sulphur or oxygen, taken from the group having the formulae

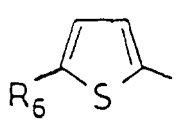 , 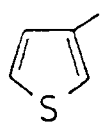 , 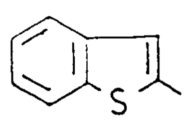 or 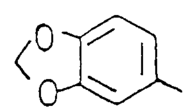

in which $R_6$ denotes a hydrogen or chlorine atom or a methyl group.

**3.** Process for the preparation of the substituted phenylacetylenes of the formula I from Claims 1 and 2, in which $R_4$ denotes an amino group according to Claim 1, method (i), characterised in that the compound of the formula III

a) is reacted with trimethylsilyl isocyanate at temperatures between 20 and 100 °C, followed by dissociation of the trimethylsilyl group, or

b) is reacted with potassium cyanate or sodium cyanate in acidic solution or with phosgene or a chloroformic acid-(low alkyl) ester or -benzyl ester in the presence of an acid-binding agent, and then with ammonia or ammonium carbonate.

**4.** Process for the preparation of the substituted phenylacetylenes of the formula I from Claims 1 and 2, in which $R_4$ denotes a methyl group, according to Claim 1, method (i), characterised in that the compound of the formula III is reacted with an acetylating agent and the O-acetyl group is dissociated by selective basic hydrolysis from the compound of the formula

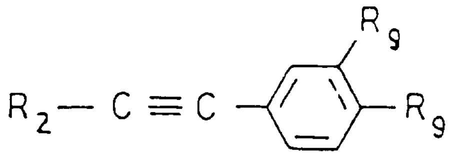

(IV)

thus obtained, in which one of the radicals $R_9$ is a hydrogen atom and the other represents the group of the formula

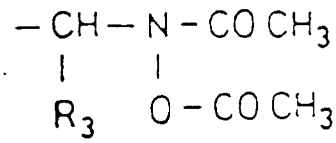

5. Process according to Claim 1, method (i), characterised in that the oxime of the compound of the formula II is formed at temperatures of from approximately 20 to 60 ° C.

6. Process according to Claim 1, method (i), characterised in that the oxime of the compound of the formula II is reduced with sodium cyanoborohydride in the presence of acids at temperatures of from approximately 20 to 60 ° C to form the hydroxylamine derivative of the formula III.

7. Process according to Claim 1, method (i), characterised in that the oxime of the compound of the formula II is reduced with borane-amine complexes in the presence of acids at temperatures of from approximately 0 to 20 ° C to form the hydroxylamine derivative of the formula III.

**Claims for the following Contracting State : GR**

1. Substituted phenylacetylenes of the general formula

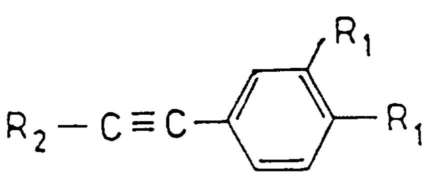

(I)

in which one of the radicals $R_1$ denotes a hydrogen atom, and the other the group of the formula

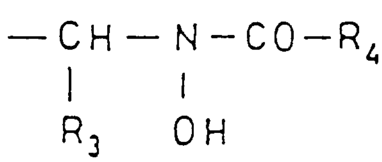

in which $R_3$ represents a hydrogen atom, a methyl radical or an ethyl radical and $R_4$ represents a methyl radical or an amino group and
in which $R_2$ stands

a) for a mononuclear or binuclear aromatic radical taken from the group having the formulae

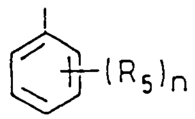 , 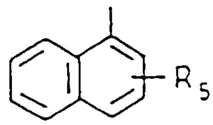 , 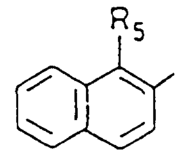 or 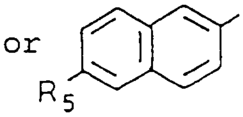

in which $R_5$ represents a hydrogen atom, a benzyloxy group, a straight- or branched-chain alkyl, alkoxy or alkylmercapto group having 1 to 4 carbon atoms, a fluorine or chlorine atom or the trifluoromethyl group, and n is an integer from 1 to 3,

or

b) for a mononuclear or binuclear heterocyclic radical containing sulphur, nitrogen or oxygen, taken from the group having the formulae

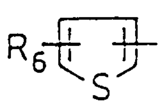 , 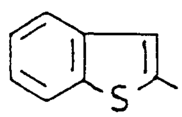 , 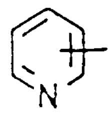 , 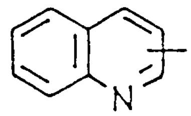 or 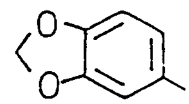

in which $R_6$ denotes a hydrogen or chlorine atom or a methyl group,

and the stereoisomers and optically active forms of the substituted phenylacetylenes of the formula I.

**2.** Substituted phenylacetylenes of the general formula I from Claim 1, in which $R_1$ and $R_4$ denote the same as hereinbefore, $R_3$ stands for a hydrogen atom or a methyl radical, and in which $R_2$ stands

a) for a mononuclear or binuclear aromatic radical taken from the group having the formulae

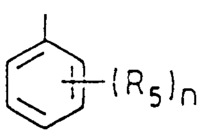 , 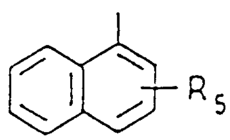 , 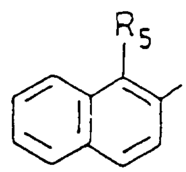 or 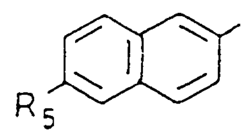

in which $R_5$ represents a hydrogen atom, a straight- or branched-chain alkyl, alkoxy or alkylmercapto group having 1 to 4 carbon atoms, a fluorine or chlorine atom or the trifluoromethyl group, and n represents an integer from 1 to 3,

or

b) for a mononuclear or binuclear heterocyclic radical containing sulphur or oxygen, taken from the group having the formulae

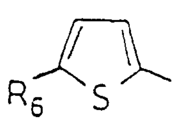 , 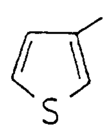 , 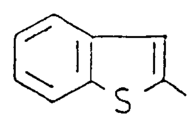 or 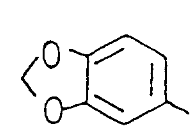

in which $R_6$ denotes a hydrogen or chlorine atom or a methyl group,

and the stereoisomers and optically active forms of the substituted phenylacetylenes of the formula I.

**3.** Substituted phenylacetylenes of the general formula I from Claim 1, in which $R_1$ and $R_4$ denote the same as hereinbefore, $R_3$ stands for a hydrogen atom or a methyl radical, and in which $R_2$ stands

a) for a mononuclear or binuclear aromatic radical taken from the group having the formulae

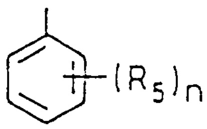 , 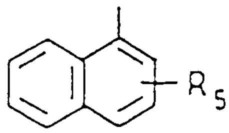 , 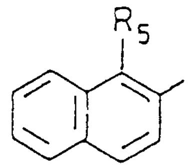 or 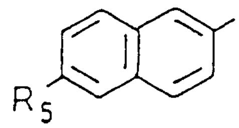

in which $R_5$ represents a hydrogen atom, a methoxy group, a straight- or branched-chain alkyl radical having 1 to 4 carbon atoms, a fluorine or chlorine atom or the trifluoromethyl group, and n represents an integer from 1 to 3,

or

b) for a mononuclear or binuclear heterocyclic radical containing sulphur or oxygen, taken from the group having the formulae

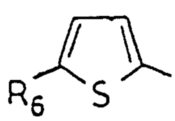 , 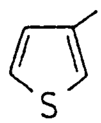 , 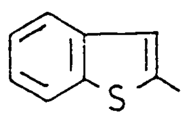 or 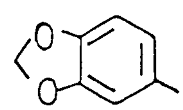

in which $R_6$ denotes a hydrogen or chlorine atom or a methyl group,

and the stereoisomers and optically active forms of the latter substituted phenylacetylenes of the formula I.

**4.** Substituted phenylacetylenes according to Claim 3 of the formula I from Claim 1, in which $R_2$ represents one of the groups

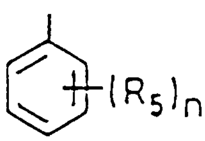 , 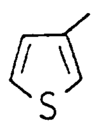 and 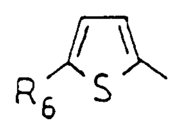

in which $R_5$, $R_6$ and n denote the same as in Claim 3.

**5.** Substituted phenylacetylenes of the formula I from Claim 1, in which $R_2$ is the 3-quinolyl radical or preferably the 3-pyridyl radical.

**6.** Substituted phneylacetylenes according to Claim 4, in which $R_5$ represents a methoxy group in 3rd and/or 4th position relative to the acetylene group, and n denotes one of the numbers 1 or 2.

**7.** Substituted phneylacetylenes according to Claim 4, in which $R_5$ represents a fluorine or chlorine atom in 4th position relative to the acetylene group.

**8.** Substituted phenylacetylenes according to Claims 2 to 4, in which $R_6$ represents a hydrogen atom.

**9.** Substituted phenylacetylenes according to Claims 2 to 4, 6 and 7 of the formula

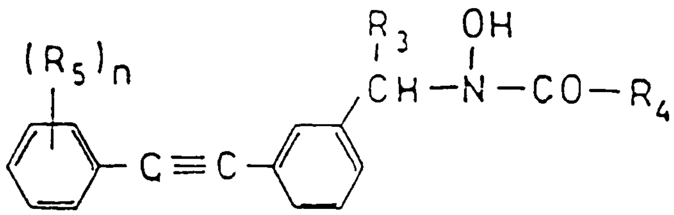

Ia)

in which $R_3$ to $R_5$ and n denote the same as hereinbefore.

**10.** Substituted phenylacetylenes according to Claims 2 to 4, 6 and 7 of the formula

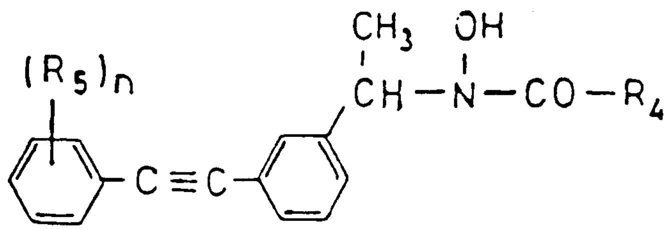

Ib)

in which $R_4$ represents a methyl or amino group and $R_5$ stands for a methoxy group in 3rd and/or 4th position relative to the acetylene group (when n = 1 or 2) or for a fluorine or chlorine atom in 4th position relative to the acetylene group.

**11.** Substituted phenylacetylenes according to Claim 10 of the formula

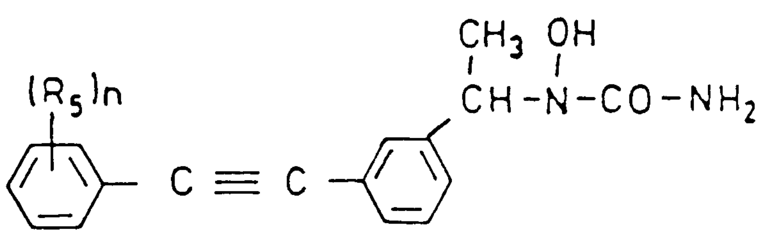

(Ic)

in which $R_5$ and n denote the same as in Claim 10.

**12.** Substituted phenylacetylenes according to Claims 1 to 4 of the formula

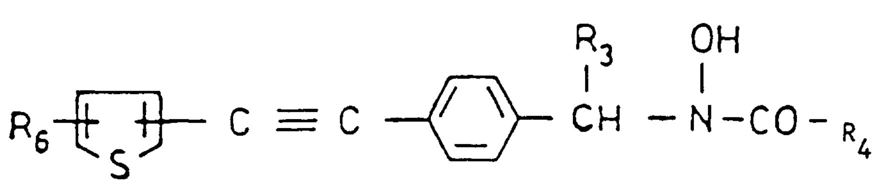

(Id)

in which $R_3$, $R_4$ and $R_6$ denote the same as hereinbefore.

**13.** Substituted phenylacetylenes according to Claims 2 to 4 and 12 of the formula

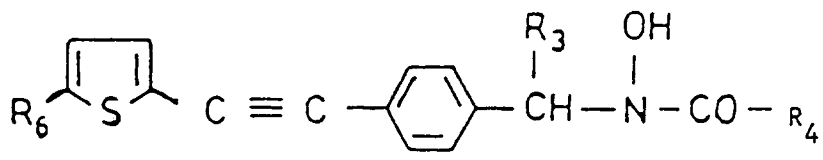

(Ie)

in which $R_3$ represents a hydrogen atom or a methyl group and $R_4$ and $R_6$ denote the same as hereinbefore.

**14.** Substituted phenylacetylenes according to Claims 2 to 4 of the formula

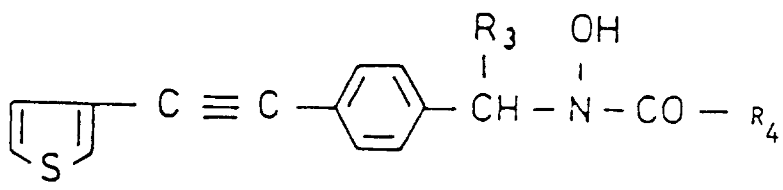

(If)

in which $R_3$ represents a hydrogen atom or the methyl group, and $R_4$ represents a methyl or an amino group.

**15.** Process for the preparation of the substituted phenylacetylenes of the formula I from Claim 1, characterised in that

(i) a compound of the formula

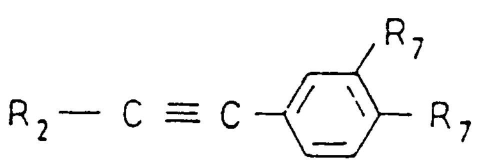

(II)

in which $R_2$ denotes the same as hereinbefore and one of the radicals $R_7$ denotes hydrogen and the other the group of the formula $-CO-R_3$,
is reacted with hydroxylamine or one of the salts thereof in the presence of bases to form the oxime, the latter is reduced by means of borohydrides or borane-amine complexes in the presence of acids to form a compound of the formula

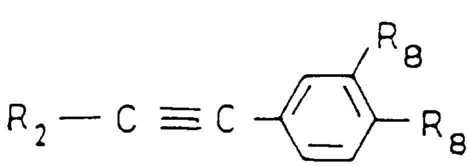

(III)

in which one of the radicals $R_8$ stands for hydrogen, and the other for the group of the formula

$-CH(R_3)-NH-OH$

and the acyl radical of the formula $-COR_4$ is introduced into the said compound or

(ii) a compound of the formula

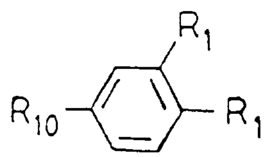

(V)

in which $R_1$ denotes the same as hereinbefore and $R_{10}$ stands for a bromine or iodine atom, is reacted at approximately 20 to 80 ° C with a compound of the formula

$R_2$-C≡CH (VI)

in which $R_2$ denotes the same as hereinbefore, in the presence of a secondary or tertiary amine which is liquid at approximately 0 to 80 ° C, in particular a dialkylamine or trialkylamine having 2 or 3 carbon atoms in each alkyl radical, pyrrolidine or piperidine, with addition of approximately 0.01 to 5 per cent of a complex palladium catalyst, in particular bis(triphenylphosphine)-palladium(II) chloride or bis(triphenylphosphine)-palladium(II) acetate or tetrakis(triphenylphosphine)-palladium, per mole of compound of the formula VI utilised, and optionally with addition of catalytic quantities of copper(I) iodide.

**16.** Process for the preparation of the substituted phenylacetylenes of the formula I from Claim 1, in which $R_4$ denotes an amino group according to Claim 15, method (i), characterised in that the compound of the formula III
a) is reacted with trimethylsilyl isocyanate at temperatures between 20 and 100 ° C, followed by dissociation of the trimethylsilyl group, or
b) is reacted with potassium cyanate or sodium cyanate in acidic solution or with phosgene or a chloroformic acid-(low alkyl) ester or -benzyl ester in the presence of an acid-binding agent, and then with ammonia or ammonium carbonate.

**17.** Process for the preparation of the substituted phenylacetylenes of the formula I from Claim 1, in which $R_4$ denotes a methyl group, according to Claim 15, method (i), characterised in that the compound of the formula III is reacted with an acetylating agent and the O-acetyl group is dissociated by selective basic hydrolysis from the compound of the formula

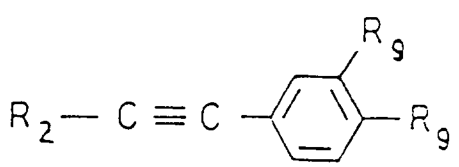

(IV)

thus obtained, in which one of the radicals $R_9$ represents a hydrogen atom and the other the group of the formula

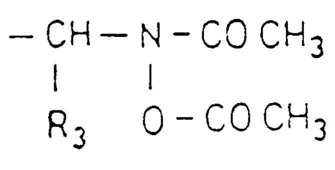

**18.** Process according to Claim 15, method (i), characterised in that the oxime of the compound of the formula II is formed at temperatures of from approximately 20 to 60 ° C.

**19.** Process according to Claim 15, method (i), characterised in that the oxime of the compound of the formula II is reduced with sodium cyanoborohydride in the presence of acids at temperatures of from approximately 20 to 60 ° C to form the hydroxylamine derivative of the formula III.

**20.** Process according to Claim 15, method (i), characterised in that the oxime of the compound of the formula II is reduced with borane-amine complexes in the presence of acids at temperatures of from approximately 0 to 20 ° C to form the hydroxylamine derivative of the formula III.

**Revendications**

**Revendications pour les Etats contractants suivants : AT, BE, CH, DE, DK, FR, GB, IT, LI, LU, NL, SE**

**1.** Phénylacétylènes substitués de formule générale

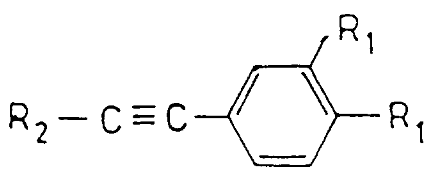

I

dans laquelle l'un des restes $R_1$ est un atome d'hydrogène, l'autre est le groupe de formule

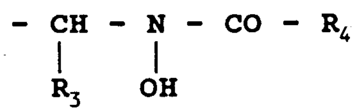

dans laquelle $R_3$ est un atome d'hydrogène, un reste méthyle ou un reste éthyle et $R_4$ est un reste méthyle ou un groupe amino

et dans laquelle $R_2$ représente :

a) un reste aromatique monocyclique ou bicyclique choisi dans le groupe des restes de formules

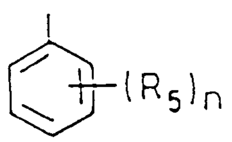
,
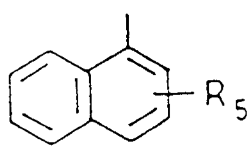
,
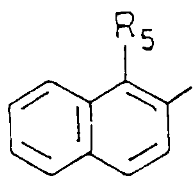
ou
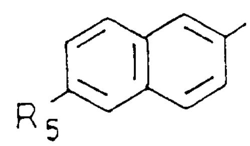

dans lesquelles $R_5$ est un atome d'hydrogène, un groupe benzyloxy, un groupe alkyl-, alkoxy- ou alkylmercapto linéaire ou ramifié ayant 1 à 4 atomes de carbone, un atome de fluor ou de chlore ou le groupe trifluorométhyle et n est un nombre entier de 1 à 3,

ou bien

b) un reste hétérocyclique à un ou deux noyaux, contenant du soufre, de l'azote ou de l'oxygène, choisi dans le groupe des restes de formules

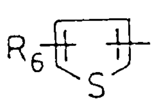
,
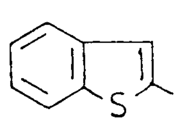
,
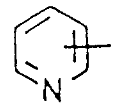
,
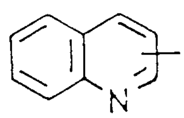
ou
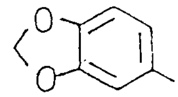

où $R_6$ désigne un atome d'hydrogène ou de chlore ou un groupe méthyle

et les stéréoisomères et les formes optiquement actives des phénylacétylènes substitués de formule I.

2. Phénylacétylènes substitués de formule générale I suivant la revendication 1, dans laquelle $R_1$ et $R_4$ ont la même définition que ci-dessus, $R_3$ représente un atome d'hydrogène ou un reste méthyle, et dans laquelle $R_2$ représente

a) un reste aromatique monocyclique ou bicyclique du groupe des restes de formules

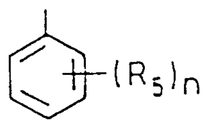 , 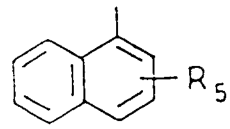 , 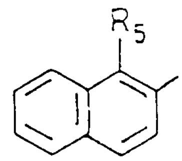 ou 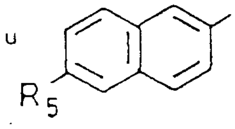

dans lesquelles $R_5$ représente un atome d'hydrogène, un groupe alkyl-, alkoxy- ou alkylmercapto linéaire ou ramifié ayant 1 à 4 atomes de carbone, un atome de fluor ou de chlore ou le groupe trifluorométhyle et n est un nombre entier de 1 à 3,

ou bien

b) un reste hétérocyclique à un ou deux noyaux, contenant du soufre ou de l'oxygène, choisi dans le groupe des restes de formules

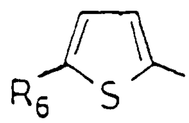 , 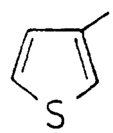 , 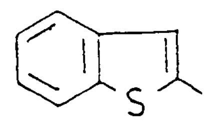 ou 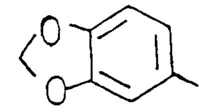

où $R_6$ représente un atome d'hydrogène ou de chlore ou un groupe méthyle

et les stéréoisomères et les formes optiquement actives des phénylacétylènes substitués de formule I.

3. Phénylacétylènes substitués de formule générale I suivant la revendication 1, dans laquelle $R_1$ et $R_4$ ont la même définition que ci-dessus, $R_3$ est un atome d'hydrogène ou un reste méthyle et dans laquelle $R_2$ représente

a) un reste aromatique monocyclique ou bicyclique du groupe des restes de formules

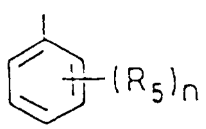 , 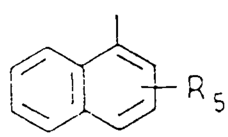 , 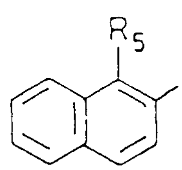 ou 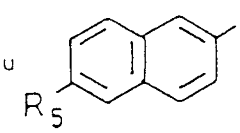

dans lesquelles $R_5$ est un atome d'hydrogène, un groupe méthoxy, un reste alkyle linéaire ou ramifié ayant 1 à 4 atomes de carbone, un atome de fluor ou de chlore ou le groupe trifluorométhyle et n est un nombre entier de 1 à 3,

ou bien

b) un reste hétérocyclique à un ou deux noyaux, contenant du soufre ou de l'oxygène, du groupe des restes de formules

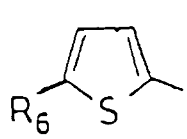 , 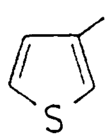 , 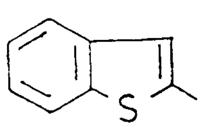 ou 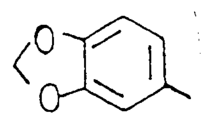

où $R_6$ représente un atome d'hydrogène ou de chlore ou un groupe méthyle,

et les stéréoisomères et les formes optiquement actives de ces phénylacétylènes substitués de

formule I.

4. Phénylacétylènes substitués suivant la revendication 3 de formule I suivant la revendication 1, dans lesquels $R_2$ représente l'un des groupes

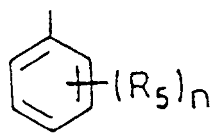 , 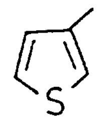 ainsi que 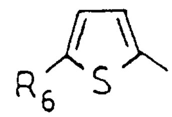

où $R_5$, $R_6$ et n ont la définition indiquée dans la revendication 2.

5. Phénylacétylènes substitués de formule I suivant la revendication 1, dans lesquels $R_2$ représente le reste 3-quinolyle ou de préférence le reste 3-pyridyle.

6. Phénylacétylènes substitués suivant la revendication 4, dans lesquels $R_5$ est un groupe méthoxy en position 3 et/ou en position 4 par rapport au groupe acétylène et n est l'un des nombres 1 et 2.

7. Phénylacétylènes substitués suivant la revendication 4, dans lesquels $R_5$ est un atome de fluor ou de chlore en position 4 par rapport au groupe acétylène.

8. Phénylacétylènes substitués suivant les revendications 2 à 4, dans lesquels $R_6$ est un atome d'hydrogène.

9. Phénylacétylènes substitués suivant les revendications 2 à 4, 6 et 7, de formule

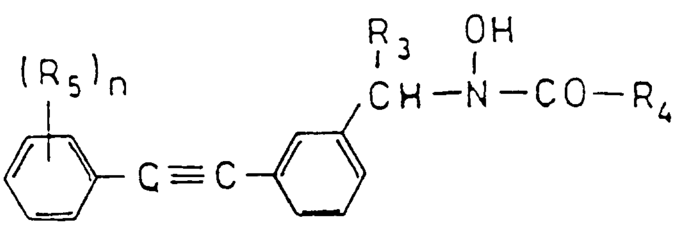

Ia

dans laquelle $R_3$ à $R_5$ et n ont la même définition que ci-dessus.

10. Phénylacétylènes substitués suivant les revendications 2 à 4, 6 et 7, de formule

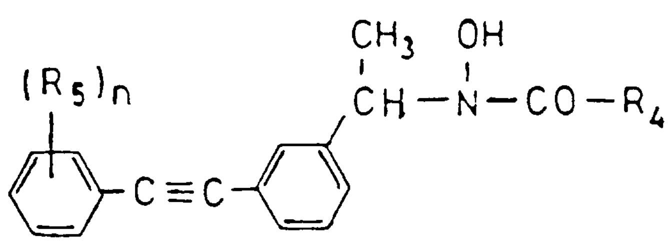

Ib

dans laquelle $R_4$ représente un groupe méthyle ou un groupe amino et $R_5$ est un groupe méthoxy en position 3 et/ou en position 4 par rapport au groupe acétylène (pour n = 1 ou 2) ou un atome de fluor ou de chlore en position 4 par rapport au groupe acétylène.

**11.** Phénylacétylènes substitués suivant la revendication 10, de formule

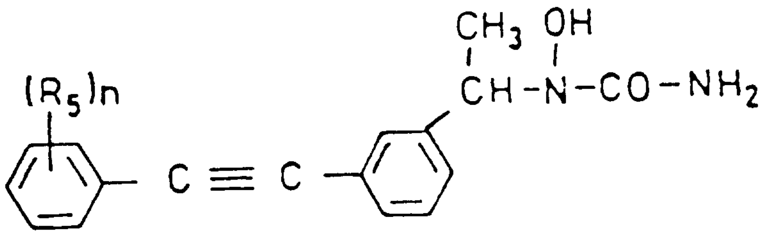

Ic

dans laquelle $R_5$ et n ont la même définition que dans la revendication 10.

**12.** Phénylacétylènes substitués suivant les revendications 1 à 4, de formule

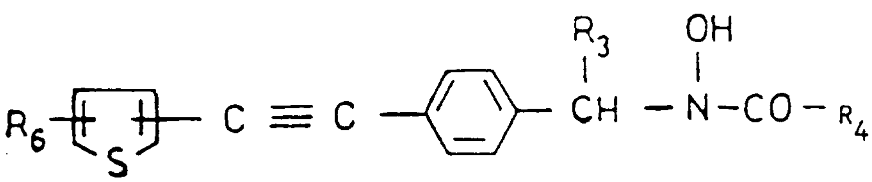

Id

dans laquelle $R_3$, $R_4$ et $R_6$ ont la même définition que ci-dessus.

**13.** Phénylacétylènes substitués suivant les revendications 2 à 4 et 12, de formule

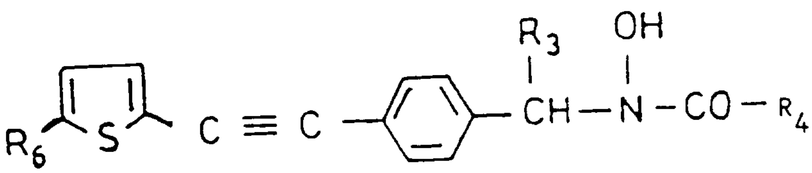

Ie

dans laquelle $R_3$ est un atome d'hydrogène ou un groupe méthyle et $R_4$ et $R_6$ ont la même définition que ci-dessus.

**14.** Phénylacétylènes substitués suivant les revendications 2 à 4, de formule

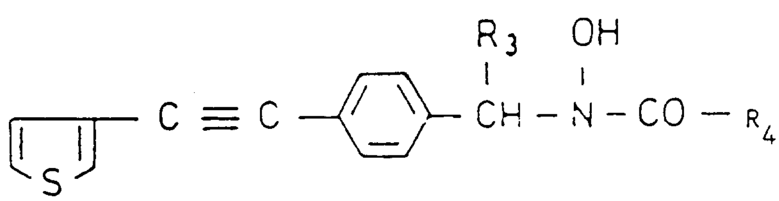

If

dans laquelle $R_3$ représente un atome d'hydrogène ou le groupe méthyle et $R_4$ est un groupe méthyle ou un groupe amino.

**15.** Médicament, caractérisé en ce qu'il contient comme substance active au moins l'un des phénylacétylènes substitués suivant les revendications 1 à 14 et en ce qu'il convient pour l'administration parentérale, orale, intranasale, percutanée ou rectale.

**16.** Médicament suivant la revendication 15, caractérisé en ce qu'il contient comme substance active, par dose unitaire, 0,01 à 50 mg d'un phénylacétylène substitué suivant les revendications 1 à 14.

**17.** Médicament suivant les revendications 15 et 16, caractérisé en ce qu'il convient pour l'administration parentérale et contient 0,01 à 10 mg d'un phénylacétylène substitué suivant les revendications 1 à 14.

**18.** Médicament suivant les revendications 15 et 16, caractérisé en ce qu'il se présente sous la forme de comprimés, de dragées ou de gélules, le cas échéant avec libération retardée de la substance active, et contient 0,1 à 50 mg d'un phénylacétylène substitué suivant les revendications 1 à 14.

**19.** Médicament suivant la revendication 15 destiné à l'administration intranasale, orale ou perorale d'un phénylacétylène substitué correspondant aux revendications 1 à 14, sous une forme pulvérisable.

**20.** Médicament suivant la revendication 15 destiné à l'administration percutanée, constitué d'un réservoir devant être appliqué à la peau, contenant un phénylacétylène substitué suivant les revendications 1 à 14 sous la forme dissoute, de préférence avec addition d'agents favorisant la pénétration à travers la peau.

**21.** Procédé d'obtention des médicaments suivant les revendications 15 à 20, caractérisé en ce qu'on formule les phénylacétylènes substitués suivant les revendications 1 à 14 d'une manière classique avec des excipients, des solvants ou des diluants ainsi que le cas échéant des liants, des agents de désintégration pour comprimés et d'autres substances auxiliaires et on prépare les formes dosées unitaires à partir du mélange obtenu.

**22.** Procédé de production des phénylacétylènes substitués de formule I suivant la revendication 1, caractérisé en ce que :

(i) on fait réagir un composé de formule

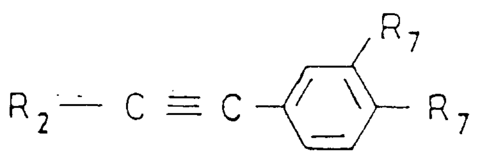

II

dans laquelle $R_2$ a la même définition que ci-dessus et l'un des restes $R_7$ représente de l'hydrogène, l'autre représente le groupe de formule $-CO-R_3$, avec l'hydroxylamine ou l'un de ses sels en présence de bases pour former une oxime, on réduit cette dernière au moyen de borohydrures ou de complexes borane-amine en présence d'acides en un composé de formule

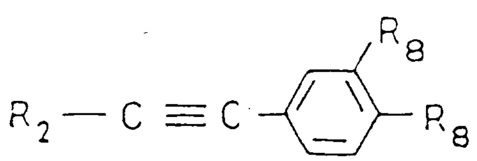

III

dont l'un des restes $R_8$ représente de l'hydrogène, l'autre est le groupe de formule

$- CH(R_3) - NH - OH$

et on introduit dans ce composé le reste acyle de formule $-COR_4$, ou

ii) on fait réagir à une température d'environ 20 à 80 °C un composé de formule

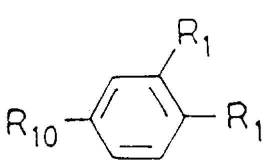

V

dans laquelle $R_1$ a la même définition que ci-dessus et $R_{10}$ représente un atome de brome ou d'iode, avec un composé de formule

$R_2 - C \equiv CH$ VI

dans laquelle $R_2$ a la même définition que ci-dessus,
en présence d'une amine secondaire ou tertiaire liquide à environ 0-80 °C, notamment d'une dialkyl- ou trialkylamine ayant 2 ou 3 atomes de carbone dans chacun des restes alkyle, la pyrrolidine ou la pipéridine avec addition d'environ 0,01 à 5 %, par mole de composé utilisé de formule VI, d'un catalyseur complexe au palladium, notamment le chlorure ou l'acétate de bis-(triphénylphosphine)-palladium (II) ou le tétrakis-(triphénylphosphine)-palladium et en ajoutant le cas échéant des quantités catalytiques d'iodure de cuivre (I).

**23.** Procédé de production des phénylacétylènes substitués de formule I suivant la revendication 1, dans lesquels $R_4$ désigne un groupe amino, suivant le mode opératoire (i) de la revendication 22, caractérisé en ce qu'on fait réagir le composé de formule III

a) avec le triméthylsilylisocyanate à des températures comprises entre 20 °C et 100 °C puis on élimine le groupe triméthylsilyle, ou bien

b) avec le cyanate de potassium ou de sodium en solution acide ou avec du phosgène ou un ester d'alkyle inférieur ou de benzyle d'acide chloroformique en présence d'un accepteur d'acide, puis on fait réagir le produit avec l'ammoniac ou le carbonate d'ammonium.

**24.** Procédé de production des phénylacétylènes substitués de formule I suivant la revendication 1, dans lesquels $R_4$ désigne un groupe méthyle, suivant le mode opératoire (i) de la revendication 22, caractérisé en ce qu'on fait réagir le composé de formule III avec un agent d'acétylation et on élimine par hydrolyse basique sélective le groupe O-acétyle du composé ainsi obtenu de formule

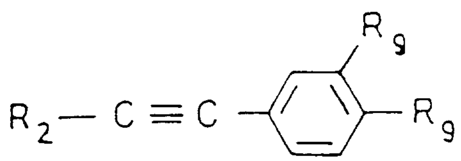

IV

dans laquelle l'un des restes $R_9$ est un atome d'hydrogène et l'autre est le groupe de formule

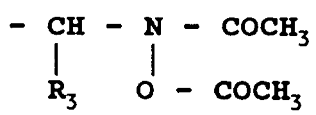

**25.** Procédé suivant la revendication 22, mode opératoire (i), caractérisé en ce que la formation de l'oxime du composé de formule II est conduite à des températures d'environ 20 à 60 °C.

**26.** Procédé suivant la revendication 22, mode opératoire (i), caractérisé en ce que l'oxime du composé de formule II est réduite avec du cyanoborohydrure de sodium en présence d'acides à des températures d'environ 20 à 60 °C en dérivé d'hydroxylamine de formule III.

**27.** Procédé suivant la revendication 22, mode opératoire (i), caractérisé en ce que l'oxime du composé de formule II est réduite avec des complexes borane-amine en présence d'acides à des températures d'environ 0 à 20 °C en le dérivé d'hydroxylamine de formule III.

**Revendications pour l'Etat contractant suivant : ES**

**1.** Procédé de production de phénylacétylènes substitués de formule générale

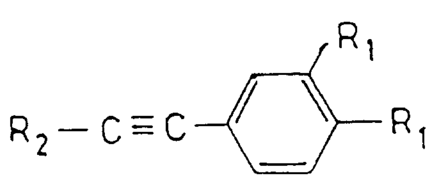

I

dans laquelle l'un des restes $R_1$ est un atome d'hydrogène, l'autre représente le groupe de formule

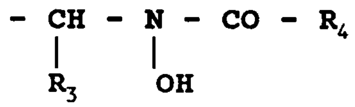

dans laquelle $R_3$ est un atome d'hydrogène, un groupe méthyle ou un groupe éthyle et $R_4$ représente un groupe méthyle ou un groupe amino,

et dans laquelle $R_2$ représente :

a) un reste aromatique monocyclique ou bicyclique du groupe des restes de formules

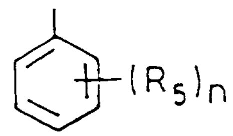 , 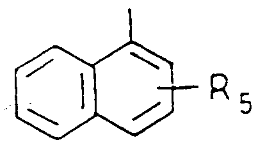 , 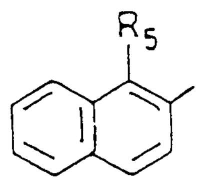 ou 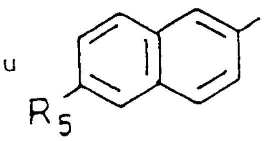

où $R_5$ est un atome d'hydrogène, un groupe benzyloxy, un reste alkyl-, alkoxy- ou alkylmercapto linéaire ou ramifié ayant 1 à 4 atomes de carbone, un atome de fluor ou de chlore ou le groupe trifluorométhyle et n est un nombre entier de 1 à 3,

ou bien

b) un reste hétérocyclique à un ou deux noyaux, contenant du soufre, de l'azote ou de l'oxygène, du groupe des restes de formules

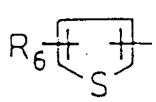 , 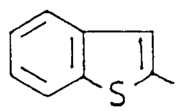 , 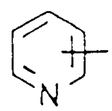 , 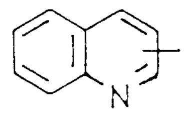 ou 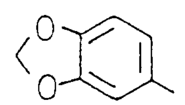

où $R_6$ représente un atome d'hydrogène ou un atome de chlore ou un groupe méthyle,

et des stéréoisomères et des formes optiquement actives de ces phénylacétylènes substitués de formule I, caractérisé en ce que

(i) on fait réagir un composé de formule

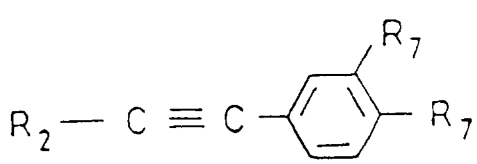 II

dans laquelle $R_2$ a la même définition que ci-dessus et l'un des restes $R_7$ est de l'hydrogène, l'autre représente le groupe de formule $-CO-R_3$,

avec l'hydroxylamine ou l'un de ses sels en présence de bases pour former une oxime, on réduit cette dernière au moyen de borohydrure ou de complexes borane-amine en présence d'acides en un composé de formule

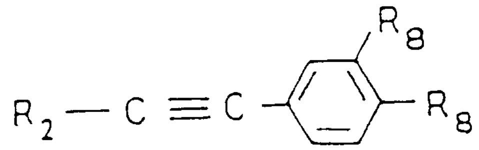

III

dans laquelle l'un des restes $R_8$ représente de l'hydrogène et l'autre est le groupe de formule $-CH(R_3)-NH-OH$ et on introduit dans ce composé le reste acyle de formule $-COR_4$, ou bien

(ii) on fait réagir à environ 20-80 °C un composé de formule

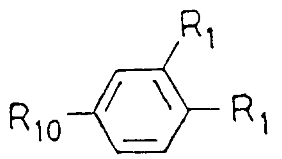

V

dans laquelle $R_1$ a la même définition que ci-dessus et $R_{10}$ est un atome de brome ou d'iode, avec un composé de formule

$R_2 - C \equiv CH$ VI

dans laquelle $R_2$ a la même définition que ci-dessus en présence d'une amine secondaire ou tertiaire liquide à environ 0-80 °C, notamment d'une dialkyl- ou trialkylamine ayant 2 ou 3 atomes de carbone dans chacun des restes alkyle, la pyrrolidine ou la pipéridine avec addition d'environ 0,01 à 5 %, par mole de composé utilisé de formule VI, d'un catalyseur complexe au palladium, notamment de chlorure ou d'acétate de bis-(triphénylphosphine)-palladium (II) ou de trétrakis-(triphénylphosphine)-palladium et le cas échéant avec addition de quantités catalytiques d'iodure de cuivre (I).

**2.** Procédé suivant la revendication 1 pour l'obtention de phénylacétylènes substitués de formule générale I suivant la revendication 1, caractérisé en ce que dans ces composés,

$R_1$ et $R_4$ ont la même définition que ci-dessus,

$R_3$ est un atome d'hydrogène ou un reste méthyle et

$R_2$ représente un reste aromatique monocyclique ou bicyclique du groupe des restes de formules

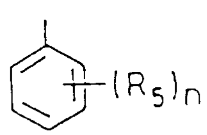 , 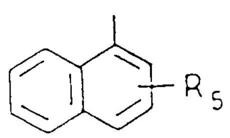 , 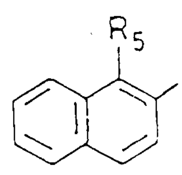 ou 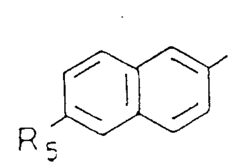

où $R_5$ représente un atome d'hydrogène, un groupe méthoxy, un reste alkyle linéaire ou ramifié ayant 1 à 4 atomes de carbone, un atome de fluor ou de chlore ou le groupe trifluorométhyle et n est un nombre entier de 1 à 3, ou bien

un reste hétérocyclique à 1 ou 2 noyaux contenant du soufre ou de l'oxygène, du groupe des restes de formules

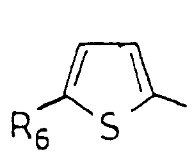 , 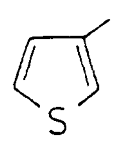 , 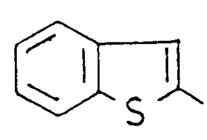 ou 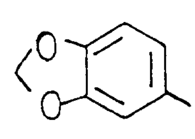

où $R_6$ représente un atome d'hydrogène ou un atome de chlore ou un groupe méthyle.

3. Procédé de production des phénylacétylènes substitués de formule I suivant les revendications 1 et 2 dans lesquels $R_4$ est un groupe amino, suivant le mode opératoire (i) de la revendication 1, caractérisé en ce qu'on fait réagir le composé de formule III
   a) avec le triméthylsilylisocyanate à des températures comprises entre 20 °C et 100 °C puis on élimine le groupe triméthylsilyle, ou bien
   b) avec le cyanate de potassium ou de sodium en solution acide ou avec le phosgène ou un ester d'alkyle inférieur ou de benzyle d'acide chloroformique en présence d'un accepteur d'acide, puis avec l'ammoniac ou le carbonate d'ammonium.

4. Procédé de production des phénylacétylènes substitués de formule I suivant les revendications 1 et 2, dans lesquels $R_4$ est un groupe méthyle, suivant le mode opératoire (i) de la revendication 1, caractérisé en ce qu'on fait réagir le composé de formule III avec un agent d'acétylation et on élimine par hydrolyse basique sélective le groupe O-acétyle du composé ainsi obtenu de formule

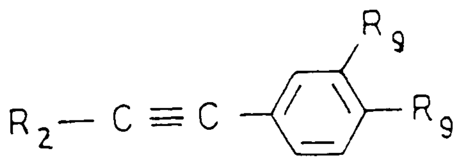

IV

dans laquelle l'un des restes $R_9$ est un atome d'hydrogène et l'autre représente le groupe de formule

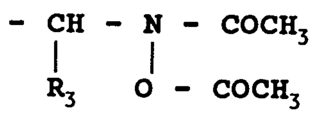

5. Procédé suivant la revendication 1, mode opératoire (i), caractérisé en ce qu'on conduit la formation de l'oxime du composé de formule II à des températures d'environ 20 à 60 °C.

6. Procédé suivant la revendication 1, mode opératoire (i), caractérisé en ce qu'on réduit l'oxime du composé de formule II avec le cyanoborohydrure de sodium en présence d'acides à des températures d'environ 20 à 60 °C, en le dérivé d'hydroxylamine de formule III.

7. Procédé suivant la revendication 1, mode opératoire (i), caractérisé en ce qu'on réduit l'oxime du composé de formule II avec des complexes borane-amine en présence d'acides à des températures d'environ 0 à 20 °C en le dérivé d'hydroxylamine de formule III.

**Revendications pour l'Etat contractant suivant : GR**

1. Phénylacétylènes substitués de formule générale

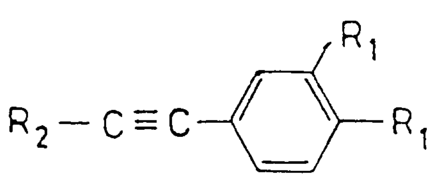

I

dans laquelle l'un des restes $R_1$ est un atome d'hydrogène, l'autre est le groupe de formule

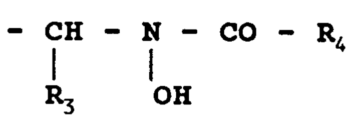

dans laquelle $R_3$ est un atome d'hydrogène, un reste méthyle ou un reste éthyle et $R_4$ est un reste méthyle ou un groupe amino
et dans laquelle $R_2$ représente :

a) un reste aromatique monocyclique ou bicyclique choisi dans le groupe des restes de formules

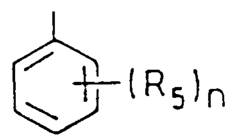 , 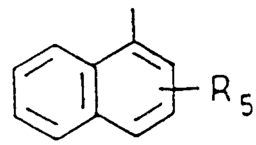 , 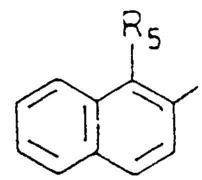 ou 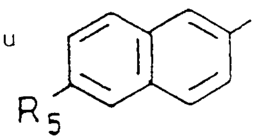

dans lesquelles $R_5$ est un atome d'hydrogène, un groupe benzyloxy, un groupe alkyl-, alkoxy- ou alkylmercapto linéaire ou ramifié ayant 1 à 4 atomes de carbone, un atome de fluor ou de chlore ou le groupe trifluorométhyle et n est un nombre entier de 1 à 3,
ou bien

b) un reste hétérocyclique à un ou deux noyaux, contenant du soufre, de l'azote ou de l'oxygène, choisi dans le groupe des restes de formules

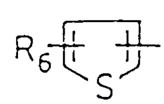 , 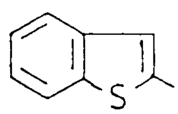 , 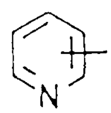 , 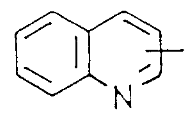 ou 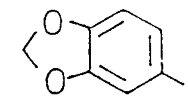

où $R_6$ désigne un atome d'hydrogène ou de chlore ou un groupe méthyle
et les stéréoisomères et les formes optiquement actives des phénylacétylènes substitués de formule I.

**2.** Phénylacétylènes substitués de formule générale I suivant la revendication 1, dans laquelle $R_1$ et $R_4$ ont la même définition que ci-dessus, $R_3$ représente un atome d'hydrogène ou un reste méthyle, et dans laquelle $R_2$ représente

a) un reste aromatique monocyclique ou bicyclique du groupe des restes de formules

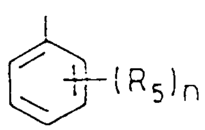 , 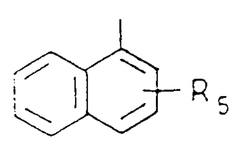 , 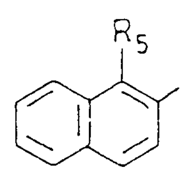 ou 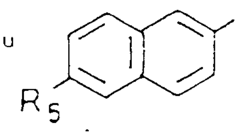 .

dans lesquelles $R_5$ représente un atome d'hydrogène, un groupe alkyl-, alkoxy- ou alkylmercapto linéaire ou ramifié ayant 1 à 4 atomes de carbone, un atome de fluor ou de chlore ou le groupe trifluorométhyle et n est un nombre entier de 1 à 3,
ou bien

b) un reste hétérocyclique à un ou deux noyaux, contenant du soufre ou de l'oxygène, choisi dans le groupe des restes de formules

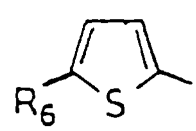 , 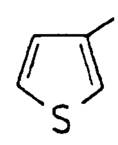 , 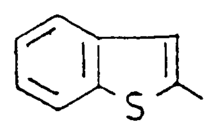 ou 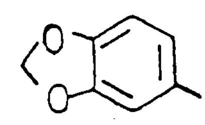

où $R_6$ représente un atome d'hydrogène ou de chlore ou un groupe méthyle

et les stéréoisomères et les formes optiquement actives des phénylacétylènes substitués de formule I.

**3.** Phénylacétylènes substitués de formule générale I suivant la revendication 1, dans laquelle $R_1$ et $R_4$ ont la même définition que ci-dessus, $R_3$ est un atome d'hydrogène ou un reste méthyle et dans laquelle $R_2$ représente

a) un reste aromatique monocyclique ou bicyclique du groupe des restes de formules

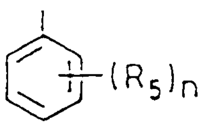 , 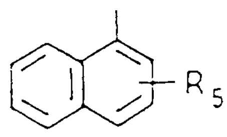 , 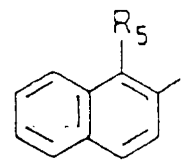 ou 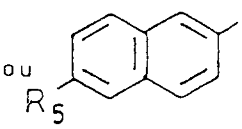

dans lesquelles $R_5$ est un atome d'hydrogène, un groupe méthoxy, un reste alkyle linéaire ou ramifié ayant 1 à 4 atomes de carbone, un atome de fluor ou de chlore ou le groupe trifluorométhyle et n est un nombre entier de 1 à 3,

ou bien

b) un reste hétérocyclique à un ou deux noyaux, contenant du soufre ou de l'oxygène, du groupe des restes de formules

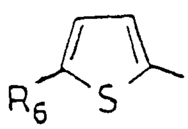 , 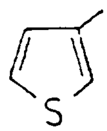 , 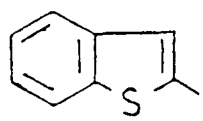 ou 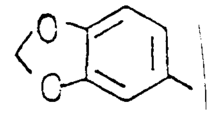

où $R_6$ représente un atome d'hydrogène ou de chlore ou un groupe méthyle,

et les stéréoisomères et les formes optiquement actives de ces phénylacétylènes substitués de formule I.

**4.** Phénylacétylènes substitués suivant la revendication 3 de formule I suivant la revendication 1, dans lesquels $R_2$ représente l'un des groupes

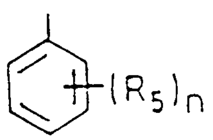 , 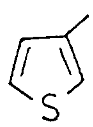 ainsi que 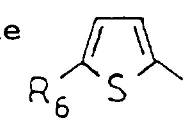

où $R_5$, $R_6$ et n ont la définition indiquée dans la revendication 3.

**5.** Phénylacétylènes substitués de formule I suivant la revendication 1, dans lesquels $R_2$ représente le reste 3-quinolyle ou de préférence le reste 3-pyridyle.

**6.** Phénylacétylènes substitués suivant la revendication 4, dans lesquels $R_5$ est un groupe méthoxy en position 3 et/ou en position 4 par rapport au groupe acétylène et n est l'un des nombres 1 et 2.

**7.** Phénylacétylènes substitués suivant la revendication 4, dans lesquels $R_5$ est un atome de fluor ou de chlore en position 4 par rapport au groupe acétylène.

**8.** Phénylacétylènes substitués suivant les revendications 2 à 4, dans lesquels $R_6$ est un atome d'hydrogène.

**9.** Phénylacétylènes substitués suivant les revendications 2 à 4, 6 et 7, de formule

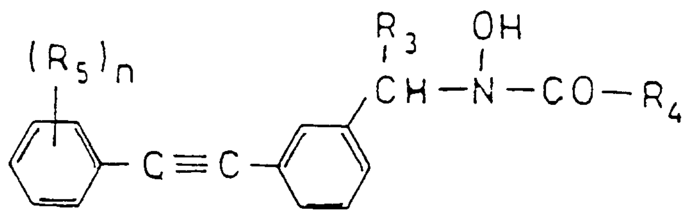

Ia

dans laquelle $R_3$ à $R_5$ et n ont la même définition que ci-dessus.

**10.** Phénylacétylènes substitués suivant les revendications 2 à 4, 6 et 7, de formule

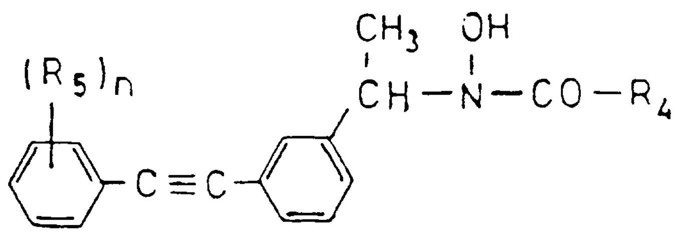

Ib

dans laquelle $R_4$ représente un groupe méthyle ou un groupe amino et $R_5$ est un groupe méthoxy en position 3 et/ou en position 4 par rapport au groupe acétylène (pour n = 1 ou 2) ou un atome de fluor ou de chlore en position 4 par rapport au groupe acétylène.

**11.** Phénylacétylènes substitués suivant la revendication 10, de formule

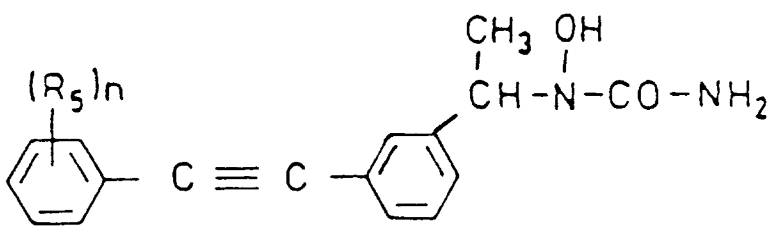

Ic

dans laquelle $R_5$ et n ont la même définition que dans la revendication 10.

**12.** Phénylacétylènes substitués suivant les revendications 1 à 4, de formule

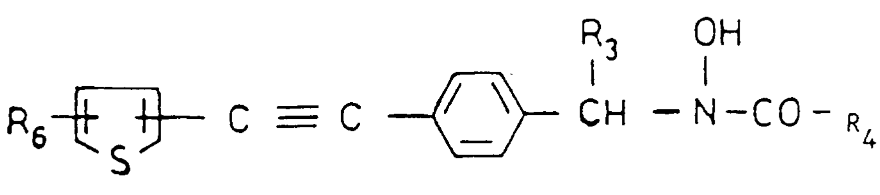

Id

dans laquelle $R_3$, $R_4$ et $R_6$ ont la même définition que ci-dessus.

**13.** Phénylacétylènes substitués suivant les revendications 2 à 4 et 12, de formule

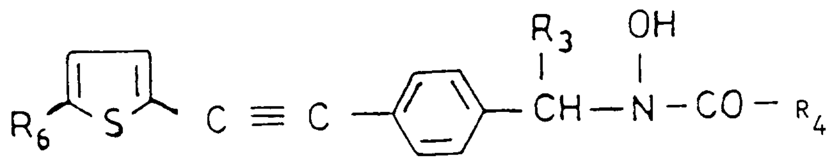

Ie

dans laquelle $R_3$ est un atome d'hydrogène ou un groupe méthyle et $R_4$ et $R_5$ ont la même définition que ci-dessus.

**14.** Phénylacétylènes substitués suivant les revendications 2 à 4, de formule

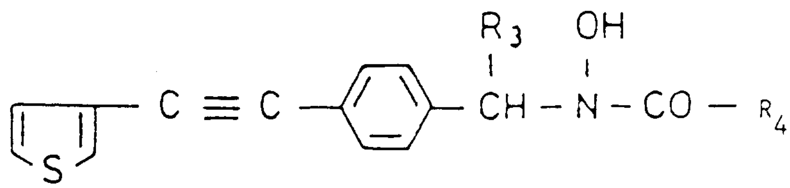

If

dans laquelle $R_3$ représente un atome d'hydrogène ou le groupe méthyle et $R_4$ est un groupe méthyle ou un groupe amino.

**15.** Procédé de production des phénylacétylènes substitués de formule I suivant la revendication 1, caractérisé en ce que :

(i) on fait réagir un composé de formule

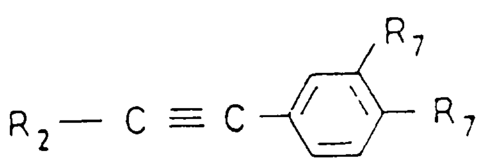

II)

dans laquelle $R_2$ a la même définition que ci-dessus et l'un des restes $R_7$ représente de l'hydrogène, l'autre représente le groupe de formule $-CO-R_3$, avec l'hydroxylamine ou l'un de ses sels en présence de bases pour former une oxime, on réduit cette dernière au moyen de borohydrures ou de complexes borane-amine en présence d'acides en un composé de formule

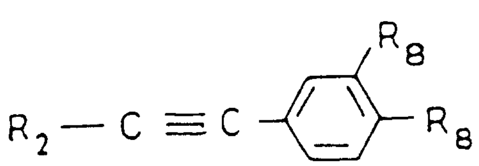

III

dont l'un des restes $R_8$ représente de l'hydrogène, l'autre est le groupe de formule

$- CH(R_3) - NH - OH$

et on introduit dans ce composé le reste acyle de formule $-COR_4$, ou

ii) on fait réagir à une température d'environ 20 à 80 °C un composé de formule

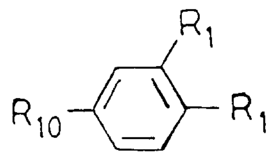

V

dans laquelle $R_1$ a la même définition que ci-dessus et $R_{10}$ représente un atome de brome ou d'iode, avec un composé de formule

$R_2 - C \equiv CH$ VI

dans laquelle $R_2$ a la même définition que ci-dessus,
en présence d'une amine secondaire ou tertiaire liquide à environ 0-80 °C, notamment d'une dialkyl- ou trialkylamine ayant 2 ou 3 atomes de carbone dans chacun des restes alkyle, la pyrrolidine ou la pipéridine avec addition d'environ 0,01 à 5 %, par mole de composé utilisé de formule VI, d'un catalyseur complexe au palladium, notamment le chlorure ou l'acétate de bis-(triphénylphosphine)-palladium (II) ou le tétrakis-(triphénylphosphine)-palladium et en ajoutant le cas échéant des quantités catalytiques d'iodure de cuivre (I).

**16.** Procédé de production des phénylacétylènes substitués de formule I suivant la revendication 1, dans lesquels $R_4$ désigne un groupe amino, suivant le mode opératoire (i) de la revendication 15, caractérisé en ce qu'on fait réagir le composé de formule III
a) avec le triméthylsilylisocyanate à des températures comprises entre 20 °C et 100 °C puis on élimine le groupe triméthylsilyle, ou bien
b) avec le cyanate de potassium ou de sodium en solution acide ou avec du phosgène ou un ester d'alkyle inférieur ou de benzyle d'acide chloroformique en présence d'un accepteur d'acide, puis on fait réagir le produit avec l'ammoniac ou le carbonate d'ammonium.

**17.** Procédé de production des phénylacétylènes substitués de formule I suivant la revendication 1, dans lesquels $R_4$ désigne un groupe méthyle, suivant le mode opératoire (i) de la revendication 15, caractérisé en ce qu'on fait réagir le composé de formule III avec un agent d'acétylation et on élimine par hydrolyse basique sélective le groupe O-acétyle du composé ainsi obtenu de formule

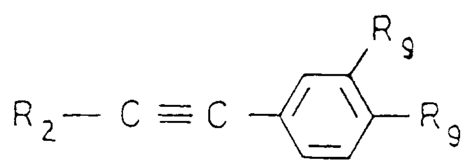

IV

dans laquelle l'un des restes $R_9$ est un atome d'hydrogène et l'autre est le groupe de formule

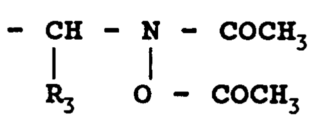

**18.** Procédé suivant la revendication 15, mode opératoire (i), caractérisé en ce que la formation de l'oxime du composé de formule II est conduite à des températures d'environ 20 à 60 °C.

**19.** Procédé suivant la revendication 15, mode opératoire (i), caractérisé en ce que l'oxime du composé de formule II est réduite avec du cyanoborohydrure de sodium en présence d'acides à des températures d'environ 20 à 60 °C en dérivé d'hydroxylamine de formule III.

**20.** Procédé suivant la revendication 22, mode opératoire (i), caractérisé en ce que l'oxime du composé de formule II est réduite avec des complexes borane-amine en présence d'acides à des températures d'environ 0 à 20 °C en le dérivé d'hydroxylamine de formule III.